(19) Europäisches Patentamt
European Patent Office
Office européen des brevets

(11) Publication number: 0 135 781 B1

(12) # EUROPEAN PATENT SPECIFICATION

(45) Date of publication of patent specification: 11.10.89

(21) Application number: 84109800.7

(22) Date of filing: 17.08.84

(51) Int. Cl.[4]: C 07 D 401/04, C 07 D 403/04, C 07 D 413/14, C 07 D 401/14, C 07 D 403/14, C 07 D 409/14, C 07 D 417/14, C 07 D 405/14, A 61 K 31/445, A 61 K 31/415, A 61 K 31/42

(54) 3-(Piperidinyl)- and 3-(pyrrolidinyl)-1H-indazoles, a process for their preparation and their use as a medicament.

(30) Priority: 22.08.83 US 525088

(43) Date of publication of application: 03.04.85 Bulletin 85/14

(45) Publication of the grant of the patent: 11.10.89 Bulletin 89/41

(84) Designated Contracting States: AT BE CH DE FR GB IT LI LU NL SE

(56) References cited:
Pharmazie, 33, H.7 (1978), pp. 419-424
CHEMICAL ABSTRACTS, vol. 88, no. 25, June 19, 1978, Columbus, Ohio, USA, C. WENTRUP; A. DAMERIUS; W. REICHEN "Intramolecular cyclization of nitrile imines. Synthesis of indazoles, fluorenes, and aza analogs", p. 753, abstract no. 190 693r
CHEMICAL ABSTRACTS, vol. 89, no. 25, December 18, 1978, Columbus, Ohio, USA, U. WRZECIONO; E. LINKOWSKA; W. FELINSKA "Azoles. Part 3. Nitro derivatives of 3-chloroindazole and the effect of C-5, C-6, and N-nitro groups on the reactivity of the C-3 chlorine atom", p. 585, abstract no. 215 290t

(73) Proprietor: HOECHST-ROUSSEL PHARMACEUTICALS INCORPORATED
Route 202-206 North
Somerville New Jersey 08876 (US)

(72) Inventor: Strupczewski, Joseph T.
4 Stewart Lane
Flemington New Jersey, 08822 (US)

(74) Representative: Meyer-Dulheuer, Karl-Hermann, Dr. et al
HOECHST Aktiengesellschaft Postfach 3540
D-6121 Wiesbaden (DE)

(56) References cited:
CHEMICAL ABSTRACTS, vol. 94, no. 23, June 8, 1981, Columbus, Ohio, USA, U. WRZECIONO; E. LINKOWSKA; "Azoles. Part 7. Reaction of 2,5-and 2,6-dinitroindazoles with cyclic amines", p. 650, abstract no. 192 210d

EP 0 135 781 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

**CHEMICAL ABSTRACTS, vol. 99, no. 1, July 4, 1983, Columbus, Ohio, USA, U. WRZECIONO; E. LINKOWSKA; S.J. LATAWIEC-DOROSZ; T.W. KOSNO "Azoles. Part 10: Behavior of 2,4-dinitroindazole on aliphatic and cyclic amines", p. 510, abstract no. 5564r**

**CHEMICAL ABSTRACTS; VOL: (/; NO: ); August 29, 1977, Columbus, Ohio, USA, Y. FUJIMURA; H. NAGANO; M. SHINDO; M. KAKIMOTO; T. IWASAKI; Y. IKEDA "Indazole derivatives", p. 553, abstract no. 68 348w**

## Description

The present invention relates to novel 3-(piperidinyl)- and 3-(pyrrolidinyl)-1H-indazoles of formula 1

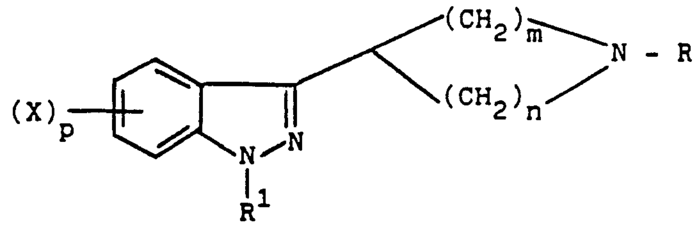

(1) ,

wherein R is hydrogen, $C_1$—$C_7$-alkyl, $C_3$—$C_7$-alkenyl, $C_3$—$C_7$-cycloalkyl-$C_1$—$C_7$-alkyl,

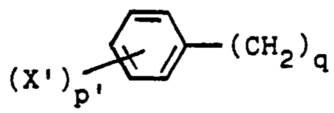

,

cyano, cyanomethyl, formyl, $C_1$—$C_7$-alkanoyl, or a group of the formulae

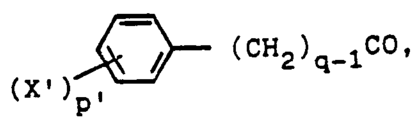

, $R^2OCO$,

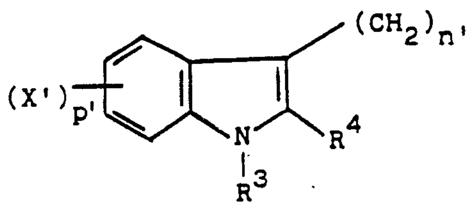

,

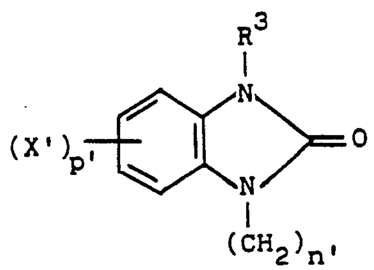

,

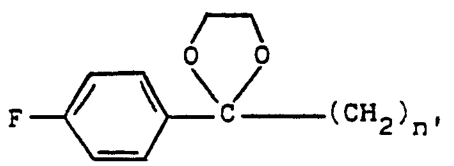

,

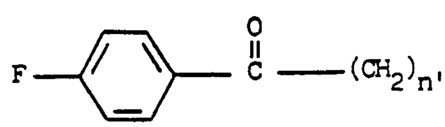

,

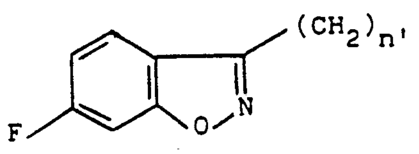

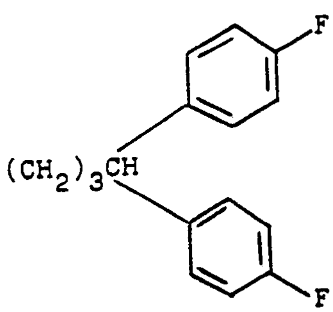

$R^1$ is hydrogen, $C_1$—$C_7$-alkyl, $C_3$—$C_7$-alkenyl, $C_3$—$C_7$-cycloalkyl-$C_1$—$C_7$-alkyl,

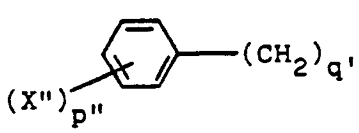

,

di-$C_1$—$C_7$-alkylamino-$C_1$—$C_7$-alkyl, cyano, cyanomethyl, formyl, $C_1$—$C_7$-alkanoyl, hydroxymethyl, $C_3$—$C_7$-cycloalkyl-$C_1$—$C_7$-alkanoyl, or a group of the formulae

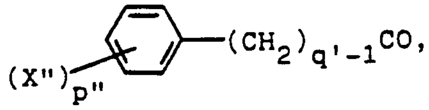 $R^{2'}CO$ ,

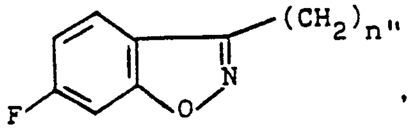 , 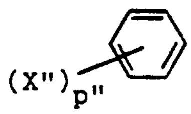 ,

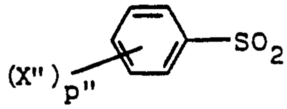

$R^5CO$, 2- or 4-pyridinyl or 2-pyrimidinyl; $R^2$ and $R^{2'}$ are independently $C_1$—$C_7$-alkyl, 2,2,2-trichloroethyl or phenyl, $R^3$ and $R^4$ are independently hydrogen or $C_1$—$C_7$-alkyl; $R^5$ represents the group furyl, thienyl, pyridinyl, thiazolyl, isothiazolyl, oxazolyl or isoxazolyl, X, X′ and X″ are independently hydrogen, halogen, $C_1$—$C_7$-alkyl, $C_1$—$C_7$-alkoxy, hydroxy, nitro, amino or trifluoromethyl; m is 2 or 3; n is 1 or 2; and the sum of m and n is 3 or 4; n′ and n″ are independently 2 or 3; p, p′ and p″ are independently 1 or 2; q and q′ are independently 1, 2, 3 or 4; the optical antipode thereof, or the pharmaceutically acceptable acid addition salt thereof, which are useful for treating psychoses and alleviating pain, alone or in combination with inert psychoses-treating and pain-alleviating adjuvants.

Preferred 3-(piperidinyl)- and 3-(pyrrolidinyl)-1H-indazoles of the present invention are those wherein R is hydrogen, $C_1$—$C_7$-alkyl or

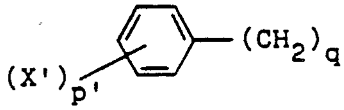

wherein, X′, p′ and q are as above and $R^1$ is hydrogen, alkyl, $C_1$—$C_7$-alkyl, $C_3$—$C_7$-cycloalkyl-$C_1$—$C_7$-alkyl, $C_1$—$C_7$-alkanoyl, hydroxymethyl,

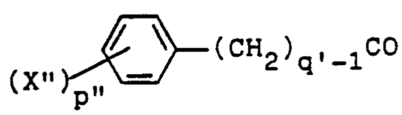

wherein X″ and p″ are as above and q′ is 1, 2, 3 or 4 or $R^{2'}OCO$ wherein $R^{2'}$ is as above or

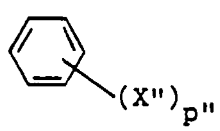

wherein X″ and p″ are as above. Most preferred are these wherein R is hydrogen or $C_1$—$C_7$-alkyl and $R^1$ is

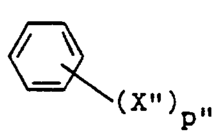 or 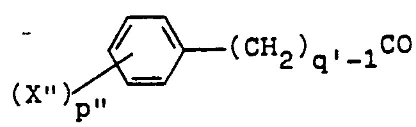

wherein X″, p″ and q′ are as above. As used through the specification and appended claims, the term "alkyl" refers to a straight or branched chain hydrocarbon radical containing no unsaturation and having 1 to 7 carbon atoms such as methyl, ethyl, 1-propyl, 2-propyl, 1-butyl, 1-pentyl, 3-hexyl, 4-heptyl; the term "alkenyl" refers to a straight or branched chain hydrocarbon radical having one olefinic bond and containing 3 to 7 carbon atoms such as 2-propenyl, 2-butenyl, 3-pentenyl, 3-hexenyl, 3-heptenyl; the term "cycloalkyl" refers to a saturated hydrocarbon group possessing at least one carbocyclic ring, the ring containing from 3 to 7 carbon atoms such as cyclopropyl, cyclobutyl, cyclopentyl, cyclohexyl, cycloheptyl; the term "alkanol" refers to a compound formed by a combination of an alkyl group and a hydroxy radical. Examples of alkanols are methanol, ethanol, 1- and 2-propanol, 1,2-dimethylethanol, hexanol. The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, hexanoic acid; the term "halogen" refers to a member of the family consisting of fluorine, chlorine,

bromine or iodine. The term "alkanoyl" refers to the radical formed by removal of the hydroxyl function from an alkanoic acid. Examples of alkanoyl groups are formyl, acetyl, propionyl, 2,2-dimethylacetyl, hexanoyl.

The compounds of the present invention which lack an element of symmetry exist as optical antipodes and as the racemic forms thereof. The optical antipode may be prepared from the corresponding racemic forms by standard optical resolution techniques, involving, for example, the separation of diasteromeric salts of those instant compounds characterized by the presence of a basic amino group and an optically active acid, or by the synthesis from optically active precursors.

The present invention comprehends all optical isomers and racemic forms thereof of the compounds disclosed and claimed herein. The formulas of the compounds shown herein are intended to encompass all optical isomers of the compounds so depicted.

The novel 3-(piperidinyl) and 3-(pyrrolidinyl)-1H-indazoles of the present invention are synthesized by processes illustrated in Reaction Schemes A to C.

To prepare the 3-(piperidinyl)- or 3-(pyrrolidinyl)-1H-indazole system of formula 1 wherein R is alkyl, a 1-alkylchloropiperidine *3* or a 1-alkylchloropyrrolidine 3 is condensed with a 2-fluorobenzonitrile *4* to a 1-alkyl-(2-fluorobenzoyl)piperidine *5* or a 1-(alkyl-(2-fluorobenzoyl)pyrrolidine *5* which is cyclized to a 3-(1-alkylpiperidinyl)- or a 3-(1-alkylpyrrolidinyl)-1H-indazole 8. See Reaction Scheme A.

The condensation of a 1-alkylchloropiperidine *3* or a 1-alkylchloropyrrolidine *3* with a 2-fluorobenzonitrile *4* is effected by conventional Grignard techniques. Typically, the benzonitrile *4*, dissolved in an ethereal solvent, such as tetrahydrofuran, is treated with a Grignard reagent, prepared from the chloropiperidine *3* or chloropyrrolidine *3* and magnesium turnings in an ethereal solvent, such as tetrahydrofuran, followed by hydrolysis under acidic conditions. An initiator such as ethyl bromide may be employed to facilitate formation of the Grignard reagent. The cyclization of a 1-alkyl-(2-fluorobenzoyl)piperidine *5* or a 1-alkyl-(2-fluorobenzyl)pyrrolidine *5* to a 3-(1-alkylpiperidinyl)-1H-indazole *8* or a 3-(1-alkylpyrrolidinyl)-1H-indazole *8* is performed by treating the benzoylpiperidine *5* or benzoylpyrrolidine *5* with hydrazine, generally as the hydrate, at an elevated temperature and pressure within the range of about 100° to about 200°C, and about 14 to about 21 bar, respectively. A cyclization temperature of about 150°C is preferred. A cyclization pressure of about 17.5 bar is also preferred.

To synthesize the 3-(piperidinyl)- or 3-(pyrrolidinyl)-1H-indazole system of formula 1 wherein R is alkenyl, cycloalkylalkyl,

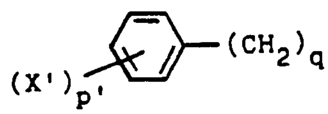

cyanomethyl,

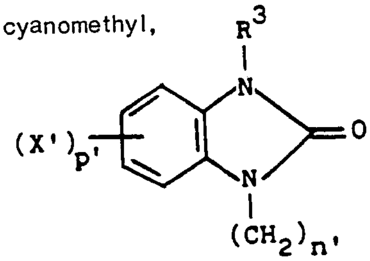

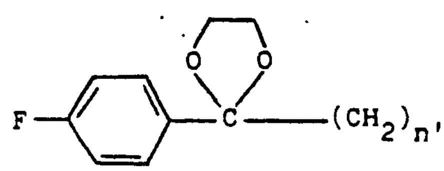

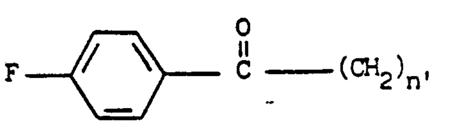

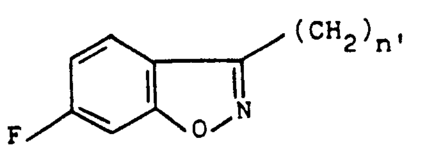

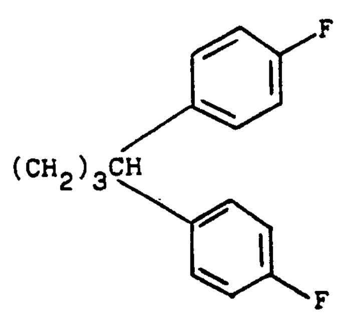

wherein $R^3$, X', n', p' and q are as above, a 1-alkyl-(2-fluorobenzoyl)piperidine *5* or 1-alkyl-(2-fluorobenzoyl)-pyrrolidine *5* is converted to a 1-phenoxycarbonyl-(2-fluorobenzoyl)piperidine *6* or 1-phenoxycarbonyl-(2-fluorobenzoyl)pyrrolidine *6* followed by hydrolysis of the 1-phenoxycarbonylpiperidine *6* or 1-phenoxycarbonylpyrrolidine *6* to a (2-fluorobenzoyl)piperidine *7* or (2-fluorobenzoyl)pyrrolidine *7*, alkylation of the

benzoylpiperidine *7* or benzoylpyrrolidine 7 to an N-substituted benzoylpiperidine *9* or N-substituted benzoylpyrrolidine *9* and cyclization to the indazole *10* wherein R is as immediately above. See Reaction Schemes A and B.

The conversion of the 1-alkylpiperidine *5* or 1-alkylpyrrolidine *5* to the phenoxycarbonylpiperidine *6* or phenoxycarbonylpyrrolidine *6* is accomplished by treating a tertiary amine *5* with phenylchloroformate in an aromatic solvent in the presence of an acid scavenger. Included among aromatic solvents are benzene, toluene or xylene. Toluene is the preferred aromatic solvent. Included among acid scavengers are sodium carbonate or potassium carbonate. Potassium carbonate is preferred. While the reaction temperature is not narrowly critical, it is preferred to conduct the conversion at the reflux temperature of the reaction mixture to assure a reasonable rate of formation of the carbamate *6*.

The hydrolysis of the phenoxycarbonylpiperidine *6* or phenoxycarbonylpyrrolidine *6* to the 1-unsubstituted piperidine *7* or 1-unsubstituted pyrrolidine *7* is performed by methods well-known in the art, involving, for example, treatment of the carbamate *6* with an aqueous solution of an alkali metal hydroxide such as sodium hydroxide or potassium hydroxide at or about the reflux temperature of the reaction mixture. Aqueous potassium hydroxide is preferred.

The alkylation of an N-unsubstituted piperidine *7* or N-unsubstituted pyrrolidine *7* to an N-substituted piperidine *9* wherein R is as immediately above defined or an N-substituted pyrrolidine *9* wherein R is also as immediately above defined is performed by means of an alkenyl, cycloalkylalkyl, or cyanomethyl halide, i.e. an iodide, bromide or chloride, or a compound of the formulae

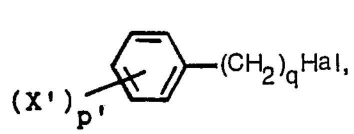

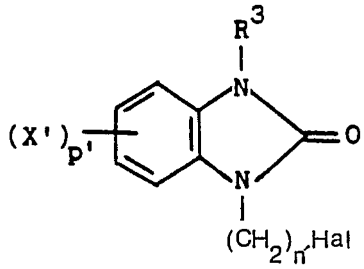

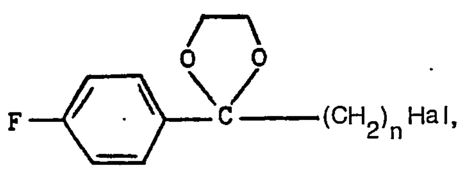

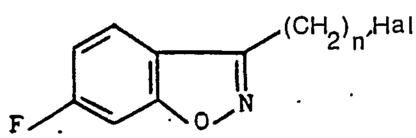

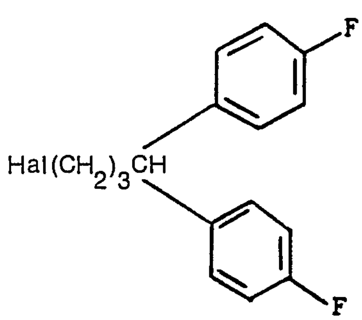

wherein $R^3$, X′, n′, p′ and q are as above and Hal is iodo, bromo or chloro, in the presence of a base suspended or dissolved in a polar aprotic solvent. Suitable bases include alkali metal carbonates and bicarbonates such as, for example, sodium and potassium carbonate and sodium and potassium bicarbonate. Suitable polar aprotic solvents include dimethylformamide, dimethylacetamide and hexamethylphosphoramide. Potassium carbonate and dimethylformamide are the preferred base and solvent. A reaction promotor such as potassium iodide and an elevated reaction temperature within the range of about 70° to about 120°C may be employed to facilitate the alkylation. A reaction temperature of about 90°C is preferred.

To furnish the 3-(piperidinyl)- or 3-(pyrrolidinyl)-1H-indazole of formula 1 wherein R is

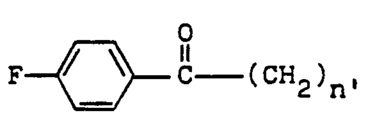

wherein n′ is as above the corresponding ethylene glycol cyclic ketal thereof, i.e., a compound of formula 1 wherein R is

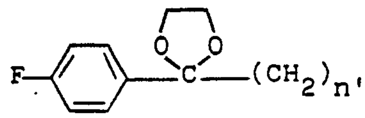

is hydrolyzed. The hydrolysis is conveniently accomplished by conventional methods involving, for example, the interaction of a mineral acid, such as hydrochloric acid, in an alkanol, such as methanol, at ambient temperature, or an elevated temperature such as the reflux temperature of the reaction system.

To introduce the indol-3-ylalkyl function, i.e., to fabricate an N-substituted piperidine or N-substituted pyrrolidine of formula 1 wherein R is a group of the formula

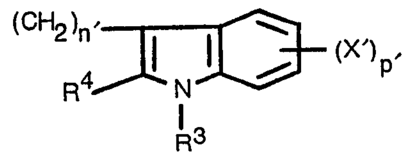

wherein $R^3$, $R^4$, X′, n′ and p′ are as above, one treats an N-unsubstituted piperidine *7* or N-unsubstituted pyrrolidine *7* with a 3-(phenylsulfonylalkyl)indole or 3-(alkylphenylsulfonylalkyl)indole of the formula

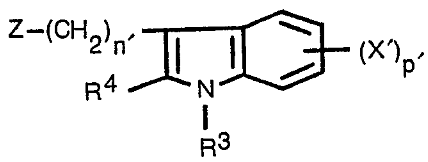

wherein Z is a group of the formula

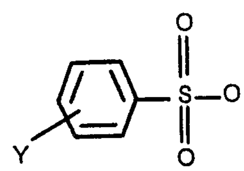

wherein Y is hydrogen or alkyl and $R^3$, $R^4$, X′, n′ and p′ are as above.

The reaction involving the displacement of the phenylsulfonyl group of the indole is accomplished by treating an N-unsubstituted piperidine *7* or N-unsubstituted pyrrolidine *7* with the phenylsulfonylindole in an aprotic polar solvent, such as dimethylformamide, dimethylacetamide and hexamethylphosphoramide, or an alkanone such as acetone, 2-butanone or 3-pentanone, dimethylformamide and 2-butanone being preferred, in the presence of an acid scavenger such as an alkali metal carbonate (sodium or potassium carbonate) or alkali metal bicarbonate (sodium or potassium bicarbonate), potassium carbonate and sodium bicarbonate being preferred, at a temperature of about 70° to about 110°C preferably a temperature of 90°C, when an aprotic polar solvent is used, and at about the reflux temperature of the reaction system when an alkanol is employed as the solvent.

The cyclization of the N-substituted benzoylpiperidine *9* or N-substituted benzoylpyrrolidine *9* to the 1H-indazole *10* is readily achieved by procedures substantially similar to those utilized for the cyclization of benzoylpiperidine *5* or benzoylpyrrolidine *5* to 1H-indazole *8*.

To prepare 1H-indazoles substituted at the 1-position, i.e., compounds of formula 11 wherein $R^1$ is alkyl, alkenyl, cycloalkylalkyl, dialkylaminoalkyl, cyano, cyanomethyl,

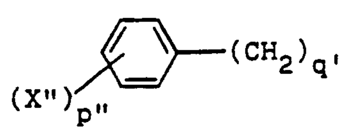 , 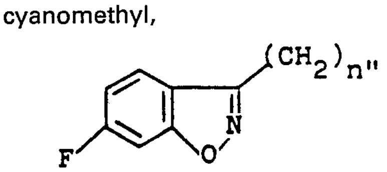 ,

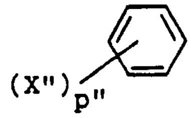 ;

2- or 4-pyridinyl or 2-pyrimidinyl, R is as hereinabove defined, with the proviso that R is not hydrogen, and X, m, n and p are as hereinbefore defined, a 1-unsubstituted 1H-indazole *8* wherein R is as hereinbefore defined, with the proviso that R is not hydrogen, and X, m and p are as hereinbefore defined, is treated, respectively, with an alkyl, alkenyl, cycloalkylalkyl, dialkylaminoalkyl, cyanogen, cyanomethyl, 2- or 4-pyridinyl or 2-pyrimidinyl halide, i.e., an iodide, bromide or chloride, or a compound of the formulae

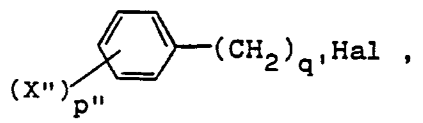 , 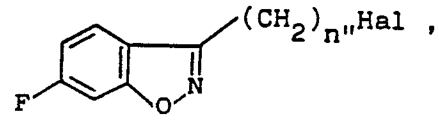 ,

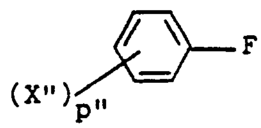 ,

wherein X'', n'', q' and p'' are as defined above and Hal is iodo, bromo or chloro, in the presence of an alkali metal hydride suspended in a polar aprotic solvent. Among alkali metal hydrides, there may be mentioned lithium hydride, potassium hydride and sodium hydride. Sodium hydride is preferred. Among polar aprotic solvents, there may be mentioned dimethylformamide, dimethylacetamide and hexamethylphosphoramide. Dimethylformamide is preferred. While the alkylation normally proceeds readily at ambient temperature, the reaction may be conducted at an elevated temperature of about 50° to about 100°C to facilitate the conversion. See Reaction Scheme C.

Alternatively, 1H-indazoles substituted at the 1-position by a group of the formula

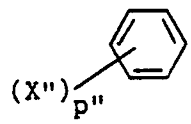

i.e., compounds of the formula 11 wherein $R^1$ is

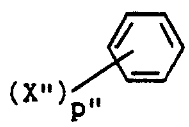

wherein X'' is hydrogen, halogen, alkyl, alkoxy, hydroxy or amino and p'' is 1 or 2, may be prepared from the corresponding compounds wherein X'' is nitro by conventional methods involving, for example, reduction of nitro group to an amino function, diazotization and subsequent displacement or reduction of the diazonium moiety.

To provide a 1-hydroxymethyl-3-(piperidinyl)-1H-indazole *13* or 1-hydroxymethyl-3-(pyrrolidinyl)-1H-indazole *13*, a 1-unsubstituted 3-(piperidinyl)-1H-indazole *8* or 1-unsubstituted 3-(pyrrolidinyl)-1H-indazole *8* wherein R is as hereinbefore defined, with the exception that R is not hydrogen, is treated with formaldehyde in the form of paraformaldehyde or trioxane in an alkanol such as methanol, ethanol or 2-propanol, preferably ethanol, in the presence of an alkali metal hydroxide such as lithium hydroxide, sodium hydroxide, or potassium hydroxide, sodium hydroxide being preferred, at an elevated temperature of about the reflux temperature of the reaction mixture.

To prepare the hydroxymethyl-1H-indazole *13* wherein R is hydrogen, one conducts the condensation on, for example, an N-phenoxycarbonyl-1H-indazole of formula 10 and then removes the carbamoyl group under conventional mild hydrolysis conditions.

To fabricate the 3-(piperidinyl)- or 3-(pyrrolidinyl)-1H-indazole system of formula 1 wherein R is

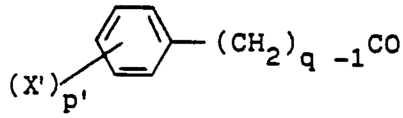

alkanoyl, formyl or $R^2OCO$ wherein $R^2$, X', p' and q are as defined above, a 3-(piperidinyl)- or 3-(pyrrolidinyl)-1H-indazole of formula 18, i.e., a compound wherein the nitrogen atom of the 3-(piperidinyl) or 3-(pyrrolidinyl) groups and the nitrogen atom occupying the 1-position of the indazole system are unsubstituted, is treated with an alkanoyl or formyl halide, i.e., an alkanoyl or formyl iodide, bromide or chloride, or halide or the formulas

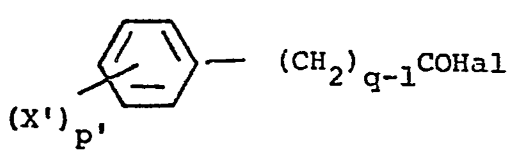

and $R^2OCOHal$, wherein $R^2$, X', p' and q are as above and Hal is iodo, bromo or chloro, in a halocarbon such as dichloromethane or trichloromethane, or a polar aprotic solvent such as dimethylformamide, dimethylacetamide or hexamethylphosphoramide in the presence of an acid scavenger such as sodium or potassium carbonate, or sodium or potassium bicarbonate to provide a 3-(piperidinyl)- or 3-(pyrrolidinyl)-1H-indazole of formula 19 wherein R, X, m, n and p are as above. Trichloromethane and dimethylformamide are the preferred solvents, and potassium bicarbonate is the preferred scavenger.

To minimize formation of disubstituted products, i.e., compounds of formula 20 wherein R, X, m, n and p are as above, one generally performs the acylation reaction, i.e., the conversion of *18* to *19*, at about ambient temperature, at which temperature only minor amounts of the N,N′-disubstituted product *20* are usually formed. The N-substituted and N,N′-disubstituted products *19* and *20* may be separated by conventional techniques such as recrystallization. In the event substantial amounts of the N,N′-disubstituted product are formed, or the separation proves to be difficult, one may transform the N,N′-disubstituted compound *20* to the monosubstituted product *19* by alcoholysis. The alcoholysis is conveniently accomplished by means of an alkali metal alkoxide in an alkanol. Suitable alkali metal alkoxides include lithium, sodium or potassium methoxide, ethoxide or 1-propoxide. Suitable alkanols include methanol, ethanol or 1-propanol. Sodium methoxide in methanol is preferred.

To provide the 1H-indazole *10* wherein R is hydrogen and X, m, n and p are as defined above an N-unsubstituted benzoylpiperidine *7* or N-unsubstituted benzoylpyrrolidine *7* is cyclized with hydrazine hydrate by the hereinbefore described process.

3-(Piperidinyl)- or 3-(pyrrolidinyl)-1H-indazol systems of formula 1 wherein R is cyano are also prepared by treating a 1-substituted 1H-indazole of formula 11 wherein R is alkyl and $R^1$ is formyl, alkanoyl or

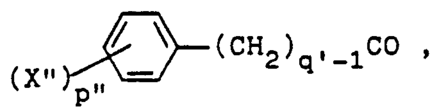

wherein X″, p″ and q′ are as above with cyanogen bromide or chloride as hereinbefore described. For example, treatment of a solution of 1-benzoyl-3-(1-methyl-4-piperidinyl)-1H-indazole (*11* wherein R is methyl, $R_1$ is benzoyl, X is hydrogen, m and n are 2 and p is 1) and chloroform with cyanogen bromide in the presence of potassium carbonate provides 1-benzoyl-3-(1-cyano-4-piperidinyl)-1H-indazole (*1* wherein R is cyano, $R^1$ is benzoyl, X is hydrogen, m and n are 2 and p is 1).

To prepare 1-substituted 1H-indazoles of formula 1 wherein $R^1$ is formyl, alkanoyl, cycloalkylalkanoyl

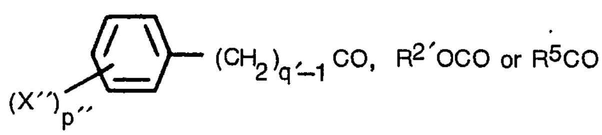

wherein $R^{2'}$, $R^5$, X″, p″ and q′ are as above, a 1-unsubstituted 1H-indazole *8* wherein R is as above, with the proviso that R is not hydrogen, and X, m, n and p are as before, is treated, respectively, with a formyl, alkanoyl or cycloalkylalkanoyl chloride, bromide or iodide, a compound of the formulae

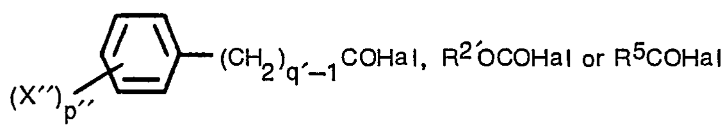

wherein $R^{2'}$, $R^5$, X″, p″ and q′ are as above and Hal is iodo, bromo or chloro, or the corresponding acid anhydrides thereof, at an elevated temperature of about the reflux temperature of the reaction medium. For example, treatment of a 1H-indazol *8* wherein R is methyl with acetic anhydride at the reflux temperature of the reaction mixture affords a 1-acetyl-1H-indazole *14* wherein $R^2$ is methyl, and treatment of a 1H-indazole *8* wherein R is methyl with benzoyl chloride at a reaction temperature of about 100°C yields a 1-benzoyl-1H-indazole *14* wherein $R^2$ is phenyl.

Alternatively, the synthesis of 1-substituted 1H-indazoles of formula 1 wherein $R^1$ is formyl, alkanoyl, cycloalkylalkanoyl,

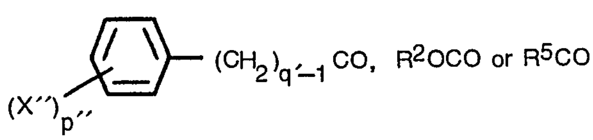

wherein $R^2$, $R^5$, X′, p′ and q′ are as above is accomplished by contacting a formyl, cycloalkylalkanoyl or alkanoyl halide or compound of the formula

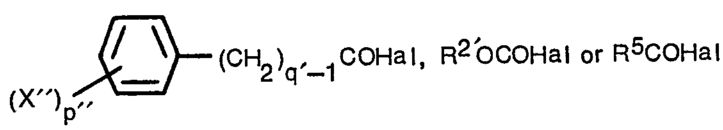

wherein $R^{2'}$, $R^5$, X'', p'' and q' are as above, with a 1-unsubstituted-1H-indazole *8* in a halocarbon such as dichloromethane or trichloromethane, preferably trichloromethane, in the presence of an acid scavenger such as sodium or potassium carbonate, or sodium or potassium bicarbonate, preferably potassium carbonate. The reaction proceeds readily at moderate temperatures. To promote the conversion however, elevated temperatures, i.e., the reflux temperature of the reaction medium are generally employed.

To provide 1-substituted 1H-indazoles *11*, wherein $R^1$ is

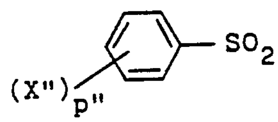

wherein X'' and p'' are as described hereinbefore, a 1-unsubstituted 1H-indazole *8* is treated with a benzenesulfonyl halide of the formula

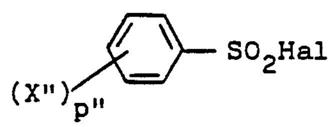 ,

wherein X'' and p'' are as described and Hal is chloride or bromide at an elevated temperature within the range of about 80° to about 150°C, a reaction temperature of about 100°C being preferred.

1H-indazoles of formula 11 wherein $R^1$, X, m, n and p are as above and R is hydrogen are prepared from 1H-indazoles of formula 11 wherein $R^1$, X, m, n and p are as above and R is alkyl by the conversion of a 3-(1-alkylpiperidinyl)- or 3-(1-alkylpyrrolidinyl)-1H-indazole *11* wherein R is alkyl and $R^1$, X, m, n and p are as above to a 3-(1-cyanopiperidinyl)- or 3-(1-cyanopyrrolidinyl)-1H-indazole *11* wherein R is cyano and $R^1$, X, m and p are as above followed by removal of the cyano group to a 1H-indazole of formula 11 wherein R is hydrogen and $R^1$, X, m, n and p are as above. The conversion of a 1-alkylpiperidine *11* or 1-alkylpyrrolidine *11* wherein R is alkyl to the corresponding 1-cyano compound *11* wherein R is cyano is accomplished by treating the alkylpiperidine *11* or alkylpyrrolidine *11* with a cyanogen halide such as cyanogen bromide or chloride in the presence of an acid acceptor such as sodium or potassium carbonate or sodium or potassium bicarbonate in a suitable solvent. Suitable solvents include halocarbons such as dichloromethane, trichloromethane or 1,2-dichloroethane, and dipolar aprotic solvents such as dimethylformamide, dimethylacetamide, hexamethylphosphoramide, and dimethylsulfoxide. Cyanogen bromide is the preferred halide and dimethyl sulfoxide containing potassium carbonate is the preferred reaction medium. The conversion temperature is not critical. To avoid side-reactions, however, it is preferred to perform the reaction at reduced temperatures between about 0° and about 30°C. A temperature of about 25°C is preferred.

The removal of the cyano group of compounds of formula 11 wherein R is cyano may be effected under conventional hydrolytic conditions, utilizing for example, aqueous organic acids such as aqueous acetic, or mineral acids such as dilute hydrochloric acid or sulfuric acid.

N-unsubstituted 1H-indazoles, i.e., 1H-indazoles of formula 18 wherein both the indazole and piperidine (or pyrrolidine) nitrogen atoms are unsubstituted, are prepared by an alternative synthesis involving the condensation of an appropriately substituted fluorobenzene of formula 15 wherein X and p are selected from the groups described above with an N-formyl- or N-alkanoyl-(piperidinyl)- or (pyrrolidinyl)carbonyl halide of formula 16 wherein R is formyl or alkanoyl and Hal is chloro or bromo, followed by cyclization of the resulting (2-fluorobenzoyl)piperidine *17* or (2-fluorobenzoyl)pyrrolidine *17* to the 1H-indazole *18*.

The condensation of fluorobenzene *15* with a carbonyl halide *16* is accomplished under Friedel-Crafts conditions as described in U.S. Patent 4,355,037.

The cyclization of a (2-fluorobenzoyl)piperidine *17* or (2-fluorobenzoyl)pyrrolidine *17* to a 1H-indazole *18* is performed with hydrazine hydrate in an alkanol at an elevated temperature, conditions under which the N-formyl or N-alkanoyl group of the piperidine or pyrrolidine ring is removed. Suitable alkanols include ethanol, 2-propanol, 1-butanol or 3-pentanol. 1-Butanol is preferred. A cyclization temperature of the boiling point of the reaction medium is also preferred.

The synthesis of 3-(3-haloalkyl)-6-fluoro-1,2-benzisoxazoles, 4,4-bis(4-fluorophenyl)butyl halides, 3-(phenylsulfonylalkyl)indoles and 3-(alkylphenylsulfonylalkyl)-indoles, 1-(3-haloalkyl)1,2-dihydro-2H-benzimidazol-2-ones; and 4-fluorobenzoylalkyl halides ethylene glycol ketals requisite precursors for the preparation of 3-(4-piperidinyl)-1H-indazoles and 3-(3-pyrrolidinyl)-1H-indazoles of the present invention, substituted at the indazole and/or piperidine or pyrrolidine nitrogen atoms, are described in U.S. Patent 4,352,811.

The 3-(piperidinyl)- and 3-(pyrrolidinyl)-1H-indazoles of the present invention are useful for treating psychoses by virtue of their ability to elicit an antipsychotic response in mammals.

Antipsychotic activity is determined in the climbing mice assay by methods similar to those described by P. Protais, et al., Psychopharmacol., *50* 1 (1976) and B. Costall, Eur. J. Pharmacol., *50* 39 (1978).

The subject CK—1 male mice (23—27 grams) are group-housed under standard laboratory conditions. The mice are individually placed in wire mesh stick cages (4″ × 4″ × 10″) and are allowed one hour for

adaptation and exploration of the new environment.

Then apomorphine is injected subcutaneously at 1.5 mg/kg, a dose causing climbing in all subjects for 30 minutes. Compounds to be tested for antipsychotic activity are injected intraperitoneally 30 minutes prior to the apomorphine challenge at a screening dose of 10 mg/kg.

For evaluation of climbing, 3 readings are taken at 10, 20 and 30 minutes after apomorphine administration according to the following scale:

| Climbing Behavior | Score |
|---|---|
| Mice with: | |
| 4 paws on bottom (no climbing) | 0 |
| 2 paws on the wall (rearing) | 1 |
| 4 paws on the wall (full climb) | 2 |

Mice consistently climbing before the injection of apomorphine will be discarded.

With full-developed apomorphine climbing, the animals are hanging onto the cage walls, rather motionless, over longer periods of time. By contrast, climbs due to mere motor stimulation usually only last a few seconds.

The climbing scores are individually totalled (maximals score: 6 per mouse over 3 readings) and the total score of the control group (vehicle intraperitoneally — apomorphine subcutaneously) is set to 100%. $ED_{50}$ values with 95% confidence limits, calculated by a Linear Regression Analysis of some of the instant 3-(piperidinyl)-1H-indazoles and 3-(pyrrolidinyl)-1H-indazoles as well as standard antipsychotics, are presented in Table 1.

TABLE 1

| Compound | Antipsychotic activity ($ED_{50}$ mg/kg) |
|---|---|
| 3-(1-methyl-4-piperidinyl)-1H-indazole | 4.5 |
| 3-(1-methyl-4-piperidinyl)-1-indazole | 46% (10 mg/kg*) |
| 1-ethyl-3-(1-methyl-4-piperidinyl)-1H-indazole | 41% (10 mg/kg*) |
| 1-acetyl-3-(1-methyl-4-piperidinyl)-1H-indazole | 5.4 |
| 1-benzoyl-3-(1-methyl-4-piperidinyl)-1H-indazole | 6.5 |
| 3-{1-[4,4-bis(4-fluorophenyl)-1-butyl]-4-piperidinyl}-1H-indazole | 7.4 |
| 1-Cyclopropylmethyl-3-(1-methyl-4-piperidinyl)-1H-indazole | 36% (10 mg/kg*) |
| haloperidol (standard) | 0.11 |
| sulpiride (standard) | 14.5 |

* decrease in climbing score at indicated dose

Antipsychotic response is achieved when the present 3-(piperidinyl)- and 3-(pyrrolidinyl)-1H-indazoles are administered to a subject requiring such treatment as an effective oral, parenteral or intravenous dose of from 0.01 to 50 mg/kg of body weight per day. A particularly preferred effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and they do not, to any extent, limit the scope or practice of the invention.

The 3-(piperidinyl)- and 3-(pyrrolidinyl)-1H-indazoles of the present invention are useful as analgetics due to their ability to alleviate pain in mammals. The analgetic utility is demonstrated in the phenyl-p-quinone writhing assay in mice, a standard assay for analgesia [Proc. Soc. Exptl. Biol. Med., *95*, 729 (1957)]. Thus, for instance, the subcutaneous dose effecting an approximately 50% inhibition of writhing ($ED_{50}$) in mice produced in this assay is as follows:

| Compound | $ED_{50}$ mg/kg |
|---|---|
| 3-(1-methyl-4-piperidinyl)-1H-indazole | 0.26 |
| 1-ethyl-3-(1-methyl-4-piperidinyl)-1-indazole | 0.65 |
| 1-acetyl-3-(1-methyl-4-piperidinyl)-1H-indazole | 0.71 |
| 1-benzoyl-3-(1-methyl-4-piperidinyl)-1H-indazole | 0.85 |
| 1-phenylmethyl-3-(1-methyl-4-piperidinyl)-1H-indazole | 75% (20 mg/kg*)<br>96% (20 mg/kg*) |
| 3-{1-[4,4-bis(4-fluorophenyl)-1-butyl]-4-piperidinyl}-1H-indazole | 31% (20 mg/kg*) |
| 1-cyclopropylmethyl-3-(1-methyl-4-piperidinyl)-1H-indazole | 1.1 |
| 1-[3-(dimethylamino)propyl]-3-(1-methyl-4-piperidinyl)-1H-indazole | 15% (20 mg/kg*) |
| 1-(4-trifluoromethyl-1-phenyl)-3-(1-methyl-4-piperidinyl)-1H-indazole | 9.8 |
| propoxyphene (standard) | 3.9 |
| pentazocine (standard) | 1.3 |

* inhibition of writhing at indicated dose

Analgesia production is achieved when the present 3-(piperidinyl)- and 3-(pyrrolidinyl)-1H-indazoles are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and that they do not, to any extent, limit the scope or practice of the invention.

Compounds of the present invention include:

(a) 3-(3-(pyrrolidinyl)-1H-indazole
(b) 3-(1-propenyl-4-piperidinyl)-1H-indazole
(c) 3-(4-cyclopropylmethyl-3-piperidinyl)-6-methyl-1-(2-pyrimidinyl)-1H-indazole
(d) 3-[1-butyl-3-pyrrolidinyl]-4-methoxy-1-(2-pyridinyl)-1H-indazole
(e) 3-(1-cyanomethyl-3-piperidinyl)-7-trifluoromethyl-1H-indazole
(f) 1-(4-fluorobenzoyl)-3-[4-piperidinyl]-5-nitro-1H-indazole
(g) 3-(1-acetyl-4-piperidinyl)-4-amino-1-(4-fluorobenzoylethyl)]-1H-indazole
(h) 3-[1-(1-methylindol-3-propyl)-3-piperidinyl]-1H-indazole
(i) 1-formyl-3-(1-formyl-4-piperidinyl)-1H-indazole
(j) 5,6-dichloro-3-[1-(4-fluorobenzoylpropyl)-4-piperidinyl]-1-(propen-1-yl)-1H-indazole
(k) 1-cyano-3-[1-(4-fluorobenzoylpropyl)-3-pyrrolidinyl]-1H-indazole ethylene ketal
(l) 1-cyanomethyl-3-[1-(6-fluoro-1,2-benzisoxazol-3-propyl)-3-piperidinyl]-1H-indazole
(m) 3-(1-phenoxycarbonyl-4-piperidinyl)-1-(4-picolinoyl)-1H-indazole
(n) 3-{1-[1,3-dihydro-3-methyl-2-oxo-2H-benzimidazol-1-ylpropyl]-4-piperidinyl}-1-(2-thiazolylcarbonyl)-1H-indazole
(o) 3-{1-(3-phenylpropyl)-4-piperidinyl}-1H-indazole
(p) 3-[1-(1-methylindol-3-propyl)-3-piperidinyl]-1H-indazole.

Effective amounts of the present invention may be administered to a subject by any one of various methods, for example, orally as in capsules or tablets, parenterally in the form of sterile solutions or suspensions, and in some cases intravenously in the form of sterile solutions. The 3-(piperidinyl)- and 3-(pyrrolidinyl)-1H-indazoles of the present invention, while effective themselves, may be formulated and administered in the form of their pharmaceutically acceptable addition salts for purposes or stability, convenience or crystallization or increased solubility.

Preferred pharmaceutically acceptable addition salts include salts of mineral acids, for example, hydrochloric acid, sulfuric acid or nitric acid, salts of monobasic carboxylic acids such as, for example, acetic acid or propionic acid, salts of dibasic acids such as, for example, maleic acid or fumaric acid, and salts of tribasic carboxylic acids such as, for example, carboxysuccinic acid or citric acid.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipients and used in the form of tablets, troches, capsules, elixirs, suspensions, syrups, wafers or chewing gums. These preparations should contain at least 0.5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 1.0—300 milligrams of the active compound.

The tablets, pills, capsules or troches may also contain the following ingredients: a binder such as microcrystalline cellulose, gum tragancanth or gelatin; an excipient such as starch or lactose, a disintegrating agent such as alginic acid or corn starch; a lubricant such as magnesium stearate, a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purposes of parenteral therapeutic administration, the active compounds of the invention may be incorporated into a solution or suspension. These preparations should contain at least 0.1% of active compound, but may be varied between 0.5 and about 50% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glykols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffer such as acetates, citrates or phosphates and agents for the adjustment of tonicity such as sodium chloride or dextrose. The parenteral preparation can be enclosed in ampules, disposable syringes or multiple dose vials made of glass or plastic.

The following examples are for illustrative purposes only and are not to be construed as limiting the invention. All temperatures are given in degrees centigrade (°C).

### Example 1

4-(2-Fluorobenzoyl)-1-methylpiperidine hydrochloride

To a suspension of 7.6 g of magnesium turnings in 25 ml of tetrahydrofuran was added a few drops of ethyl bromide, with stirring under nitrogen. After the reaction began approximately 50.0 g of N-methyl-4-chloropiperidine in 125 ml of tetrahydrofuran was added dropwise at a rate such that moderate reflux was maintained. The reaction was heated under reflux for an additional hour. A solution of 37.2 g of o-fluorobenzonitrile in 30 ml of tetrahydrofuran was added dropwise. After completion of the reaction mixture was heated under reflux for two hrs and stirred overnight at room temperature. The reaction mixture was poured into a solution of 85 g of ammonium chloride in 1200 ml of ice water and heated on a steam bath for 3 hrs. The mixture was cooled, extracted with benzene (3×, 250 ml) and dried over anhydrous sodium sulfate. Removal of the solvent under reduced pressure gave an oil. A 1.0 g-portion of the oil was dissolved in ether and a solution of etheral hydrogen chloride was added. The precipitate was collected, dried and twice recrystallized from ethanol-ether to give 0.5 g (42%) of product, mp 167—169°.

*Analysis:*

| | | | | |
|---|---|---|---|---|
| Calculated for $C_{13}H_{17}ClFNO$: | 60.58% C | 6.65% H | 5.45% N | 7.37% F |
| Found: | 60.30% C | 6.78% H | 5.43% N | 7.59% F |

### Example 2

1-Phenoxycarbonyl-4-(2-fluorobenzoyl)piperidine

To a solution of 57.5 g of 4-(2-fluorobenzoyl)-1-methyl piperidine and 68.7 g of potassium carbonate in 750 ml of toluene was added with stirring 47 g of phenylchloroformate. The reaction mixture was heated under reflux for 5 hrs, cooled to room temperature, filtered, and the solvent removed under reduced pressure to give an oil. Trituration of the oil with hexane gave 48.6 g (46%) of product. Recrystallization of 1.0 g of product from ethanol-water (2×) and from ethanol gave the analytical sample, mp 95—96°.

*Analysis:*

| | | | | |
|---|---|---|---|---|
| Calculated for $C_{19}H_{18}FNO_3$: | 69.71% C | 5.54% H | 4.28% N | 5.81% F |
| Found: | 69.45% C | 5.67% H | 4.13% N | 6.10% F |

Example 3

4-(2-Fluorobenzoyl)piperidine hydrochloride

A solution of 40.5 g of 1-phenoxycarbonyl-4-(2-fluorobenzoyl)piperidine, 500 ml of ethanol and 500 ml of 30% of potassium hydroxide solution was stirred at a temperature slightly below reflux overnight. The reaction temperature was cooled to room temperature, diluted with 250 ml of water, and the ethanol partially removed under reduced pressure. The aqueous suspension was extracted with ether (2 × 150 ml) and the ether fraction subsequently extracted with 1N hydrochloric acid (2 × 200 ml). The aqueous solution was basified with 25% sodium hydroxide solution, extracted with ether (2 × 150 ml) and dried over anhydrous sodium sulfate. The solvent was removed under reduced pressure to give an oil. The oil was dissolved in a minimum amount of ethanol and a solution of ethereal hydrogen chloride was added dropwise until a precipitate was formed. The solid was collected and dried to give 9.7 g (40%) of product. Recrystallization twice from ethanol-ether gave the analytical sample, mp 185—187°.

*Analysis:*

| | | | | |
|---|---|---|---|---|
| Calculated for $C_{12}H_{15}ClFNO$: | 59.14% C | 6.20% H | 5.74% N | 7.80% F |
| Found: | 58.90% C | 6.36% H | 5.50% N | 7.56% F |

Example 4

3-(1-Methyl-4-piperidinyl)-1H-indazole

An autoclave was charged with 10.0 g of 4-(2-fluorobenzoyl)-1-methylpiperidine and 14 ml of hydrazine hydrate. The reaction was heated at 150° for 20 hrs, cooled, and poured into water. The resultant solid was collected. The solid was recrystallized twice from toluene to yield 2.4 g (23.7%) of product, mp 168—170°.

*Analysis:*

| | | | |
|---|---|---|---|
| Calculated for $C_{13}H_{17}N_3$: | 72.52% C | 7.96% H | 19.52% N |
| Found: | 72.60% C | 8.04% H | 19.51% N |

Example 5

3-{1-[4,4-bis(4-Fluorophenyl)-1-butyl]-4-piperidinyl]-1H-indazole

A mixture of 10.0 g of 4-(2-fluorobenzoyl)piperidine, 16.9 g of potassium carbonate, 28.8 g of 4-chloro-1,1-bis(4-fluorophenyl)butane, 250 ml of dimethylformamide and a few crystals of potassium iodide was heated at 90° for 8 hrs with stirring. The reaction mixture was poured into water and extracted with ethyl acetate. The ethyl acetate extract was washed with water, dried over anhydrous magnesium sulfate and the solvent removed *in vacuo* to yield an oil. The oil was dissolved in ether, and oxalic acid was added. Recrystallization of the resultant solid from ethanol gave 10.6 g (34.6%) of 1-[4,4-bis(4-fluorophenyl)-1-butyl]-4-(2-fluorobenzoyl)piperidine oxalate, mp 179—181°.

A solution of 8.0 g of 1-[4,4-bis(4-fluorophenyl)-1-butyl]-4-(2-fluorobenzoyl)-piperidine and 110 ml of hydrazine hydrate was heated at 150° in an autoclave for 20 hrs, with stirring. The resultant solid was diluted with water, and the mixture extracted with dichloromethane. The dichloromethane was evaporated *in vacuo* to yield a solid. The solid was recrystallized from toluene to give 3.8 g (47.4%) of product, mp 154—156°.

*Analysis:*

| | | | |
|---|---|---|---|
| Calculated for $C_{28}H_{29}F_2N_3$: | 75.48% C | 6.56% H | 9.43% N |
| Found: | 75.29% C | 6.59% H | 9.40% N |

Example 6

1-Ethyl-3-(1-methyl-4-piperidinyl)-1H-indazole hydrobromide

To a stirred suspension of 0.86 g of sodium hydride (50% oil dispersion) in 50 ml of dimethylformamide was added, dropwise, 3.0 g of 3-(1-methyl-4-piperidinyl)-1H-indazole in 15 ml of hot dimethylformamide. The reaction mixture was stirred at ambient temperature and 1.34 ml of ethyl bromide in 10 ml of dimethylformamide was added, dropwise. The reaction was stirred overnight at ambient temperature and poured into water. The aqueous suspension was extracted with ethyl acetate (2×, 100 ml). The extracts were combined, washed with water, dried over anhydrous magnesium sulfate, and the solvent evaporated *in vacuo* to yield an oil. The oil was dissolved in a minimum amount of ethanol-ether, and a saturated solution of ether-hydrogen bromide was added. The salt was recrystallized from ethanol-ether to yield 2.4 g (52.8%) of product, mp 240—242°.

*Analysis:*

| | | | |
|---|---|---|---|
| Calculated for $C_{15}H_{21}N_3HBr$: | 55.56% C | 6.84% H | 12.96% N |
| Found: | 55.39% C | 6.68% H | 12.59% N |

Example 7

1-[4-Nitro-1-phenyl]-3-(1-methyl-4-piperidinyl)-1H-indazole hydrochloride

To a stirred suspension, under nitrogen, of 0.86 g of sodium hydride (50% oil dispersion) was added, dropwise, 3.2 g of 3-(1-methyl-4-piperidinyl)-1H-indazole dissolved in 25 ml of hot dimethylformamide. After completion of the addition, the reaction mixture was stirred at ambient temperature for 1 hr and then 2.2 g of 1-fluoro-4-nitrobenzene was added, dropwise. The reaction mixture was stirred at ambient temperature for 16 hr and then poured into water. The resultant solid was collected and dried. The solid

was dissolved in absolute ethanol and the solution was treated with saturated hydrogen chloride/ether solution to precipitate a salt. Recrystallization of the salt from methanol-ether (twice) gave 3.0 g (52%) of product, mp 272—274°.

*Analysis:*

| | | | |
|---|---|---|---|
| Calculated for $C_{19}H_{20}N_4O_2 \cdot HCl$: | 61.20% C | 5.68% H | 15.03% N |
| Found: | 61.09% C | 5.70% H | 14.82% N |

Example 8

1-[3-(Dimethylamino)propyl]-3-(1-methyl-4-piperidinyl)-1H-indazole difumarate

To a stirred suspension of 1.15 g of sodium hydride (50% oil dispersion) in 30 ml of dimethylformamide, under nitrogen, was added dropwise 4.3 g of 3-(1-methyl-4-piperidinyl)-1H-indazole dissolved in 30 ml of hot dimethylformamide. After stirring at ambient temperature for 1 hr, a solution of 3.3 g of dimethylaminopropylchloride in 30 ml of toluene was added dropwise. The reaction was then heated at 60° for 1 hr and then stirred at ambient temperature for 16 hrs. The reaction mixture was poured into water and the aqueous suspension was extracted with ethyl acetate (3× 150 ml). The organic extracts were combined, washed with water, brine, dried over anhydrous magnesium sulfate, and the solvent evaporated *in vacuo* to yield an oil. The oil was dissolved in 25 ml of acetonitrile and 4.6 g of fumaric acid was added. The mixture was warmed on the steam bath (ca. 15 min), and then left at ambient temperature for 15 hrs. The resultant solid was filtered and recrystallized from dimethylformamide (thrice) with cooling (ca. 5°) to yield .1.4 g (18.7%) of product, mp 161—163°.

*Analysis:*

| | | | |
|---|---|---|---|
| Calculated for $C_{18}H_{28}N_4 \cdot 2C_4H_4O_4$: | 58.61% C | 6.81% H | 10.52% N |
| Found: | 58.82% C | 6.91% H | 11.51% N |

Example 9

1-(4-Trifluoromethyl-1-phenyl)-3-(1-methyl-4-piperidinyl)-1H-indazole hydrochloride

To a stirred suspension of 1.15 g of sodium hydride (50% oil dispersion) in 30 ml of dimethylformamide, under nitrogen, was added dropwise 4.3 g of 3-(1-methyl-4-piperidinyl)-1H-indazole dissolved in 30 ml of hot dimethylformamide. The reaction mixture was stirred at ambient temperature for 1 hr and then 3.9 g of 4-fluorobenzotrifluoride was added. The temperature was raised to 90° and held there for 16 hrs. The reaction mixture was poured into water and the aqueous suspension extracted with ethyl acetate (2×, 150 ml). The extracts were combined, washed with water, brine, dried over anhydrous magnesium sulfate and the solvent removed *in vacuo* to yield an oil. The oil was dissolved in ether and saturated ether-hydrogen chloride solution was added to precipitate a salt. Recrystallization (twice) from isopropanol-ether gave 3.3 g (41.7%) of product, mp 221—223°.

*Analysis:*

| | | | |
|---|---|---|---|
| Calculated for $C_{20}H_{20}F_3N_3 \cdot HCl$: | 60.68% C | 5.34% H | 10.62% N |
| Found: | 60.86% C | 5.55% H | 10.62% N |

Example 10

1-Hydroxymethyl-3-(1-methyl-4-piperidinyl)-1H-indazole

A solution of 0.52 g of paraformaldehyde, 3.0 g of 3-(1-methyl-4-piperidinyl)-1H-indazole, 15 ml of ethanol and 0.34 ml of 5% aqueous sodium hydroxide was refluxed for 3 hrs. The ethanol was removed *in vacuo* and the residue diluted with water. The aqueous suspension was extracted with dichloromethane (2×, 50 ml) and the combined extracts were washed with water and dried over anhydrous magnesium sulfate. The dichloromethane was evaporated *in vacuo* to yield an oil, which crystallized upon standing. The solid was recrystallized twice from ethyl acetate to yield 1.3 g (57.8%) of product, mp 128—130°.

*Analysis:*

| | | | |
|---|---|---|---|
| Calculated for $C_{14}H_{19}N_3O$: | 68.54% C | 7.81% H | 17.13% N |
| Found: | 68.41% C | 7.81% H | 17.24% N |

Example 11

1-(6-Fluoro-1,2-benzisoxazole-3-propyl)-3-(1-methyl-4-piperidinyl)-1H-indazole maleate

To a stirred suspension under nitrogen of 0.8 g of sodium hydride (50% oil dispersion) in 45 ml of dimethylformamide, was added dropwise, 3.0 g of 3-(1-methyl-4-piperidinyl)-1H-indazole dissolved in 50 ml of hot dimethylformamide. The reaction mixture was stirred at ambient temperature for 45 min and then 3.9 g of 3-3-chloroprop-1-yl)-6-fluoro-1,2-benzisoxazole was added. The reaction mixture was stirred at ambient temperature overnight (ca. 16 hr) and then poured into water. The aqueous suspension was extracted with ether and the ether extract was washed with water, dried over anhydrous magnesium sulfate, and filtered. Treatment of the etheral solution with 2.0 g of maleic acid suspended in 10 ml of ethanol resulted in an oil. Decantation of the supernatant ether solution and subsequent trituration of the oil will ethyl acetate gave a solid. Recrystallization of the solid from ethyl acetate yielded 3.0 g (43%) of product, mp 127—129°.

*Analysis:*

| | | | |
|---|---|---|---|
| Calculated for $C_{23}H_{25}FN_4O \cdot C_4H_4O_4$: | 63.77% C | 5.75% H | 11.02% N |
| Found: | 63.53% C | 5.71% H | 11.15% N |

Example 12

1-Cyclopropylmethyl-3-(1-methyl-4-piperidinyl)-1H-indazole hydrobromide

To a stirred mixture of 1.19 g of sodium hydride (50% oil dispersion) in 65 ml of dimethylformamide was added, dropwise, 4.0 g of 3-(1-methyl-4-piperidinyl)-1H-indazole in 20 ml of dimethylformamide. The mixture was stirred for 1 hr at ambient temperature and then 1.40 g of chloromethylcyclopropane in 10 ml of dimethylformamide was added dropwise. The reaction mixture was stirred 2½ days at ambient temperature. The reaction mixture was quenched with water, extracted with ethyl acetate, washed with water, dried over anhydrous magnesium sulfate and the solvent was removed *in vacuo* to yield an oil. The oil was dissolved in ether and saturated hydrogen bromide/ether solution was added dropwise to precipitate a salt. The salt was recrystallized from toluene and ethyl acetate to yield 2.8 g (32%) of product, mp 177—179°C.

*Analysis:*

| | | | |
|---|---|---|---|
| Calculated for $C_{17}H_{23}N_3\cdot HBr$: | 58.29% C | 6.86% H | 12.00% N |
| Found: | 58.20% C | 6.59% H | 11.82% N |

Example 13

1-Phenylmethyl-3-(1-methyl-4-piperidinyl)-1H-indazole-hydrobromide

To a stirred suspension of 0.86 g of sodium hydride (50% oil dispersion) in 50 ml of dimethylformamide under nitrogen, was added, dropwise, 3.0 g of 3-(1-methyl-4-piperidinyl)-1H-indazole dissolved in 15 ml of hot dimethylformamide. After stirring one hr at ambient temperature, 2.6 g of benzyl bromide in 5 ml of dimethylformamide was added. The reaction mixture was stirred at ambient temperature for 15 hrs and then poured into water. The aqueous mixture was extracted with ethyl acetate. The ethyl acetate extract was washed with water, brine and dried over anhydrous magnesium sulfate. The ethyl acetate was evaporated *in vacuo* to yield an oil. The oil was dissolved in ether and gaseous hydrogen bromide was passed through the solution to yield an oily material. The oily material was triturated with boiling ethyl acetate. The solid was collected and recrystallized from absolute ethanol to yield 2.4 g (44.3%) of product, mp 218—220°.

*Analysis:*

| | | | |
|---|---|---|---|
| Calculated for $C_{20}H_{23}N_3\cdot HBr$: | 62.18% C | 6.00% H | 10.88% N |
| Found: | 62.13% C | 6.24% H | 10.87% N |

Example 14

1-Phenethyl-3-(1-methyl-4-piperidinyl)-1H-indazole hydrobromide

To a stirred mixture of 0.86 g of sodium hydride (50% oil dispersion) in 50 ml of dimethylformamide was added, dropwise, 3.0 g of 3-(1-methyl-4-piperidinyl)-1H-indazole in 15 ml of hot dimethylformamide. The reaction mixture was stirred at ambient temperature for 1 hr and then a solution of 3.33 g of (2-bromoethyl)benzene in 10 ml of dimethylformamide was added dropwise. The reaction mixture was stirred for 2½ days at ambient temperature, cooled to 0°, and water was added dropwise. The mixture was extracted with ethyl acetate, washed with water, dried over anhydrous magnesium sulfate and the solvent was removed *in vacuo* to yield an oil. The oil was dissolved in ether and a saturated bromide/ether solution was added dropwise. The resultant solid was recrystallized two times from ethanol to yield 2.0 g (36%) of product, mp 163—164°.

*Analysis:*

| | | | |
|---|---|---|---|
| Calculated for $C_{21}H_{25}N_3\cdot HBr$: | 63.00% C | 6.50% H | 10.50% N |
| Found: | 62.93% C | 6.58% H | 10.55% N |

Example 15

1-Acetyl-3-(1-methyl-4-piperidinyl)-1H-indazole hydrochloride

A mixture of 2.0 g of 3-(1-methyl-4-piperidinyl)-1H-indazole, 20 ml of acetic anhydride and a few drops of pyridine was stirred under reflux for 2 hrs. Most acetic anhydride was evaporated *in vacuo* and the resultant solution was poured into 80 ml of water. The solution was made basic with ammonium hydroxide solution. A white solid precipitated. The solid was collected, dried and dissolved in ether. Saturated ether-hydrogen chloride solution was added. The salt was reprecipitated from cold methanol with ether to yield 1.6 g (60.5%) of product, mp 238—239°.

*Analysis:*

| | | | |
|---|---|---|---|
| Calculated for $C_{15}H_{19}N_3O\cdot HCl$: | 61.32% C | 6.86% H | 14.30% N |
| Found: | 61.08% C | 6.76% H | 14.32% N |

Example 16

1-Benzoyl-3-(1-methyl-4-piperidinyl)-1H-indazole hydrochloride

A mixture of 3.0 g of 3-(1-methyl-4-piperidinyl)-1H-indazole and 3.2 ml of benzoyl chloride was heated at 100° for 2 hrs. The resultant solid was triturated with ether and collected. The salt was twice recrystallized from ethanol-ether to yield 3.1 g (62.2%) of product, mp 234—236°.

*Analysis:*

| | | | |
|---|---|---|---|
| Calculated for $C_{20}H_{21}N_3O\cdot HCl$: | 67.50% C | 6.23% H | 11.81% N |
| Found: | 67.22% C | 6.15% H | 11.82% N |

Example 17

3-(1-Methyl-4-piperidinyl)-1-trichloroethoxycarbonyl-1H-indazole hydrochloride

A mixture of 2.3 g of trichloroethyl chloroformate, 3.0 g of potassium carbonate, 2.1 g of 3-(1-methyl-4-piperidyl)-1H-indazole and chloroform was heated under reflux for 16 hrs. The reaction mixture was cooled, filtered, and the filtrate was concentrated to a solid *in vacuo*. The solid was triturated with ethyl acetate, collected, and recrystallized from ethanol to yield 3.9 g (91%) of product, mp 190—192°.

*Analysis:*

| | | | |
|---|---|---|---|
| Calculated for $C_{16}H_{18}Cl_3N_3O_2 \cdot HCl$: | 44.99% C | 4.48% H | 9.85% N |
| Found: | 44.64% C | 4.46% H | 9.84% N |

Example 18

1-Phenylsulfonyl-3-(1-methyl-4-piperidinyl)-1H-indazole hydrochloride

A mixture of 2.5 g of 3-(1-methyl-4-piperidinyl)-1H-indazole and 50 ml of benzenesulfonyl chloride was heated on a steam bath for 1 hr. The solution was cooled to ambient temperature and then poured into ether. The precipitate was collected and triturated with ethyl acetate to yield a solid. The solid was combined with 1.5 g of solid from another experiment and recrystallized twice from isopropanol to yield 2.3 g (36%) of product, mp 222—224°.

*Analysis:*

| | | | |
|---|---|---|---|
| Calculated for $C_{19}H_{21}N_3O_2S$: | 58.24% C | 5.66% H | 10.72% N |
| Found: | 58.17% C | 5.77% H | 10.80% N |

Example 19

1-(4-Fluorobenzoyl)-3-(1-methyl-4-piperidinyl)-1H-indazole

A solution of 3.0 g of 3-(1-methyl-4-piperidinyl)-1H-indazole in 15 ml of 4-fluorobenzoyl chloride was heated at 100° for 2 hrs. The reaction mixture was poured into ether and the precipitate was collected. The precipitate was treated with dilute aqueous sodium hydroxide solution and extracted with chloroform. The chloroform extract was dried over anhydrous magnesium sulfate and the solvent removed *in vacuo* to yield 2.5 g (53%) of product, mp 132—133°.

*Analysis:*

| | | | |
|---|---|---|---|
| Calculated for $C_{20}H_{20}FN_3O$: | 71.20% C | 5.98% H | 12.45% N |
| Found: | 71.42% C | 6.21% H | 12.50% N |

Example 20

1-Ethoxycarbonyl-3-(1-methyl-4-piperidinyl)-1H-indazole hydrochloride

To a stirred solution of the 3.5 g of 3-(1-methyl-4-piperidinyl)-1H-indazole, 2.7 g of potassium carbonate and 40 ml of chloroform was added, dropwise, 21.1 g of ethyl chloroformate. The reaction mixture was stirred under reflux for 16 hrs, cooled, filtered, and the filtrate was concentrated *in vacuo*. The residue was triturated in ether. Recrystallization from ethanol-ether and then from isopropyl alcohol-ether gave 2.3 g (43.8%) of product, mp 181—183°C (gas evolution).

*Analysis:*

| | | | |
|---|---|---|---|
| Calculated for $C_{16}H_{21}N_3OHCl$: | 59.34% C | 6.85% H | 12.98% N |
| Found: | 58.76% C | 7.05% H | 12.80% N |

Example 21

1-(4-Methoxybenzoyl)-3-(1-methyl-4-piperidinyl)-1H-indazole hydrochloride

A solution of 3.0 g of 3-(1-methyl-4-piperidinyl)-1H-indazole in 10 ml of p-jb coyl chloride was heated at 100°C for 3 hr. The reactions mixture was cooled to ambient temperature, ether was added and the solid was collected. The solid, suspended in ether, was stirred for 16 hr. The mixture was filtered and dried. Recrystallization from ethanol (twice) yielded 4.0 g (74.4%) of product, mp 235—237°C.

*Analysis:*

| | | | |
|---|---|---|---|
| Calculated for $C_{21}H_{23}N_3O_2 \cdot HCl$: | 65.36% C | 6.27% H | 10.89% N |
| Found: | 64.83% C | 6.16% H | 10.82% N |

Example 22

1-(2-Chlorophenyl)-3-(1-methyl-4-piperidinyl)-1H-indazole fumarate

To a stirred suspension of 1.1 g of sodium hydride 50% oil dispersion) in 40 ml of dimethylformamide under nitrogen was added, dropwise, 4.0 g of 3-(1-methyl-4-piperidinyl)-1H-indazole in 25 ml of hot dimethylformamide. The reaction was stirred at ambient temperature for 1 hr and then 3.6 g of 1-chloro-2-fluorobenzene was added. The temperature was then raised to 120° and held at 120° for 20 hrs. The reaction was poured into water and the aqueous suspension was extracted with ether. The ether extract was washed with water, dried over anhydrous magnesium sulfate, and the ether was removed *in vacuo*. The residue was chromatographed on a Water's preparative high-pressure liquid chromatograph using a silica gel column eluted with tetrahydrofuran-$(C_2H_5)_2NH$ (99:1). Evaporation of the appropriate fractions yielded an oil. The oil was dissolved in ether and 1.5 g of fumaric acid was added. The mixture was stirred at ambient temperature for 16 hrs, and the solid was collected. Recrystallization from isopropyl alcohol-ether

and then from ethanol-ether gave 2.5 g (31.4%) of product, mp 179—181°C.

*Analysis:*

| | | | |
|---|---|---|---|
| Calculated for $C_{19}H_{20}ClN_3 \cdot C_4H_4O_4$: | 62.51% C | 5.47% H | 9.51% N |
| Found: | 62.46% C | 5.50% H | 9.43% N |

Example 23

1-Benzoyl-3-(1-cyano-4-piperidinyl)-1H-indazole

A sample of 8.4 g of 1-benzoyl-3-(1-methyl-4-piperidinyl)-1H-indazole hydrochloride was converted to its free base and the base was dissolved in 135 ml of chloroform. The solution was stirred and 4.0 g of potassium carbonate was added followed by 2.7 g of cyanogen bromide. The reaction mixture was stirred under reflux for 16 hrs. The mixture was filtered, and the solvent was removed *in vacuo*. The mixture was chromatographed on a high-pressure column chromatography apparatus (silica gel), eluting with 0.3% methanol/dichloromethane. Evaporation of the appropriate fraction followed by recrystallization from toluene hexane yielded 2.2 g (28%) of the product, mp 144—146°.

*Analysis:*

| | | | |
|---|---|---|---|
| Calculated for $C_{20}H_{18}N_4O$: | 72.71% C | 5.59% H | 16.97% N |
| Found: | 72.56% C | 5.57% H | 17.18% N |

Example 24

6-Fluoro-3-(4-piperidinyl)-1H-indazole

A solution of 20.0 g of 1-acetyl-4-(2,4-difluorobenzoyl)-piperidine, 60 ml of hydrazine hydrate and 150 ml of n-butanol was refluxed for 48 hrs. The reaction was poured into water, and the mixture was extracted with ethyl acetate. The ethyl acetate extract was washed with water, dried over anhydrous magnesium sulfate and the solvent was evaporated *in vacuo*. Recrystallization of the residue from isopropanol-water and then from isopropanol yielded 3.3 g (20.1%) of product, mp 214—216°.

*Analysis:*

| | | | |
|---|---|---|---|
| Calculated for $C_{12}H_{14}N_3F$: | 65.73% C | 6.44% H | 19.17% N |
| Found: | 65.55% C | 6.35% H | 19.30% N |

Example 25

1-(2-Furoyl)-3-(1-methyl-4-piperidinyl)-1H-indazole hydrochloride

A mixture of 4.0 g of 3-(4-piperidinyl)-1H-indazole, 2.7 mol of 2-furoyl chloride (95% pure) and 60 ml of chloroform was refluxed for 3 hrs. The mixture was filtered. The filtrate was evaporated under reduced pressure, the residue was triturated with ethyl acetate and the solid was collected. The solid and filtrate were combined and recrystallized twice from ethanol to yield 3.3 g (51.5%) of product, mp 268—270°C.

*Analysis:*

| | | | |
|---|---|---|---|
| Calculated for $C_{18}H_{19}N_3O_2 \cdot HCl$: | 62.51% C | 5.83% H | 12.15% N |
| Found: | 62.78% C | 6.00% H | 12.11% N |

Example 26

1-(4-Chlorophenyl)-3-(1-methyl-4-piperidinyl)-1H-indazole furmarate

To a stirred suspension of 1.1 g of sodium hydride (50% oil dispersion) in 40 ml of dimethylformamide was added, dropwise, under nitrogen, 4.0 g of 3-(1-methyl-4-piperidinyl)-1H-indazole dissolved in 25 ml of hot dimethylformamide. The reaction was stirred at ambient temperature for 45 min, and 2.9 ml of 4-fluorochlorobenzene was added. The temperature was increased to 120° and the reaction proceeded at this temperature for 24 hr. The reaction was poured into water and the aqueous mixture was extracted with ethyl acetate. The ethyl acetate extract was washed with water, dried over anhydrous magnesium sulfate and evaporated *in vacuo*. The residue was heated (steam bath) with 3N hydrochloric acid and the salt was collected. The salt was immediately recrystallized from water. The wet salt was treated with ammonium hydroxide and the base was extracted into dichloromethane. Evaporation of the dichloromethane *in vacuo* gave an oil. The oil solidified on standing. The solid was dissolved in ether and 1.4 g of fumaric acid was added. Recrystallization from 2-propanol gave 3.9 g (47.5%) of product, mp 176—178°.

*Analysis:*

| | | | |
|---|---|---|---|
| Calculated for $C_{19}H_{20}ClN_3O \cdot C_4H_4O_4$: | 62.25% C | 5.47% H | 9.51% N |
| Found: | 62.32% C | 5.40% H | 9.56% N |

Example 27

6-Fluoro-3-[1-(prop-1-yl)-4-piperidinyl]-1H-indazole

A mixture of 3.0 g of 6-fluoro-2-(4-piperidinyl)-1H-indazole, 1.8 g of 1-bromopropane, 2.5 g of sodium bicarbonate and 30 ml of dimethylformamide was stirred and heated at 60° for 2 hr. After stirring at ambient temperature for 14 hrs, the reaction was poured into water and the aqueous suspension was extracted with ethyl acetate. The ethyl acetate was washed with water, dried over anhydrous magnesium sulfate and the solvent was removed *in vacuo*. Recrystallization of the residue from ethyl acetate yielded 2.3 g (63%) of product. This material was combined with a sample from another experiment and recrystallized from ethyl acetate to yield the analytical sample, mp 175—177°.

*Analysis:*

| | | | |
|---|---|---|---|
| Calculated for $C_{15}H_{20}FN_3$: | 68.94% C | 7.71% H | 16.08% N |
| Found: | 69.24% C | 7.62% H | 16.15% N |

Example 28

6-Fluoro-2-(1-pheneth-2-yl-4-piperidinyl)-1H-indazole

A mixture of 5.0 g of 6-fluoro-3-(4-piperidinyl)-1H-indazole, 4.6 g of (2-bromoethyl)benzene, 4.2 g of sodium bicarbonate and 50 ml of dimethylformamide was stirred at 60° for 3 hrs and at ambient temperature for 15 hrs. The reaction was poured into water and the aqueous mixture extracted with ethyl acetate. The ethyl acetate was washed with water, dried over anhydrous magnesium sulfate and the solvent was removed *in vacuo*. The residue was recrystallized twice from 2-propanol (one charcoal treatment) to yield 2.6 (35%) of product, mp 163—165°.

*Analysis:*

| | | | |
|---|---|---|---|
| Calculated for $C_{22}H_{22}FN_3$: | 74.27% C | 6.86% H | 12.99% N |
| Found: | 74.45% C | 6.96% H | 13.49% N |

Example 29

1-Benzoyl-3-(1-pheneth-2-yl-4-piperidinyl)-1H-indazole hydrochloride

A mixture of 1.6 g of 3-(1-pheneth-2-yl-4-piperidinyl)-1H-indazole and 7 ml of benzoyl chloride was heated at 100° for 2 hrs. After cooling, ether was added and the solid was collected. The solid was combined with material from a prior experiment and recrystallized from methanol to yield 2.4 g (51%) of product, mp 248—250.

*Analysis:*

| | | | |
|---|---|---|---|
| Calculated for $C_{27}H_{26}FN_3O \cdot HCl$: | 69.89% C | 5.86% H | 9.06% N |
| Found: | 70.03% C | 5.96% H | 9.22% N |

Example 30

1-(3-Chlorobenzoyl)-3-(1-methyl-4-piperidinyl)-1H-indazole hydrochloride

A mixture of 3.0 g of 3-(1-methyl-4-piperidinyl)-1H-indazole and 8 ml of 3-chlorobenzoyl chloride was heated at 100° for 2 hrs. The reaction was allowed to cool, and ether was added. The resultant solid was collected and recrystallized twice from isopropyl alcohol to yield 3.7 g (67.7%) of product, mp 209—211°.

*Analysis:*

| | | | |
|---|---|---|---|
| Calculated for $C_{20}H_{20}ClN_3O \cdot HCl$: | 61.54% C | 5.42% H | 10.77% N |
| Found: | 61.54% C | 5.57% H | 10.66% N |

Example 31

1-(2-Chlorobenzoyl)-3-(1-methyl-4-piperidinyl)-1H-indazole-fumarate

A mixture of 3.0 g of 3-(4-piperidinyl)-1H-indazole and 8 ml of 2-chlorobenzoyl chloride was heated at 100° (steam bath) for 2 hrs. The reaction was cooled to ambient temperature, and ether was added. The oil, which separated, solidified upon scratching. The solid was treated with ammonium hydroxide and the precipitate was dissolved in ether ethanol (350:10 ml). Fumaric acid (1.4 g) was added, and the mixture was stirred at ambient temperature for 4 hrs. The resultant solid was collected, dried, triturated with hot acetone, and recrystallized from isopropyl alcohol to yield 3.6 g (54.7%) of the product, mp 182—184°.

*Analysis:*

| | | | |
|---|---|---|---|
| Calculated for $C_{20}H_{20}ClN_3O \cdot C_4H_4O_4$: | 61.34% C | 5.15% H | 8.94% N |
| Found: | 61.54% C | 5.30% H | 8.96% N |

Example 32

6-Fluoro-3-(1-methyl-4-piperidinyl)-1H-indazole

To a stirred suspension, under nitrogen, of 4.7 g of lithium aluminum hydride (50—55% oil dispersion) in 90 ml of tetrahydrofuran was added, dropwise, 8.9 g of 6-fluoro-3-(1-methoxycarbonyl-4-piperidinyl)-1H-indazole, dissolved in 50 ml of tetrahydrofuran. After the addition was complete, the reaction was heated under reflux for 2 hrs. The reaction was cooled in an ice-salt bath and water was carefully added. The mixcture was filtered and the filter cake was washed with tetrahydrofuran and twice with hot methanol. The filtrate was concentrated *in vacuo*. The residue was triturated with ethyl acetate to yield 5.1 g (68%) of product, mp 217—220°. Recrystallization from ethanol (twice) provided the analytical sample, mp 218—220°.

*Analysis:*

| | | | |
|---|---|---|---|
| Calculated for $C_{13}H_{16}FN_3$: | 66.93% C | 6.91% H | 18.01% N |
| Found: | 67.08% C | 6.97% H | 18.09% N |

Example 33

1-(2-Nitrophenyl)-3-(1-methyl-4-piperidinyl)-1H-indazole fumarate

To a stirred suspension, under nitrogen, of 0.86 g (0.018 mole) of 50% oil dispersion of sodium hydride in 20 ml of dimethylformamide was added, dropwise, 3.2 g (0.015 mole) of 3-(1-methyl-4-piperidinyl)-1H-indazole dissolved in 25 ml of hot dimethylformamide. Upon completion of the addition, the reaction mixture was stirred at ambient temperature for one hr and a solution of 2.2 g (0.0115 mole) of 1-fluoro-2-nitrobenzene in 5 ml of dimethylformamide was added slowly. The reaction mixture was stirred at ambient temperature for 2 hrs and then poured into water. The precipitate was collected and then dissolved in ethanol-ether. To the stirred solution, 1.7 g of fumaric acid was added. After stirring at ambient temperature for four hrs, the mixture was filtered and the filter cake was recrystallized from methanol-ether (twice) to yield 4.3 g (67.8%) of product, mp 210—212°C.

*Analysis:*

| | | | |
|---|---|---|---|
| Calculated for $C_{19}H_{20}N_4O_2 \cdot C_4H_4O_4$: | 61.05% C | 5.35% H | 12.38% N |
| Found: | 61.08% C | 5.43% H | 12.47% N |

Example 34

1-(2-Chlorophenyl)-3-(4-piperidinyl)-1H-indazole fumarate

To a stirred mixture of 4.2 g (0.04 mole) of cyanogen bromide, 6.3 g of potassium carbonate and 125 ml of dimethylsulfoxide was added, dropwise, 12.0 g (0.037 mole) of 1-(2-chloropohenyl)-3-(1-methyl-4-piperidinyl)-1H-indazole in 30 ml of dimethylsulfoxide. The reaction mixture was stirred at ambient temperature for two hrs and then poured into water. The aqueous mixture was extracted with ethyl acetate. The ethyl acetate extracts were washed with water, dried over anhydrous magnesium sulfate, and the solvent was removed in *vacuo* to give an oil. The oil was triturated with ether to give 7.1 g (57%) of 1-(2-chlorophenyl)-3-(1-cyano-4-piperidinyl)-1H-indazole, mp 109—111°C.

A stirred mixture of 6.5 g (0.0019 mole) of 1-(2-chlorophenyl)-3-(1-cyano-4-piperidin yl)-1H-indazole and 60 ml of 25% aqueous sulfuric acid was heated under reflux for 16 hrs. The reaction mixture was cooled in an ice-bath and 50% aqueous sodium hydroxide solution was added dropwise until the mixture was basic. The aqueous mixture was extracted with ethyl acetate. The extracts were washed with water, dried over anhydrous magnsium sulfate and the solvent removed *in vacuo* to give an oil. The oil was dissolved in ethanol-ether and 2.2 g of fumaric acid was added. After stirring at ambient temperature for 6 hrs, the salt was collected. The salt was recrystallized twice from methanol-ether to yield 2.,4 g (30%) of product, mp 213—215°C.

*Analysis:*

| | | | |
|---|---|---|---|
| Calculated for $C_{18}H_{18}ClN_3 \cdot C_4H_4O_4$: | 61.75% C | 5.18% H | 9.82% N |
| Found: | 61.55% C | 5.48% H | 9.62% N |

Example 35

1-(2-Aminophenyl)-3-(1-methyl-4-piperidinyl)-indazole fumarate

To a cooled (5°C), stirred solution of 9.5 g (0.042 mole) of stannous chloride dihydrate in 20 ml of conc. hydrochloric acid and 10 ml of tetrahydrofuran was added, dropwise, 3.6 g (0.01 mole) of 1-(2-nitrophenyl)-3-(1-methyl-4-piperidinyl)-1H-indazole dissolved in 25 ml of tetrahydrofuran. After completion of the addition, the cooling bath was removed and the reaction mixture was stirred at ambient temperature for 3 hrs. The reaction mixture was poured into ice water and made basic with 10% sodium hydroxide solution. The mixture was extracted with ethyl acetate. The extract was washed with water, dried over anhydrous magnesium sulfate and the solvent was removed *in vacuo*. The residue was dissolved in ethanol (warming) and 2.0 g of fumaric acid was added. The mixture was then warmed on the steam bath for a few mins. The salt was collected to give 3.4 g (80%) of product. The salt was combined with 1.1 g of another experiment and recrystallized first from dimethylformamide-ethyl acetate (twice) and then from methanol-ether to yield the analytical sample, mp 208—210°c.

*Analysis:*

| | | | |
|---|---|---|---|
| Calculated for $C_{19}H_{22}N_4 \cdot C_4H_4O_4$: | 65.40% C | 6.21% H | 13.24% N |
| Found: | 65.46% C | 6.40% H | 13.35% N |

Example 36

1-(2-Fluorophenyl)-3-(1-methyl-4-piperidinyl)-1H-indazole hydrochloride

To a stirred suspension of 1.0 g (0.021 mole) of a 50% oil dispersion of sodium hydride in 20 ml of dimethylformamide, under nitrogen, was added, dropwise, 3.2 g (0.0125 mole) of 3-(1-methyl-4-piperidinyl)-1H-indazole dissolved in 25 ml of hot dimethylformamide. After ageing at ambient temperature for 45 mins, 3.6 g (0.032 mole) of 1,2-difluorobenzene was added and the temperature was raised to 120°C and held there for 6 hrs. The reaction mixture was quenched with water and the aqueous mixture extracted with ether. The ether extract was washed with water dried over anhydrous magnesium sulfate and solvent removed *in vacuo* to yield an oil. The oil was combined with 2.7 g of oil from another experiment and the combined sample (6.9 g) was chromatographed. Flash chromatography was utilized with 210 g of silica gel and 10% ethyl acetate-diethylamine as the eluent. Evaporation of the appropriate fractions gave 4.3 g (57%) of product, as an oil. Treatment of the oil with ethereal hydrogen chloride gave the hydrochloride salt. The salt was recrystallized from ethanol-ether and then from acetonitrile to yield the analytical sample, mp 215—217°C.

*Analysis:*

| | | | |
|---|---|---|---|
| Calculated for $C_{19}H_{20}FN_3O \cdot HCl$: | 65.98% C | 6.11% H | 12.16% N |
| Found: | 65.79% C | 6.16% H | 12.21% N |

Example 37

1-(3-Fluorophenyl)-3-(1-methyl-4-piperidinyl)-1H-indazole hydrochloride

To a suspension of 2.0 g (0.042 mole) of a 50% oil dispersion of sodium hydride in 40 ml of dimethylformamide was added, dropwise, 6.4 g (0.030 mole) of 3-(1-methyl-4-piperidinyl)-1H-indazole in 50 ml of hot dimethylformamide. After stirring for 40 mins at ambient temperature, 7.2 g (0.063 mole) of 1,3-difluorobenzene was added. The mixture was heated at 120°C for 20 hrs, cooled and poured into water. The mixture was extracted with ether. The extracts were dried over anhydrous magensium sulfate and concentrated to yield an oil. The oil was purified using flash chromatography (silica gel, diethylamine-ethyl acetate, 1:9, $R_f = 0.43$). Evaporation of appropriate fractions gave an oil. The oil was dissolved in anhydrous ether and hydrogen chloride gas was bubbled into the solution. The ether was decanted and the solid was recrystallized twice from ethanol-ether to yield 2.6 g (25%) of product, mp 254—256°C.

*Analysis:*

| | | | |
|---|---|---|---|
| Calculated for $C_{19}H_{20}FN_3 \cdot HCl$: | 65.99% C | 6.12% H | 12.15% N |
| Found: | 65.83% C | 6.09% H | 12.15% N |

Example 38

6-Fluoro-1-(2-fluorophenyl)-3-(1-methyl-4-piperidinyl)-1H-indazole fumarate

To a stirred suspension, under nitrogen, of 1.2 g (0.025 mole) of a 50% oil dispersion of sodium hydride in 25 ml of dimethylformamide was added, dropwise, 4.2 g (0.018 mole) of 6-fluoro-3-(1-methyl-4-piperidinyl)-1H-indazole dissolved in 30 ml of hot dimethylformamide. The reaction mixture was stirred at ambient temperature for 45 mins and then 4.4 g (0.038 mole) of 1,2-difluorobenzene was added. The temperature was raised to 120°C and held there for 6 hrs. The reaction mixture was poured into water and the aqueous mixture was extracted with ethyl acetate. The extract was washed with water, dried over anhydrous magnesium sulfate and the solvent concentrated to yield an oil. The oil was triturated with hexane to yield 1.3 g of the indazol starting material. The filtrate was concentrated to an oil. The oil was purified by flash chromatography utilizing a silica gel solumn (50 × 145 mm) and 10% ethyl acetate-diethylamine as the eluent. Concentration of the appropriate fractions gave 2.0 g (33%) of product, as an oil. The oil was dissolved in ether, stirred, and 0.8 g of fumaric acid was added. The salt was collected, dried and had mp 181—183°C.

*Analysis:*

| | | | |
|---|---|---|---|
| Calculated for $C_{19}H_{19}F_2N_3O \cdot C_4H_4O_4$: | 62.29% C | 5.23% H | 9.48% N |
| Found: | 62.15% C | 5.40% H | 9.47% N |

Example 39

3-(1-Acetyl-4-piperidinyl)-6-fluoro-1-phenyl-1H-indazole

To a stirred solution of 3.0 g (0.008 mole) of 1-acetyl-4-(2,4-difluorobenzoyl)piperidine phenylhydrazone in 30 ml of dimethylformamide, under nitrogen, was added 0.44 g (0.009 mole) of a 50% oil dispersion of sodium hydride. The reaction mixture was heated, and at about 40°C a vigorous solution of hydrogen occurred. The temperature was then raised to 80°C and held there for 1.5 hrs. The reaction mixture was poured into water and the aqueous suspension was extracted with ethyl acetate. The ethyl acetate extracts were washed with water, dried over anhydrous magnesium sulfate and concentrated to yield an oil. The oil was combined with the oil from another experiment and a total of 13.5 g of material was chromatographed on a Water's Prep LC 500. Two silica gel columns were utilized and 3% dichloromethane-methanol was used as the eluent. The appropriate fractions were evaporated. The residue was recrystallized twice from isopropyl alcohol-water (one charcoal treatment) to yield 2.4 g (18%) of product, mp 126—128°C.

*Analysis:*

| | | | |
|---|---|---|---|
| Calculated for $C_{20}H_{20}FN_3O$: | 71.20% C | 5.98% H | 12.45% N |
| Found: | 70.98% C | 5.99% H | 12.48% N |

Example 40

1-(2-Trifluoromethylphenyl)-3-(1-methyl-4-piperidinyl)-1H-indazole

To a stirred suspension of 1.0 g (0.021 mole) of a 50% oil dispersion of sodium hydride in 20 ml of dimethylformamide was added, dropwise, 3.2 g (0.0145 mole) of 3-(1-methyl-4-piperidinyl)-1H-indazole dissolved in 25 ml of hot dimethylformamide. After stirring for one hr at ambient temperature, 3.75 ml (0.029 mole) of 2-fluorobenzotrifluoride was added. The reaction mixture was heated to 90°C and stirred for 17 hrs. The mixture was then poured into water, extracted with ether, dried over anhydrous magnesium sulfate and concentrated. The residue was purified using flash chromatogrpahy (silica gel, diethylamine-ethyl acetate, 1:9, $R_f = 0.47$). The appropriate fractions were evaporated. The residue was recrystallized once from hexane, then combined with a previously prepared sample (1.1 g), and recrystallized from hexane to yield 2.17 g (24%) of product, mp 110—111°C.

*Analysis:*

| | | | |
|---|---|---|---|
| Calculated for $C_{20}H_{20}F_3N_3$: | 66.84% C | 5.61% H | 11.69% N |
| Found: | 66.65% C | 5.59% H | 11.82% N |

Example 41

1-(2-Fluorophenylmethyl)-3-(1-methyl-4-piperidinyl)-1H-indazole hydrobromide

To a suspension of 0.86 g (0.0179 mole) of a 50% oil dispersion of sodium hydride in 50 ml of dimethylformamide was added, dropwise, 3.0 g (0.014 mole) of 3-(1-methyl-4-piperidinyl)-1H-indazole dissolved in 15 ml of hot dimethylformamide. After stirring for 1 hr, 2.19 g (0.015 mole) of 2-fluorobenzyl chloride was added. The reaction mixture was stirred at ambient temperature for 17 hrs and then poured into 250 ml of water. The mixture was extracted with ether. The extract was washed with water, brine and dried over anhydrous magnesium sulfate. The solvent was evaporated. The residue was dissolved in anhydrous ether and hydrogen bromide gas was bubbled through the solution. The salt was triturated with ether and recrystallized from ethanol-ether and then ethanol to yield 3.06 g (54%) of product, mp 205—206°C.

*Analysis:*

| | | | |
|---|---|---|---|
| Calculated for $C_{20}H_{22}FN_3 \cdot CHBr$: | 59.41% C | 5.73% H | 10.39% N |
| Found: | 59.02% C | 5.68% H | 10.29% N |

Example 42

1-Benzoyl-6-chloro-3-(1-methyl-4-piperidinyl)-1H-indazole hydrochloride

A mixture of 3.0 g (0.012 mole) of 6-chloro-3-(1-methyl-4-piperdinyl)-1H-indazole and 8 ml of benzoyl chloride was heated at 100°C on a steam bath for 4 hrs. The reaction mixture was cooled to ambient temperature and ether was added. The precipitate was filtered, washed with ether and dried. Recrystallization from ethanol (twice) yielded 2.6 g (56%) of product, mp 248—250°C

*Analysis:*

| | | | |
|---|---|---|---|
| Calculated for $C_{20}H_{20}ClN_3O \cdot HCl$: | 61.54% C | 5.42% H | 10.77% N |
| Found: | 61.34% C | 5.46% H | 10.67% N |

Example 43

1-(4-Chlorobenzoyl)-3-(1-ethyl-4-piperidinyl)-6-fluoro-1H-indazole hydrochloride

A mixture of 3.0 g (0.012 mole) of 3-(1-ethyl-4-piperidinyl)-6-fluoro-1H-indazole and 8 ml of 4-chlorobenzoyl chloride was heated at 100°C in a steam bath for 4 hrs. Ether was added and the solid was collected. The solid was crystallized twice from ethanol-ether to yield 2.9 g (57%) of product, mp 240—242°C.

*Analysis:*

| | | | |
|---|---|---|---|
| Calculated for $C_{21}H_{21}ClFN_3O \cdot HCl$: | 59.72% C | 5.01% H | 9.95% N |
| Found: | 59.69% C | 5.39% H | 9.92% N |

Example 44

1-(4-Chlorobenzoyl)-6-fluoro-3-(1-methyl-4-piperidinyl-1H-indazole hydrochloride

A mixture of 2.0 g (0.0086 mole) of 6-fluoro-3-(1-methyl-4-piperidinyl)-1H-imidazole and 5 ml of 4-chlorobenzoyl chloride was heated at 100°C in a steam bath for 2 hrs. After cooling, ether was added and the solid was collected. The solid was combined with a 2.5 g sample from another experiment and recrystallized twice from ethanol-ether to yield 4.7 g (56%, calculated on the combination of both experiments) of product, mp 258—260°C.

*Analysis:*

| | | | |
|---|---|---|---|
| Calculated for $C_{20}H_{19}ClN_3 \cdot HCl$: | 58.83% C | 4.69% H | 10.29% N |
| Found: | 59.02% C | 5.07% H | 10.30% N |

Example 45

1-(4-Methylbenzoyl)-3-(1-methyl-4-piperidinyl)-1H-indazole fumarate

A mixture of 3.0 g (0.014 mole) of 3-(1-methyl-4-piperidinyl)-1H-indazole and 8 ml of 4-toluoyl chloride was heated at 100°C in a steam bath for 4 hrs. Ether was added to the cooled mixture and the salt was collected. The salt was converted to its free base by means of ammonium hydroxide solution. The base was dissolved in ethanol (90 ml)-ether (175 ml) and 1.5 g of fumaric acid was added. The mixture was stirred at ambient temperature for 16 hrs, and the resultant fumarate salt was collected. Recrystallization twice from methanol-ether yielded 3.8 g (60%) of product, mp 205—207°C.

*Analysis:*

| | | | |
|---|---|---|---|
| Calculated for $C_{21}H_{23}ClN_3O \cdot C_4H_4O_4$: | 66.80% C | 6.06% H | 9.35% N |
| Found: | 66.98% C | 6.14% H | 9.44% N |

Example 46

1-(2,3-Dichlorobenzoyl)-3-(1-methyl-4-piperidinyl)-1H-indazole

A mixture of 3.0 g (0.014 mole) of 3-(1-methyl-4-piperinyl)-1H-indazole and 8 ml of 3,4-dichlorobenzoyl chloride was heated at 100°C in a steam bath for 4 hrs. After the reaction mixture cooled to ambient temperature, ether was added and the salt was collected. The salt was mixed with water and ammonium hydroxide solution was added until the mixture was basic. The mixture was warmed on the steam bath for about 5 mins, cooled and extracted with dichloromethane. Evaporation of the dichloromethane *in vacuo* provided the free base. The base was recrystallized from methanol-trichloromethane (with concentration of the mother liquor) to yield 3.0 g (57%) of product, mp 187—189°C.

*Analysis:*

| | | | |
|---|---|---|---|
| Calculated for $C_{20}H_{19}Cl_2N_3O$: | 61.86% C | 4.93% H | 10.82% N |
| Found: | 61.83% C | 5.02% H | 10.84% N |

Example 47

1-Benzoyl-6-fluoro-3-(1-methyl-4-piperidinyl)-1H-indazole fumarate

A mixture of 3.1 g (0.013 mole) of 6-fluoro-3-(1-methyl-4-piperidinyl)-1H-indazole and 8 ml of benzoyl chloride was heated at 100°C in a steam bath for 8 hrs. After cooling, ether was added and the solid was collected. The solid was treated with ammonium hydroxide solution to give an oil. The oil was dissolved in 300 ml of ether, stirred and 1.2 g of fumaric acid was added. After four hrs at ambient temperature, the fumarate salt was collected. Recrystallized from ethanol-ether yielded 3.0 g (50.9%) of product, mp 180—182°C.

*Analysis:*

| | | | |
|---|---|---|---|
| Calculated for $C_{20}H_{20}FN_3O \cdot C_4H_4O_4$: | 63.57% C | 5.33% H | 9.27% N |
| Found: | 63.51% C | 5.43% H | 9.35% N |

Example 48

1-(4-Trifluoromethylbenzoyl)-3-(1-methyl-4-piperidinyl)-1H-indazole fumarate

A mixture of 3.0 g (0.014 mole) of 3-(1-methyl-4-piperidinyl)-1H-indazole and 8 ml of 4-trifluoromethylbenzoyl chloride was heated at 100°C on a steam bath. After 2 hrs, an additional 5 ml of 4-trifluoromethylbenzoyl chloride was added and heating was continued for 2 additional hrs. After cooling to ambient temperature, ether was added and the salt collected. The salt was converted to its free base by means of ammonium hydroxide solution. The free base was dissolved in ethanol-ether and 0.75 g of fumaric acid was added to give 2.7 g (38%) of product. Two samples of the salt were combined and recrystallized from ethanol (twice) to yield the analytical sample, mp 212—214°C.

*Analysis:*

| | | | |
|---|---|---|---|
| Calculated for $C_{21}H_{20}F_3N_3O \cdot C_4H_4O_4$: | 59.64% C | 4.80% H | 8.35% N |
| Found: | 59.57% C | 4.86% H | 8.41% N |

Example 49

1-(4-Chlorobenzoyl)-3-(1-methyl-4-piperidinyl)-1H-indazole fumarate

A solution of 3.0 g (0.014 mole) of 3-(1-methyl-4-piperidinyl)-1H-indazole and 8 ml of 4-chlorobenzoyl chloride was heated at 100°C in a steam bath for 4 hrs. After cooling to ambient temperature, ether was added and the salt was collected. The salt was converted into its free base by means of ammonium hydroxide solution and extractive isolation with dichloromethane. The base was dissolved in 150 ml of hot ethanol and 1.6 g of fumaric acid was added. Ether (200 ml) was added to the solution and the fumarate salt was collected. Two recrystalizations from ethanol yielded 3.5 g (54%) of product, mp 216—218°C.

*Analysis:*

| | | | |
|---|---|---|---|
| Calculated for $C_{20}H_{20}ClN_3O \cdot C_4H_4O_4$: | 61.34% C | 5.15% H | 8.94% N |
| Found: | 61.42% C | 5.18% H | 9.00% N |

Example 50

6-Chloro-3-(1-methyl-4-piperidinyl)-1H-indazole

To a stirred solution, under nitrogen, of 13.0 g (0.044 mole) of 4-(6-chloroindazol-3-yl)piperidinyl-1-carboxylic acid methyl ester in 150 ml of tetrahydrofuran was added, dropwise, 48 ml of a 1M solution of lithium aluminium hydride (0.048 mole) in tetrahydrofuran. The solution was stirred under reflux for 1 hr. The reaction mixture was cooled in an ice-salt bath and water was added slowly. The reaction mixture was filtered, the filter cake washed with tetrahydrofuran and methanol, and the filtrate was concentrated. Recrystallization of the residue from ethanol-water yielded 7.6 g (69%) of product, mp 211—213°C.

*Analysis:*

| | | | |
|---|---|---|---|
| Calculated for $C_{13}H_{16}ClN_3$: | 62.52% C | 6.46% H | 16.83% N |
| Found: | 62.73% C | 6.61% H | 16.96% N |

Example 51

3-(1-Ethyl-4-piperidinyl)-6-fluoro-1H-indazole

To a stirred suspension, under nitrogen, of 14.0 g (0.053 mole) of 3-(1-acetyl-4-piperidinyl)-6-fluoro-1H-indazole of 180 ml of tetrahydrofuran was added, dropwise, 55 ml of a 1M solution of lithium aluminium hydride (0.055 mole) in tetrahydrofuran. The reaction mixture was stirred under reflux for 1 hr and then stirred at ambient temperature for 15 hrs. The reaction mixture was cooled in an ice-salt bath and water was added slowly. The reaction mixture was filtered. The filter cake was washed with tetrahydrofuran and the filtrate was concentrated. Recrystallization of the residue from toluene afforded 9.0 g (68%) of product. Recrystallization of a 3.0 g sample from toluene yielded the analytical sample, mp 175—178°C.

*Analysis:*

| | | | |
|---|---|---|---|
| Calculated for $C_{14}H_{18}FN_3$: | 67.99% C | 7.34% H | 16.99% N |
| Found: | 68.07% C | 7.33% H | 17.11% N |

Example 52

6-Chloro-3-(4-piperidinyl)-1H-indazole

A solution of 18.0 g (0.063 mole) of 1-acetyl-4-(4-chloro-2-fluorobenzoyl)piperidine, 130 ml of n-butanol and 51 ml of hydrazine monohydrate was stirred under reflux for 48 hrs. Most of the n-butanol was evaporated *in vacuo* and the residue was diluted with water. The aqueous mixture was extracted with ethyl acetate, and the extract was washed with water, dried over anhydrous magnesium sulfate and concentrated. The residue was recrystallized from acetonitrile to yield 3.0 g (20%) of product. This product was combined with a 1.3 g sample from another experiment and the combined samples (4.3 g) were recrystallized from acetonitrile to yield the analytical sample, mp 174—176°C.

*Analysis:*

| | | | |
|---|---|---|---|
| Calculated for $C_{12}H_{14}ClN_3$: | 61.14% C | 5.99% H | 17.88% N |
| Found: | 61.02% C | 5.94% H | 17.94% N |

Example 53

3-(1-Methyl-4-piperidinyl)-1H-indazole

A solution of 4.7 g (0.018 mole) of 4-(1H-indazole-3-yl)-piperidine-1-carboximidic acid methyl ester and 80% of acetic acid was heated under reflux for 48 hrs. The reaction mixture was cooled and poured into water. The aqueous phase was extracted with ether and then made basic by the slow addition of a 50% aqueous sodium hydroxide solution. A solid separated from the solution. The solid was collected and dried. Recrystallization from ethanol yielded 1.7 g (46%) of product. Concentration of the mother liquors from this and another experiment gave additional product, mp 213—215°C.

*Analysis:*

| | | | |
|---|---|---|---|
| Calculated for $C_{12}H_{15}N_3$: | 71.61% C | 7.51% H | 20.88% N |
| Found: | 70.89% C | 7.48% H | 20.74% N |

Example 54

6-Bromo-1-methyl-3-(4-piperidinyl)-1H-indazole hydrochloride

A solution of 3.3 g (0.01 mole) of 4-(3-bromo-2-fluorobenzoyl)-1-acetylpiperidine, 1.1 g (0.013 mole) of methylhydrazine and 30 ml of n-butanol was heated under reflux for 16 hrs. The reaction mixture was concentrated *in vacuo* and the residue was diluted with water. The aqueous suspension was made basic with ammonium hydroxide solution and extracted with dichloromethane. The extract was washed with water, dried over anhydrous potassium carbonate and the solvent was concentrated to give 3-(1-acetyl-4-piperidinyl)-6-bromo-1H-indazole, as an oil.

A solution of 3.0 g (0.009 mole) of 3-(1-acetyl-4-piperidinyl)-6-bromo-1H-indazole and 30 ml of 6N hydrochloric acid was heated under reflux for 4 hrs. The reaction mixture was cooled in an ice-bath, stirred and 50% aqueous sodium hydroxide solution was added dropwise until the reaction mixture was basic. The aqueous mixture was extracted with ether. The ether extracts werre washed with water, dried over anhydrous magnesium sulfate and the solvent was concentrated to yield 1.6 g (62%) of product, as an oil. The product from three experiments were combined and converted to the hydrochloride salt with ethereal hydrogen chloride. The salt was recrystallized twice from ethanol-ether and then once from trichloromethane-ether to yield the analytical sample, mp 238—240°C.

*Analysis:*

| | | | |
|---|---|---|---|
| Calculated for $C_{13}H_{16}BrN_3O \cdot HCl$: | 47.22% C | 5.18% H | 12.71% N |
| Found: | 46.96% C | 5.12% H | 12.66% N |

Example 55

3-(1-Acetyl-4-piperidinyl)-6-fluoro-1H-indazole

A solution of 15.0 g (0.069 mole) of 4-(2,4-difluorobenzoyl)-1-acetylpiperidine, 6.9 g (0.14 mole) of hydrazine hydrate and 140 ml of ethanol was heated under reflux for 4 hrs. The ethanol was removed *in vacuo* to give a solid. The solid was triturated with water, filtered and dried to yield 12.5 g (64%) of 1-acetyl-4-(2,4-difluorobenzoyl)piperidine hydrazone, mp 139—142°C.

A mixture of 12.1 g (0.046 mole) of 1-acetyl-4-(2,4-difluorobenzoyl)piperidine hydrazone, 11.8 g of potassium carbonate and 120 ml of dimethylformamide was stirred and heated at 120°C for 16 hrs. The reaction mixture was poured into water and the aqueous solution was extracted with ethyl acetate. The extract was washed with water, dried over anhydrous magnesium sulfate and the solvent concentrated to give a solid. Recrystallization of the solid from ethyl acetate (twice) yielded 4.1 g (42% with concentration of mother liquor) of product, mp 162—164°C.

*Analysis:*

| | | | |
|---|---|---|---|
| Calculated for $C_{14}H_{16}FN_3O$: | 64.35% C | 6.17% H | 16.08% N |
| Found: | 64.10% C | 6.31% H | 15.89% N |

Example 56

3-(1-Acetyl-4-piperidinyl)-6-chloro-1H-indazole

A mixture of 19.6 g (0.066 mole) of 1-acetyl-4-(2-fluoro-4-chlorobenzoyl)piperidine hydrazone, 18.2 g (0.13 mole) of potassium carbonate and 200 ml of dimethylformamide was stirred, under nitrogen, at 120°C

for 16 hrs. After cooling to ambient temperature, the reaction mixture was poured into water and the aqueous mixture was extracted with ethyl acetate. The extract was washed with water, dried over anhydrous magnesium sulfate and concentrated to an oil. Upon standing, a solid formed. The solid was triturated with ethyl acetate to yield 3.8 g (21%) of product. Recrystallization from acetonitrile gave the analytical sample, mp 172—174°C.

*Analysis:*

| | | | |
|---|---|---|---|
| Calculated for $C_{14}H_{16}ClN_3O$: | 60.54% C | 5.81% H | 15.13% N |
| Found: | 60.55% C | 5.83% H | 15.33% N |

Example 57

4-(6-Chlorindazol-3-yl)piperidine-1-carboxylic acid methyl ester

To a stirred suspension of 5.8 g (0.025 mole) of 6-chloro-3-(4-piperidinyl)-1H-indazole, 4.6 g (0.055 mole) of sodium bicarbonate and trichloromethane (25 ml)-tetrahydrofuran (25 ml) was added, dropwise, 5.2 g (0.055 mole) of methyl chloroformate. The reaction mixture was stirred at ambient temperature for 16 hrs and then poured into water. The organic layer was collected, washed with water, dried over anhydrous potassium carbonate and the solvent concentrated to give 4-(6-chloro-1-methoxyoxycarbonyl-3-yl)-pipeidine-1-carboxylic acid methyl ester, as an oil. The oil was dissolved in 50 ml of methanol and 1.5 ml of a 25% sodium methoxide-methanol solution was added. The reaction mixture was stirred at ambient temperature for 0.5 hrs, poured into water and the aqueous mixture extracted with dichloromethane. The dichloromethane extract was washed with water, dried over anhydrous magnesium sulfate and the solvent evaporated to an oil. The oil was triturated with hexane. Recrystallization from toluenehexane (twice) yielded 3.5 g (47%) or product, mp 132—134°C.

*Analysis:*

| | | | |
|---|---|---|---|
| Calculated for $C_{14}H_{16}ClN_3O_2$: | 57.24% C | 5.49% H | 14.30% N |
| Found: | 57.22% C | 5.46% H | 14.33% N |

Example 58

1-(4-Pyridinyl)-3-(1-methyl-4-piperidinyl)-1H-indazole sesquifumarate

To a stirred suspension of 1.72 g (0.036 mole) of a 50% oil dispersion of sodium hydride, under nitrogen, in 30 ml of dimethylformamide was added, dropwise, 3.2 g (0.0148 mole) of 3-(1-methyl-4-piperidinyl)-1H-indazole in 25 ml of hot dimethylformamide., After stirring at ambient temperature for 45 mins, 2.3 g (0.0155 mole) of 4-chloropyridine hydrochloride was added portionwise. The reaction mixture was stirred at ambient temperature for 16 hrs and then heated under reflux for 12 hrs. The reaction mixture was cooled to ambient temperature and 0.43 g of a 50% oil dispersion of sodium hydride was added followed by 0.6 g of 4-chloropyridine. The reacton mixture was then heated under reflux for an addition 4 hrs, cooled to ambient temperature and poured into water. The aqueous mixture was extracted with ethyl acetate. The ethyl acetate extract was washed with water, dried over anhydrous magnesium sulfate and the solvent was removed *in vacuo*. The residue was purified in two runs by flash chromatography (silica gel, 150 × 50 mm), eluting with ethyl acetate-diethylamine (9:1). Similar fractions were combined and evaporated. The residue was converted to the fumarate salt by dissolution in ethanol (25 ml)-ether (175 ml) and by adding 1.9 g of fumaric acid. Recrystallization from dimethylformamide yielded 3.2 g (46%) of product, mp 205—207°C.

*Analysis:*

| | | | |
|---|---|---|---|
| Calculated for $C_{18}H_{20}N_4O \cdot 1.5C_4H_4O_4$: | 61.79% C | 5.62% H | 12.01% N |
| Found: | 61.50% C | 5.78% H | 12.18% N |

Example 59

1-(2-Pyridinyl)-3-(1-methyl-4-piperidinyl)-1H-indazole fumarate

To a stirred suspension, under nitrogen, of 0.86 g (0.018 mole) of a 50% oil dispersion of sodium hydride in 20 ml of dimethylformamide was added, dropwise, 3.2 g (0.015 mole) of 3-(1-methyl-4-piperidinyl)-1H-indazole dissolved in 25 ml of hot dimethylformamide. The reaction mixture was stirred at ambient temperature for 1 hr and then 1.5 g (0.0155 mole) of 2-fluoropyridine was added. The temperature was raised to 95—100°C and held there for 6 hrs. The reaction mixture was allowed to cool and was then poured into water. A solid separated from the solution. The solid was collected and dried to yield 3.5 g (79.8%) of product, mp 123—125°C, as the free base. The fumarate salt was prepared by dissolving the product in hot ethanol, adding 2.7 g of fumaric acid and heating the mixture on the steam bath. After a few mins, the fumarate salt precipitated from the solution. The mixture was allowed to stand overnight at ambient temperature and the salt was collected. Two recrystallizations from methanol-ether yielded 2.8 g (46%) of product, mp 203—205°C.

*Analysis:*

| | | | |
|---|---|---|---|
| Calculated for $C_{18}H_{20}N_4O \cdot C_4H_4O_4$: | 64.69% C | 5.92% H | 13.72% N |
| Found: | 64.69% C | 6.00% H | 13.77% N |

Example 60

1-(3-Hydroxy-1-propyl)-3-(1-methyl-4-piperidinyl)-1H-indazole

To a stirred suspension of 0.35 g (0.0046 mole) of a 50—55% oil dispersion of lithium aluminum hydride in 50 ml of tetrahydrofuran was added, dropwise, under nitrogen, 2.4 g (0.007 mole) of 2-(3-ethoxycarbonyl-1-ethyl)-3-(1-methyl-4-piperidinyl)-1H-indazole in 25 ml of tetrahydrofuran. The reaction mixture was stirred under reflux for 1.5 hrs, cooled in an ice-salt bath, and water was added dropwise. The reaction mixture was filtered. The filter cake was washed with tetrahydrofuran and the filtrate concentrated *in vacuo* to give an oil. The oil was taken up in dichloromethane. The dichloromethane was washed with water, dried over anhydrous magnesium sulfate and the solvent removed *in vacuo* to yield 1.7 g (81%) of product. The product was combined with that from another experiment (total 3.2 g) and recrsytallized twice from ethyl acetate-hexane to yield the analytical sample, mp 96—98°C.

*Analysis:*

| | | | |
|---|---|---|---|
| Calculated for $C_{16}H_{23}N_3O$: | 70.29% C | 8.48% H | 15.37% N |
| Found: | 70.53% C | 8.34% H | 15.47% N |

Example 61

4-(4-Bromo-2-fluorobenzoyl)-1-acetylpiperidine

To a stirred suspension of 21.1 g (0.16 mole) of aluminum chloride and 63 ml of 3-fluorobromobenzene was added, portionwise, 16.0 (0.08 mole) of 1-acetyl-isonipecotoyl chloride. The reaction mixture was stirred under reflux for 4 hrs. Most of the supernatant 3-fluorobromobenzene was decanted and ice water was added. The aqueous mixture was extracted with dichloromethane and the extract was washed with water, dried over anhydrous magnesium sulfate and the solvent concentrated to give an oil. The oil was triturated with ether. Recrystallization from cyclohexane gave 4.9 g (19%) of product. Recrystallization fro cyclohexane yielded the analytical sample, mp 111—113°C.

*Analysis:*

| | | | |
|---|---|---|---|
| Calculated for $C_{14}H_{15}BrFNO_2$: | 51.23% C | 4.61% H | 4.24% N |
| Found: | 51.40% C | 4.61% H | 4.92% N |

Example 62

1-(3-Ethoxycarbonyl-1-ethyl)-3-(1-methyl-4-piperidinyl)-1H-indazole

To a stirred suspension, under nitrogen, of 1.7 g of a 50% oil dispersion of sodium hydride in 75 ml of dimethylformamide was added, dropwise, 6.0 g of 3-(1-methyl-4-piperidinyl)-1H-indazole in 30 ml of hot dimethylformamide. Upon completion of the addition, the reaction mixture was stirred at ambient temperature for 1 hr and a solution of 6.5 g of ethyl 3-bromopropionate was added dropwise. The reaction mixture was stirred at ambient temperature for 3 hrs and then poured into water. The aqueous mixture was extracted with ethyl acetate and the extracts were washed with water, dried over anhydrous magnesium sulfate and filtered. Evaporation of the filtrate under vacuum gave 6.5 g (70.8%) of product, as an oil.

REACTION SCHEME A

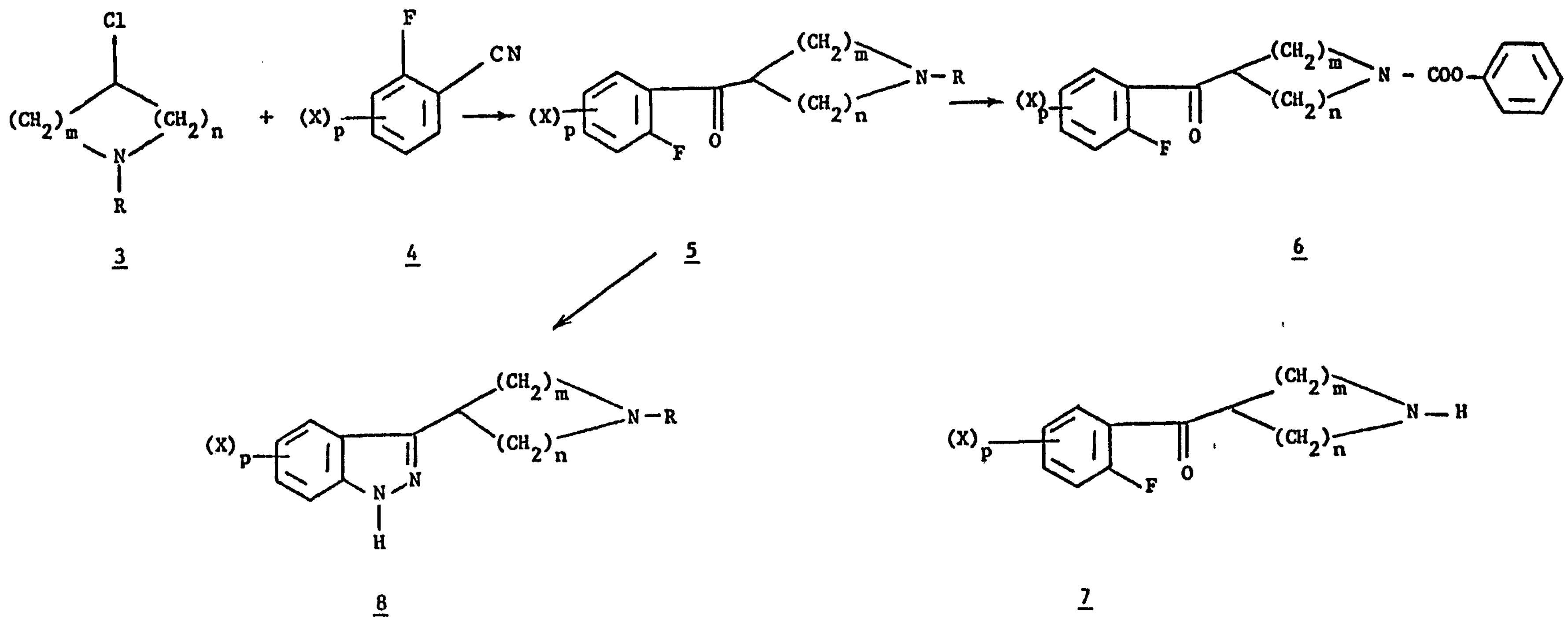

3 4 5 6

8 7

wherein R, X, m, n and p are as hereinbefore described.

REACTION SCHEME B

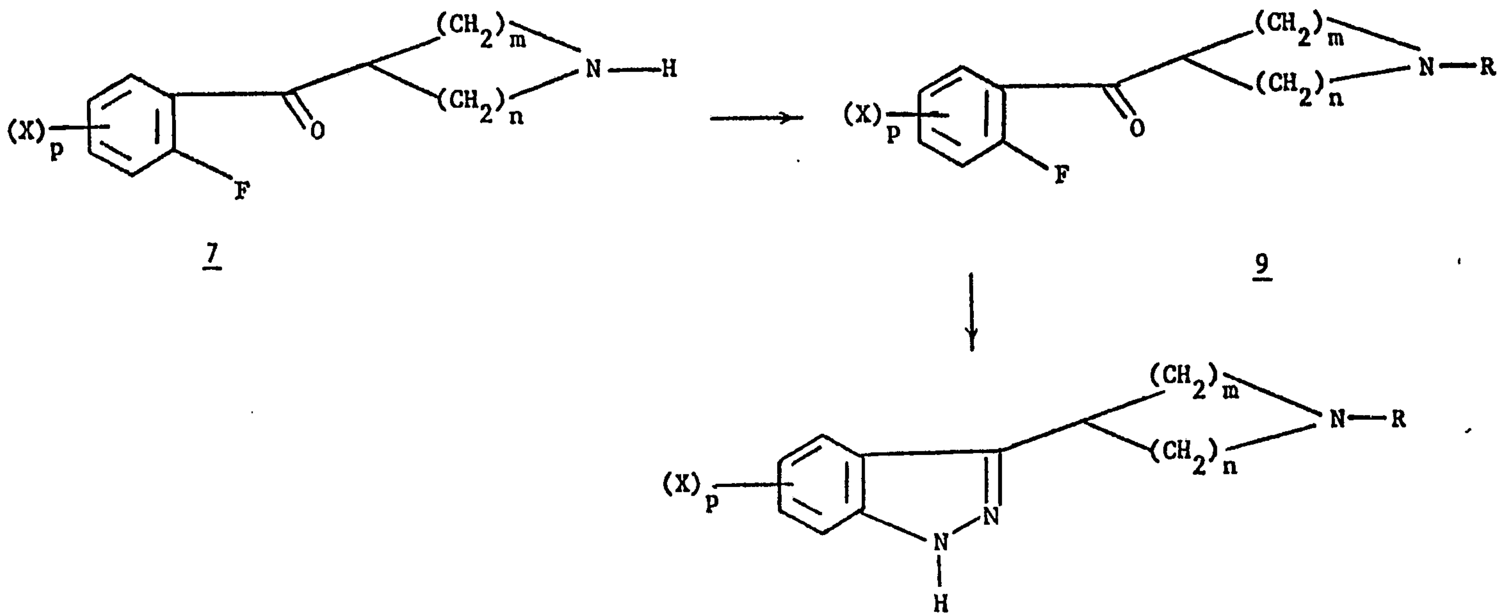

7

9

10

wherein R, X, m, n and p are as hereinbefore described.

REACTION SCHEME C

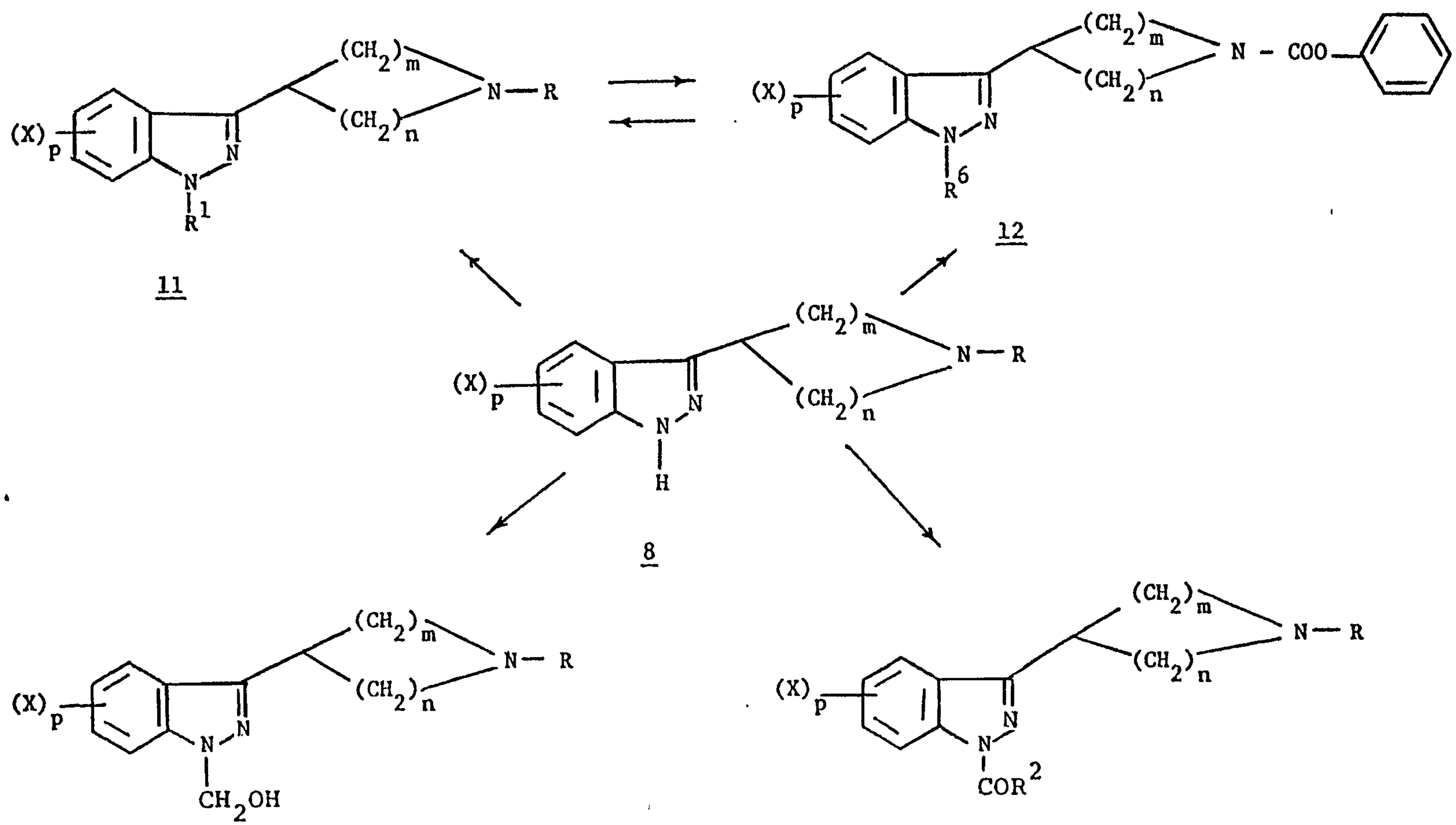

11

12

8

13

14

wherein R, $R^1$, $R^2$, $R^6$, X, m, n and p are as hereinbefore described.

REACTION SCHEME D

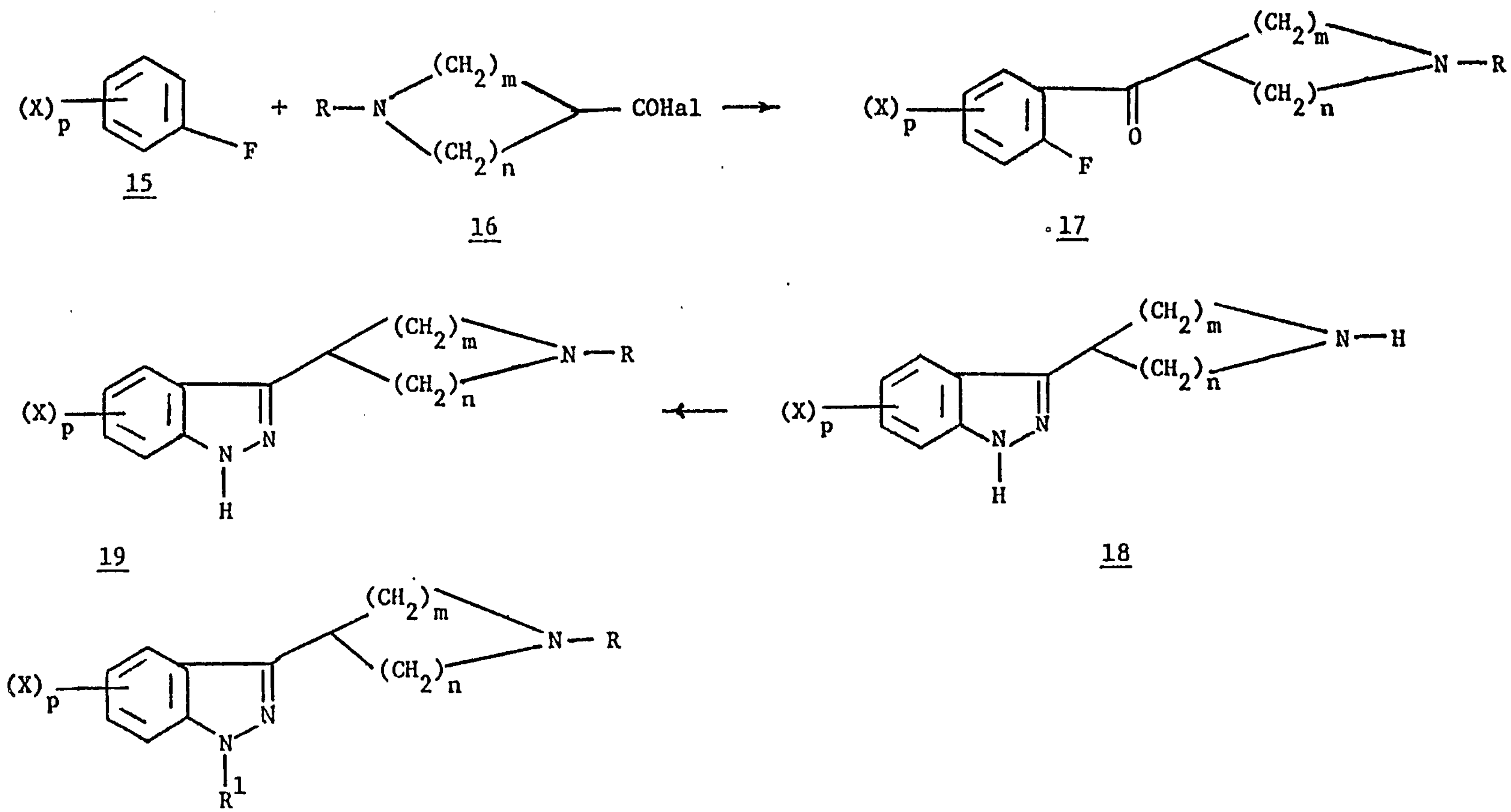

15 16 17 18 19 20

wherein R, $R^1$, X, Hal, m, n and p are as before.

**Claims for the Contracting States: BE CH DE FR GB IT LI LU NL SE**

1. A compound of the formula 1

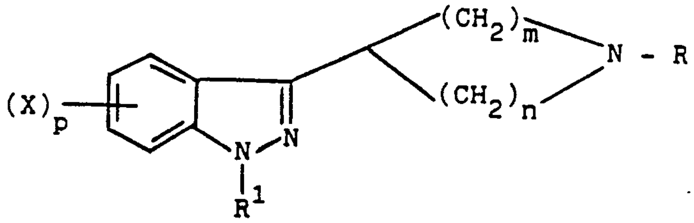

(1),

wherein R is hydrogen, $C_1$—$C_7$-alkyl, $C_3$—$C_7$-alkenyl, $C_3$—$C_7$-cycloalkyl-$C_1$—$C_7$-alkyl,

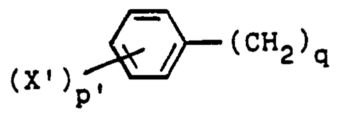

,

cyano, cyanomethyl, formyl, $C_1$—$C_7$-alkanoyl, or a group of the formulae

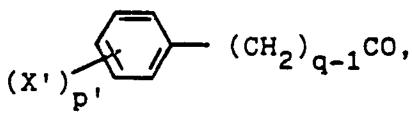

$R^2OCO$,

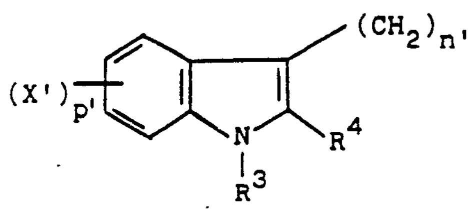

,

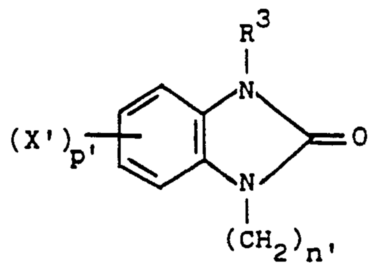

,

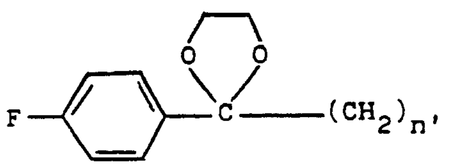

,

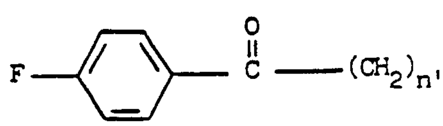

,

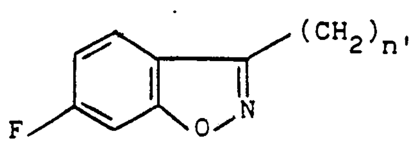

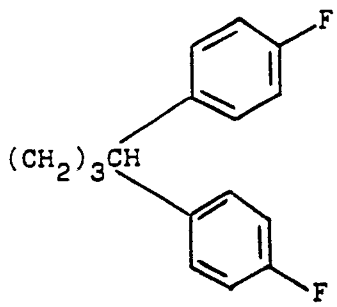

$R^1$ is hydrogen, $C_1$—$C_7$-alkyl, $C_3$—$C_7$-alkenyl, $C_3$—$C_7$-cycloalkyl-$C_1$—$C_7$-alkyl,

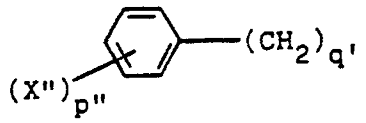 ,

di-$C_1$—$C_7$-alkylamino-$C_1$—$C_7$-alkyl, cyano, cyanomethyl, formyl, $C_1$—$C_7$-alkanoyl, hydroxymethyl, $C_3$—$C_7$-cycloalkyl-$C_1$—$C_7$-alkanoyl, or a group of the formulae

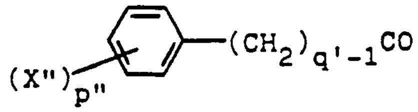 $R^{2'}OCO$,

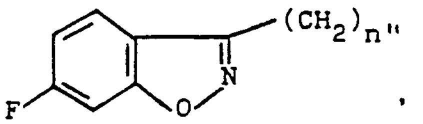 ,

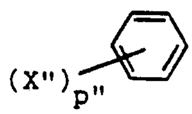 ,

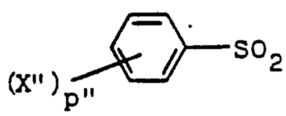

$R^5CO$ or 2- or 4-pyridinyl or 2-pyrimidinyl; $R^2$ and $R^{2'}$ are independently $C_1$—$C_7$-alkyl, 2,2,2-trichloroethyl or phenyl; $R^3$ and $R^4$ are independently hydrogen or $C_1$—$C_7$-alkyl; $R^5$ is a member selected from the group furyl, thienyl, pyridinyl, thiazolyl, isothiazolyl, oxazolyl or isoxazolyl, X, X′ and X″ are independently hydrogen, halogen, $C_1$—$C_7$-alkyl, $C_1$—$C_7$-alkoxy, hydroxy, nitro, amino or trifluoromethyl; m is 2 or 3, n is 1 or 2, and the sum of m and n is 3 or 4; n′ and n″ are independently 2 or 3; p, p′ and p″ are independently 1 or 2; q and q′ are independently 1, 2, 3 or 4; the optical antipode thereof; or the pharmaceutically acceptable salt thereof.

2. A compound according to claim 1 wherein R is hydrogen or $C_1$—$C_7$-alkyl or

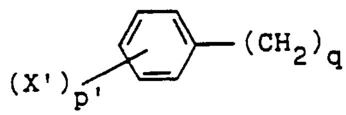

and $R^1$ is hydrogen, $C_1$—$C_7$-alkyl, $C_3$—$C_7$-cycloalkyl-$C_1$—$C_7$-alkyl, $C_1$—$C_7$-alkanoyl, hydroxymethyl,

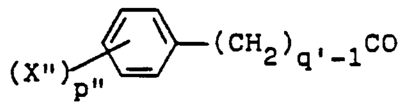 ,

wherein X′ and X″ are independently hydrogen, halogen, $C_1$—$C_7$-alkyl, $C_1$—$C_7$-alkoxy, hydroxy, nitro, amino or trifluoromethyl, p′ and p″ are 1 or 2 and 1 and q′ are independently 1, 2, 3 or 4; or $R^1$ is $R^{2'}OCO$ where $R^{2'}$ is $C_1$—$C_7$-alkyl, 2,2,2-trichloroethyl, or phenyl or $R^1$ is

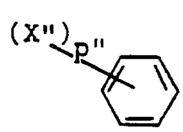

wherein X″ is hydrogen, halogen, $C_1$—$C_7$-alkyl, $C_1$—$C_7$-alkoxy, hydroxy, nitro, amino or trifluoromethyl; and p″ is 1 or 2.

3. A compound according to claim 2 wherein R is hydrogen or $C_1$—$C_7$-alkyl and $R_1$ is hydrogen, $C_1$—$C_7$-alkyl or a group of the formulae

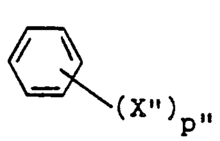 or 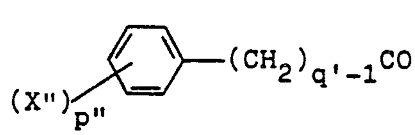

wherein X'' is hydrogen, $C_1$—$C_7$-alkyl, $C_1$—$C_7$-alkoxy, hydroxy, nitro, amino or trifluoromethyl; p'' is 1 or 2; and q' is 1.

4. The compound of claim 1 which is 3-(1-methyl-4-piperidinyl)-1H-indazole.

5. The compound of claim 1 which is 1-ethyl-3-(1-methyl-4-piperidinyl)-1H-indazole.

6. The compound of claim 1 which is 1-acetyl-3-(1-methyl-4-piperidinyl)-1H-indazole.

7. The compound of claim 1 which is 1-benzoyl-3-(1-methyl-4-piperidinyl)-1H-indazole.

8. The compound of claim 1 which is 1-phenylmethyl-3-(1-methyl-4-piperidinyl)-1H-indazole.

9. The compound of claim 1 which is 1-cyclopropylmethyl-3-(1-methyl-4-piperidinyl)-1H-indazole.

10. A compound as defined in claim 1 for use as a medicament.

11. A pharmaceutical composition comprising as active ingredient compound of the formula 1 or a pharmaceutically acceptable salt thereof as defined in claim 1 in association with a pharmaceutically acceptable carrier.

12. A process for the preparation of a compound as claimed in claim 1, which comprises cyclizing a compound of the formula

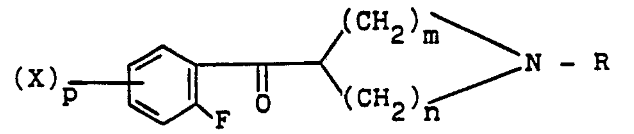 ,

wherein R, X, m, n and p are as defined in claim 1, with hydrazine hydrate, to yield a compound of the formula 1 wherein $R^1$ is hydrogen and R, X, M, n and p are as defined, optionally treating a compound of the formula 1 wherein $R^1$ is hydrogen and R is as defined with the proviso that R is not hydrogen, with an $C_1$—$C_7$-alkyl halide, $C_3$—$C_7$-alkenyl halide, $C_3$—$C_7$-cycloalkyl-$C_1$—$C_7$-alkyl halide, di-$C_1$—$C_7$-alkylamino-$C_1$—$C_7$- alkyl halide, cyanogen halide, cyanomethyl halide, 2- or 4-pyridyl halide, 2-pyrimidinyl halide, a compound of the formulae

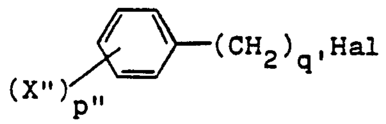 , 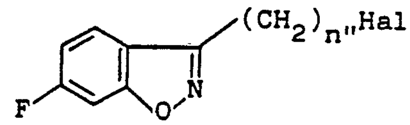 ,

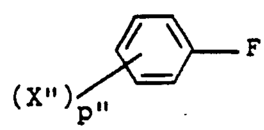 ,

wherein X'', n'', q' and p'' are as defined and Hal is iodo, bromo or chloro in the presence of an alkali metal hydride to yield a compound of the formula 1 wherein $R^1$ is $C_1$—$C_7$-alkyl, $C_3$—$C_7$-alkenyl-$C_3$—$C_7$-cycloalkyl-$C_1$—$C_7$-alkyl, di-$C_1$—$C_7$-aalkylamino-$C_1$—$C_7$-alkyl, cyano, cyanomethyl or a group of the formulae

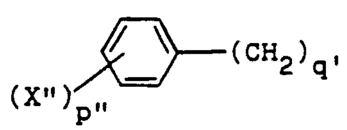 , 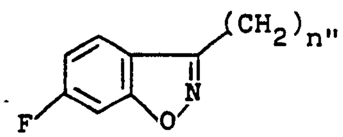 ,

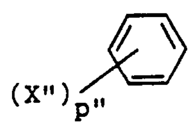 ;

2- or 4-piperidinyl or 2-pyrimidinyl, and X, m, n and p are as defined above, or optionally reacting a compound of the formula 1 wherein $R^1$ is hydrogen and R is as defined above with the proviso that R is not hydrogen, and X, m, n and p are as defined above, with a formyl-, $C_1$—$C_7$-alkanoyl- or $C_3$—$C_7$-cycloalkyl-$C_1$—$C_7$-alkanoyl chloride, bromide or iodide or a compound of the formula

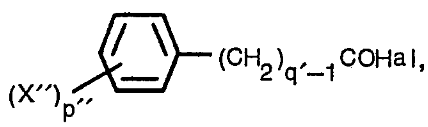, $R^{2'}OCOHal$ or $R^5COHal$

where $R^{2'}$, $R^5$, X'', p'' and q' are as defined above and Hal is chloro, bromo or iodo, or the corresponding acid anhydrides to yield a compound of the formula 1 wherein $R^1$ is formyl, $C_1$—$C_7$-alkanoyl, $C_3$—$C_7$-cycloalkyl-$C_1$—$C_7$-alkanoyl or a group of the formulae

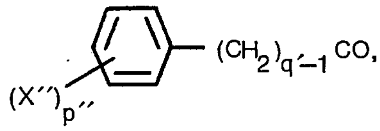

$R^{2'}OCO$ or $R^5CO$ , or

optionally reacting a compound of the formula 1 wherein $R^1$ is hydrogen and R is as defined above with the proviso that R is not hydrogen, with formaldehyd to yield a compound of the formula 1 wherein $R^1$ is hydroxymethyl or reacting a compound of the formula 1 wherein $R^1$ is hydrogen and R is phenoxycarbonyl with formaldehyde and then removing the carbamoyl group by hydrolysis to yield a compound of the formula 1 wherein $R^1$ is hydroxymethyl and R is hydrogen, or optionally reacting a compound of the formula 1 wherein $R^1$ is hydrogen and R is as defined above but is not hydrogen, with a benzenesulfonyl halide of the formula

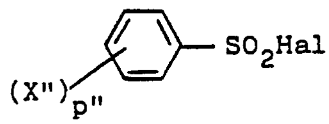

wherein X″ and p″ are defined above and Hal is chlorine or bromine, to yield a compound of the formula 1 wherein $R^1$ is the group

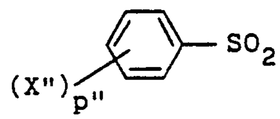

, or

optionally converting a compound of the formula 1 wherein $R^1$ is a group of the formulae

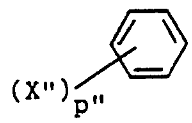

wherein X″ is the nitro group, by reduction of the nitro group to an amino function, diazotization and subsequent displacement or reduction of the diazonium moiety to a compound of the formula 1 wherein $R^1$ is a group of the formula

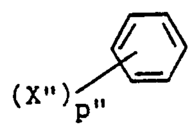

where X″ is hydrogen, halogen, $C_1$—$C_7$-alkyl, $C_1$—$C_7$-alkoxy, hydroxy or amino and p″ is 1 or 2, optionally reacting a compound of the formula 1 wherein R and $R^1$ are both hydrogen with a $C_1$—$C_7$-alkanoyl chloride, bromide or iodide or with a halide of the formulae

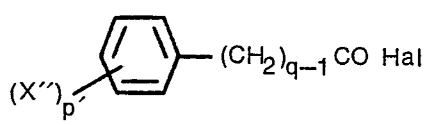

or $R^{2'}OCO$ Hal

where $R^{2'}$, X′, p′ and q are as defined above and Hal is chlorine, bromine or iodine, to yield a compound of the formula 1 wherein R is $C_1$—$C_7$-alkanoyl or a group of the formulae

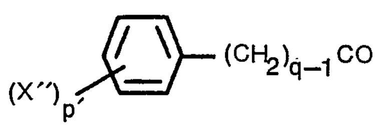

or $R^2OCO$, or

optionally reacting a compound of the formula 1 where R is $C_1$—$C_7$-alkyl and $R^1$ is formyl, $C_1$—$C_7$-alkanoyl or a group of the formula

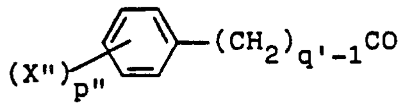

wherein X'', p'' and q' are as defined above with cyanogen bromide or chloride to yield a compound of the formula 1 wherein R is cyano and $R^1$ is as hereinbefore described, or optionally removing the cyano group from a compound of the formula 1 wherein $R^1$ is as defined above and R is cyano by hydrolysis to yield a compound of the formula 1 wherein R is hydrogen, or optionally hydrolyzing a compound of the formula 1 wherein R is a group of the formula

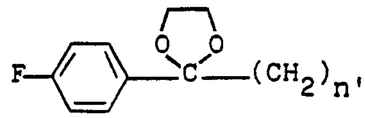

to yield a compound of the formula 1 wherein R is a group of the formula

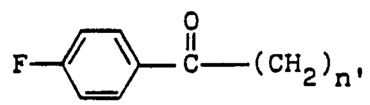

wherein n' is as defined above, and optionally preparing in a conventional way the pharmaceutically acceptable salts thereof.

13. The process of claim 12 wherein the cyclization is performed with hydrazine hydrate.

14. The process of claim 12 wherein the cyclization is performed at a temperature within the range of 100° to 200°C and a pressure of 14 to 21 bar and in a solvent.

15. A pharmaceutical composition comprising as active ingredient a compound of the formula 1 as claimed in claim 1 or a pharmaceutically acceptable acid addition salt thereof in association with a pharmaceutically acceptable carrier.

**Claims for the Contracting State: AT**

1. A process for the preparation of a compound of the formula 1

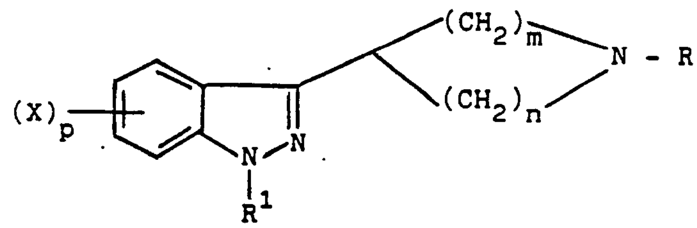

(1),

wherein R is hydrogen, $C_1$—$C_7$-alkyl, $C_3$—$C_7$-alkenyl, $C_3$—$C_7$-cycloalkyl-$C_1$—$C_7$-alkyl,

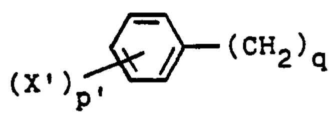

cyano, cyanomethyl, formyl, $C_1$—$C_7$-alkanoyl, or a group of the formulae

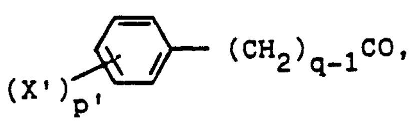

$R^2OCO$,

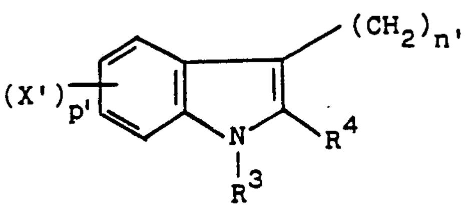

,

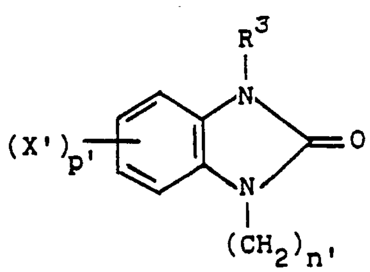

,

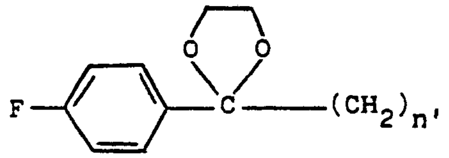

,

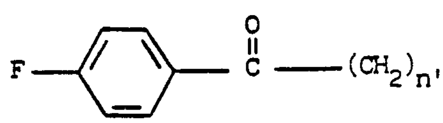

,

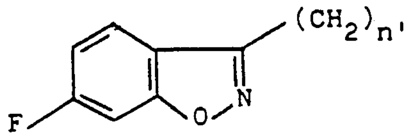

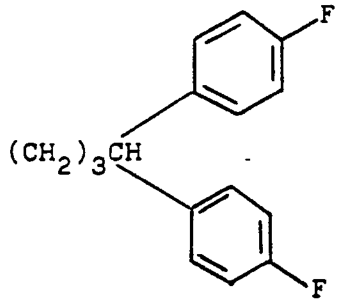

$R^1$ is hydrogen, $C_1$—$C_7$-alkyl, $C_3$—$C_7$-alkenyl, $C_3$—$C_7$-cycloalkyl-$C_1$—$C_7$-alkyl,

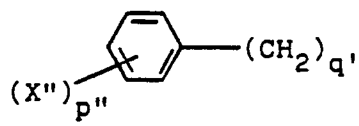

di-$C_1$—$C_7$-alkylamino-$C_1$—$C_7$-alkyl, cyano, cyanomethyl, formyl, $C_1$—$C_7$-alkanoyl, hydroxymethyl, $C_3$—$C_7$-cycloalkyl-$C_1$—$C_7$-alkanoyl, or a group of the formulae

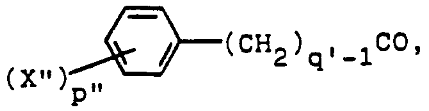 $R^{2'}OCO$,

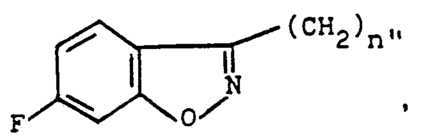 , 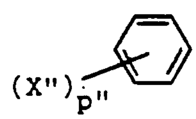 ,

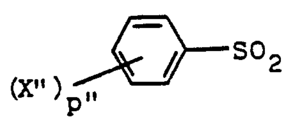 , or

$R^5CO$, or 2- or 4-pyridinyl or 2-pyrimidinyl; $R^2$ and $R^{2'}$ are independently $C_1$—$C_7$-alkyl, 2,2,2-trichloroethyl or phenyl; $R^3$ and $R^4$ are independently hydrogen or $C_1$—$C_7$-alkyl; $R^5$ is a member selected from the group furyl, thienyl, pyridinyl, thiazolyl, isothiazolyl, oxazolyl or isoxazolyl, X, X' and X'' are independently hydrogen, halogen, $C_1$—$C_7$-alkyl, $C_1$—$C_7$-alkoxy, hdyroxy, nitro, amino or trifluoromethyl; m is 2 or 3, n is 1 or 2, and the sum of m and n is 3 or 4; n' and n'' are independently 2 or 3; p, p' and p'' are independently 1 or 2; q and q' are independently 1, 2, 3 or 4; the optical antipode thereof; or the pharmaceutically acceptable salt thereof, or the pharmaceutically acceptable acid addition salt thereof, which comprises cyclizing a compound of the formula

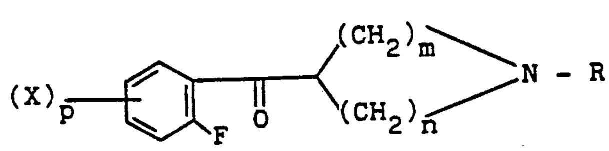

wherein R, X, m, n and p are as above with hydrazine hydrate, to yield a compound of the formula 1 wherein $R^1$ is hydrogen and R, X, M, n and p are as defined above, optionally treating a compound of the formula 1 wherein $R^1$ is hydrogen and R is as defined with the proviso that R is not hydrogen, with an $C_1$—$C_7$-alkyl halide, $C_3$—$C_7$-alkenyl halide, $C_3$—$C_7$-cycloalkyl-$C_1$—$C_7$-alkyl halide, di-$C_1$—$C_7$-alkylamino-$C_1$—$C_7$- alkyl halide, cyanogen halide, cyanomethyl halide, 2- or 4-pyridyl halide, 2-pyrimidinyl halide, a compound of the formulae

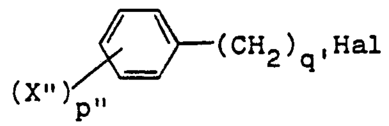 ,  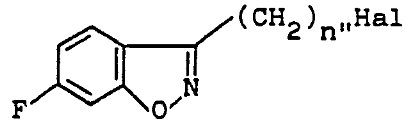 ,

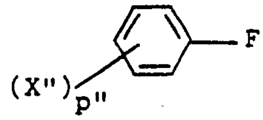 ,

wherein X'', n'', q' and p'' are as defined above and Hal is iodo, bromo or chloro in the presence of an alkali metal hydride to yield a compound of the formula 1 wherein $R^1$ is $C_1$—$C_7$-alkyl, $C_3$—$C_7$-alkenyl-$C_3$—$C_7$-cycloalkyl-$C_1$—$C_7$-alkyl, di-$C_1$—$C_7$-alkylamino-$C_1$—$C_7$-alkyl, cyano, cyanomethyl or a group of the formulae

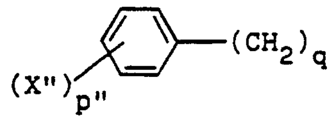 ,  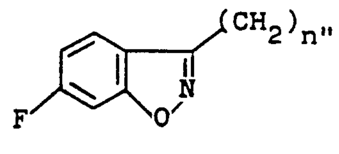 ,

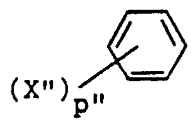 ;

2- or 4-piperidinyl or 2-pyrimidinyl, and X, m, n and p are as defined above, or optionally reacting a compound of the formula 1 wherein $R^1$ is hydrogen and R is as defined above with the proviso that R is not hydrogen, and X, m, n and p are as defined above, with a formyl-, $C_1$—$C_7$-alkanoyl- or $C_3$—$C_7$-cycloalkyl-$C_1$—$C_7$-alkanoyl chloride, bromide or iodide or a compound of the formula

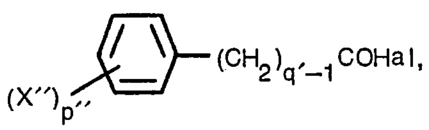, $R^{2'}OCOHal$ or $R^5COHal$,

where $R^{2'}$, $R^5$, X'', p'' and q' are as defined above and Hal is chloro, bromo or iodo, or the corresponding acid anhydrides to yield a compound of the formula 1 wherein $R^1$ is formyl, $C_1$—$C_7$-alkanoyl, $C_3$—$C_7$-cycloalkyl-$C_1$—$C_7$-alkanoyl or a group of the formulae

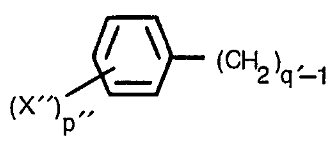, $R^{2'}OCO$ or $R^5CO$ , or

optionally reacting a compound of the formula 1 wherein $R^1$ is hydrogen and R is as defined above with the proviso that R is not hydrogen, with formaldehyd to yield a compound of the formula 1 wherein $R^1$ is hydroxymethyl or reacting a compound of the formula 1 wherein $R^1$ is hydrogen and R is phenoxycarbonyl with formaldehyde and then removing the carbamoyl group by hydrolysis to yield a compound of the formula 1 wherein $R^1$ is hydroxymethyl and R is hydrogen, or optionally reacting a compound of the formula 1 wherein $R^1$ is hydrogen and R is as defined above but is not hydrogen, with a benzenesulfonyl halide of the formula

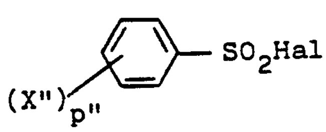 .

wherein X'' and p'' are defined above and Hal is chlorine or bromine, to yield a compound of the formula 1 wherein $R^1$ is the group

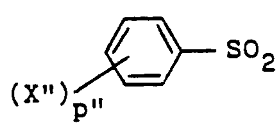 , or

optionally converting a compound of the formula 1 wherein $R^1$ is a group of the formulae

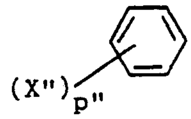

wherein X'' is the nitro group, by reduction of the nitro group to an amino function, diazotization and subsequent displacement or reduction of the diazonium moiety to a compound of the formula 1 wherein $R^1$ is a group of the formula

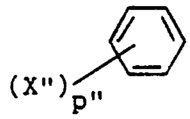

where X'' is hydrogen, halogen, $C_1$—$C_7$-alkyl, $C_1$—$C_7$-alkoxy, hydroxy or amino and p'' is 1 or 2, optionally reacting a compound of the formula 1 wherein R and $R^1$ are both hydrogen with a $C_1$—$C_7$-alkanoyl chloride, bromide or iodide or with a halide of the formulae

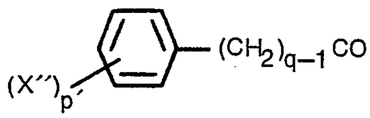 or $R^{2'}OCO$ Hal

where $R^{2'}$, X', p' and q are as defined above and Hal is chlorine, bromine or iodine, to yield a compound of the formula 1 wherein R is $C_1$—$C_7$-alkanoyl or a group of the formulae

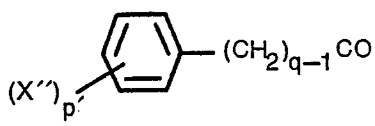 or $R^2OCO$, or

optionally reacting a compound of the formula 1 where R is $C_1$—$C_7$-alkyl and $R^1$ is formyl, $C_1$—$C_7$-alkanoyl or a group of the formula

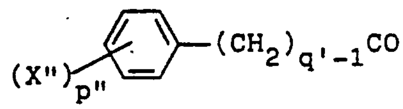 ,

wherein X'', p'' and q' are as defined above with cyanogen bromide or chloride to yield a compound of the formula 1 wherein R is cyano and $R^1$ is as hereinbefore described, or optionally removing the cyano group from a compound of the formula 1 wherein $R^1$ is as defined above and R is cyano by hydrolysis to yield a compound of the formula 1 wherein R is hydrogen, or optionally hydrolyzing a compound of the formula 1 wherein R is a group of the formula

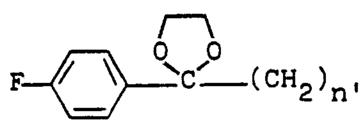

to yield a compound of the formula 1 wherein R is a group of the formula

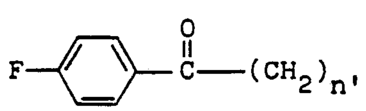

wherein n' is as defined above, and optionally preparing in a conventional way the pharmaceutically acceptable salts thereof.

2. The process of claim 1 wherein the cyclization is performed with hydrazine hydrate.

3. The process of claim 2 wherein the cyclization is performed at a temperature within the range of 100° to 200°C and a pressure of 14 to 21 bar and in a solvent.

**Patentansprüche für die Vertragsstaaten: BE CH DE FR GB IT LI LU NL SE**

1. Eine Verbindung der Formel 1

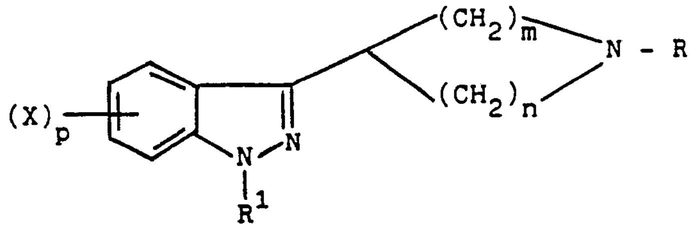

(1) ,

worin R Wasserstoff, $C_1$—$C_7$-Alkyl, $C_3$—$C_7$-Alkenyl, $C_3$—$C_7$-Cycloalkyl-$C_1$—$C_7$-alkyl,

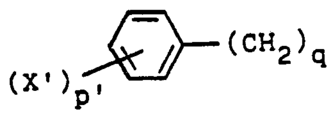

,

Cyano, Cyanomethyl, Formyl, $C_1$—$C_7$-Alkanoyl, oder eine Gruppe der Formeln

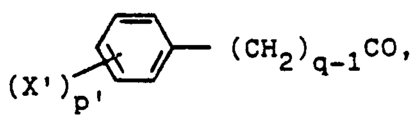

$R^2OCO$,

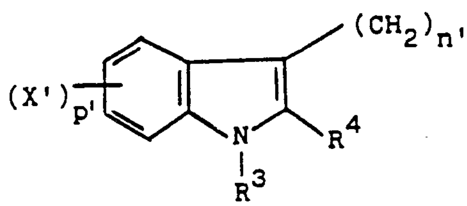

,

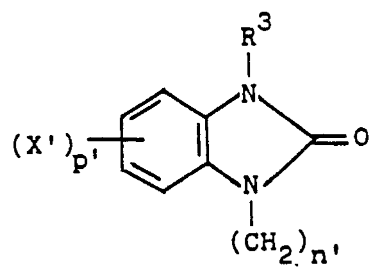

,

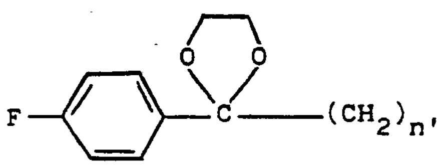

,

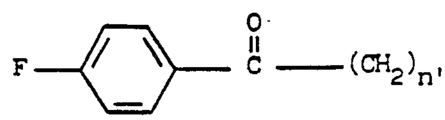

,

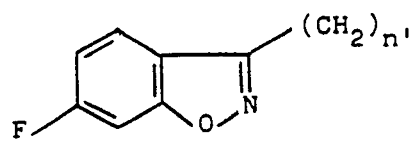

oder

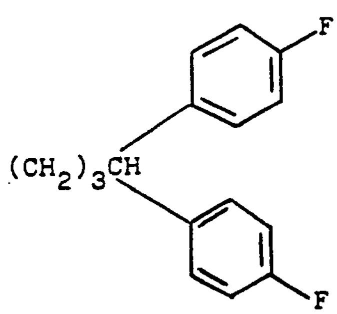

ist, $R^1$ Wasserstoff, $C_1$—$C_7$-Alkyl, $C_3$—$C_7$-Alkenyl, $C_3$—$C_7$-Cycloalkyl-$C_1$—$C_7$-alkyl,

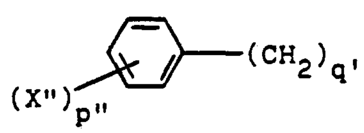

,

Di-$C_1$—$C_7$-alkylamino-$C_1$—$C_7$-alkyl, Cyano, Cyanomethyl, Formyl, $C_1$—$C_7$-Alkanoyl, Hydroxymethyl, $C_3$—$C_7$-Cycloalkyl-$C_1$—$C_7$-alkanoyl, oder eine Gruppe der Formeln

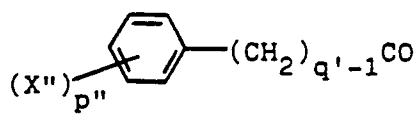 , $R^{2'}CO$ ,

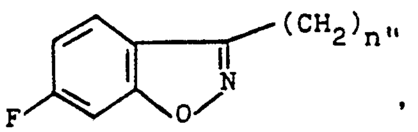 ,

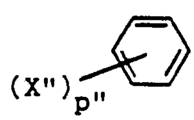 ,

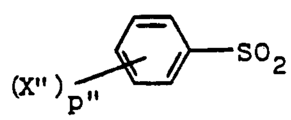 oder

$R^5CO$, 2- oder 4-Pyridinyl oder 2-Pyrimidinyl ist; $R^2$ und $R^{2'}$ unabhängig $C_1$—$C_7$-Alkyl, 2,2,2-Trichlorethyl oder Phenyl sind; $R^3$ und $R^4$ unabhängig Wasserstoff oder $C_1$—$C_7$-Alkyl sind; $R^5$ Furyl, Thienyl, Pyridinyl, Thiazolyl, Isothiazolyl, Oxazolyl oder Isoxazolyl bedeutet; X, X' und X'' unabhängig Wasserstoff, Halogen, $C_1$—$C_7$-Alkyl, $C_1$—$C_7$-Alkoxy, Hydroxy, Nitro, Amino oder Trifluormethyl sind; m 2 oder 3 ist; n 1 oder 2 ist; und die Summe von m und n 3 oder 4 ist; n' und n'' unabhängig 2 oder 3 sind; p, p' und p'' unabhängig 1 oder 2 sind; und q und q' unabhängig 1, 2, 3 oder 4 sind; der optische Antipode davon; oder das pharmazeutisch annehmbare Salz davon.

2. Eine Verbindung nach Anspruch 1, worin R Wasserstoff oder $C_1$—$C_7$-Alkyl oder

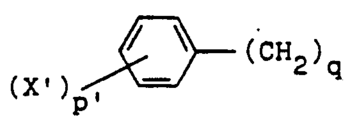

ist; und $R^1$ Wasserstoff, $C_1$—$C_7$-Alkyl, $C_3$—$C_7$-Cycloalkyl-$C_1$—$C_7$-alkyl, $C_1$—$C_7$-Alkanoyl, Hydroxymethyl oder

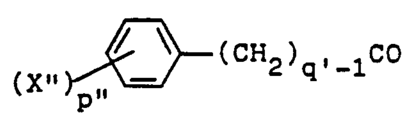

ist, wobei X' und X'' unabhängig Wasserstoff, Halogen, $C_1$—$C_7$-Alkyl, $C_1$—$C_7$-Alkoxy, Hydroxy, Nitro, Amino oder Trifluormethyl sind, p' und p'' 1 oder 2 sind, und q und q' unabhängig 1, 2, 3 oder 4 sind; oder $R^1$ $R^{2'}OCO$ ist, wobei $R^{2'}$ $C_1$—$C_7$-Alkyl, 2,2,2-Trichlorethyl oder Phenyl ist, oder $R^1$

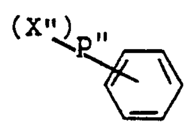

ist, wobei X'' Wasserstoff, Halogen, $C_1$—$C_7$-Alkyl, $C_1$—$C_7$-Alkoxy, Hydroxy, Nitro, Amino oder Trifluormethyl ist; und p'' 1 oder 2 ist.

3. Eine Verbindung nach Anspruch 2, worin R Wasserstoff oder $C_1$—$C_7$-Alkyl ist, und $R^1$ Wasserstoff, $C_1$—$C_7$-Alkyl oder eine Gruppe der Formeln

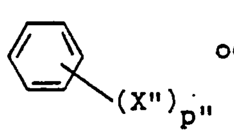 oder 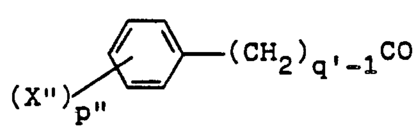

ist, wobei X'' Wasserstoff, $C_1$—$C_7$-Alkyl, $C_1$—$C_7$-Alkoxy, Hydroxy, Nitro, Amino oder Trifluormethyl ist; p'' 1 oder 2 ist; und q' 1 ist.

4. Die Verbindung des Anspruchs 1, welche 3-(1-Methyl-4-piperidinyl)-1H-indazol ist.

5. Die Verbindung des Anspruchs 1, welche 1-Ethyl-3-(1-methyl-4-piperidinyl)-1H-indazol ist.

6. Die Verbindung des Anspruchs 1, welche 1-Acetyl-3-(1-Methyl-4-piperidinyl)-1H-indazol ist.

7. Die Verbindung des Anspruchs 1, welche 1-Benzoyl-3-(1-methyl-4-piperidinyl)-1H-indazol ist.

8. Die Verbindung des Anspruchs 1, welche 1-Phenylmethyl-3-(1-methyl-4-piperidinyl)-1H-indazol ist.

9. Die Verbindung des Anspruchs 1, welche 1-Cyclopropylmethyl-3-(1-methyl-4-piperidinyl)-1H-indazol ist.

10. Eine Verbindung wie in Anspruch 1 definiert, zur Verwendung als ein Arzneimittel.

11. Eine pharmazeutische Zusammensetzung, enthaltend eine Verbindung der Formel 1 oder ein pharmazeutisch annehmbares Salz davon, wie in Anspruch 1 definiert, als Wirkbestandteil, gemeinsam mit einem pharmazeutisch annehmbaren Träger.

12. Ein Verfahren zur Herstellung einer Verbindung wie in Anspruch 1 beansprucht, welches umfaßt: das Cyclisieren einer Verbindung der Formel

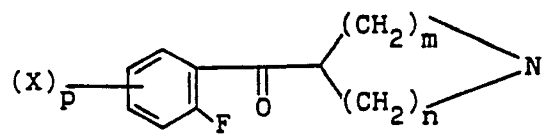 ,

worin R, X, m, n und p wie in Anspruch 1 definiert sind, mit Hydrazin oder Hydrazinhydrat, zur Gewinnung einer Verbindung der Formel 1, worin $R^1$ Wasserstoff ist, und R, X, m, n und p wie oben definiert sind, gegebenenfalls das Behandeln einer Verbindung der Formel 1, worin $R^1$ Wasserstoff ist, und R wie oben definiert ist, mit der Maßgabe, daß R nicht Wasserstoff ist, mit einem $C_1$—$C_7$-Alkylhalogenid, $C_3$—$C_7$-Alkenylhalogenid, $C_3$—$C_7$-Cycloalkyl-$C_1$—$C_7$-alkylhalogenid, Di-$C_1$—$C_7$-alkylamino-$C_1$—$C_7$-alkylhalogenid, Halogencyan, Cyanomethylhalogenid, 2- oder 4-Pyridylhalogenid, 2-Pyrimidinylhalogenid, oder einer Verbindung der Formeln

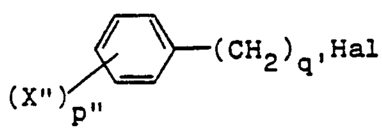 , 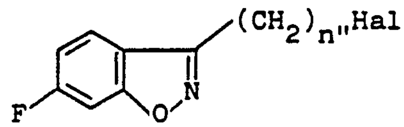 ,

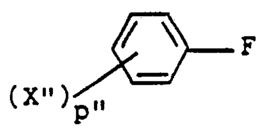 ,

worin X'', n'', q' und p'' wie oben definiert sind, und Hal Iod, Brom oder Chlor ist, in Gegenwart eines Alkalimetallhydrids, zur Gewinnung einer Verbindung der Formel 1, worin $R^1$ $C_1$—$C_7$-Alkyl, $C_3$—$C_7$-Alkenyl, $C_3$—$C_7$-Cycloalkyl-$C_1$—$C_7$-alkyl, Di-$C_1$—$C_7$-alkylamino-$C_1$—$C_7$-alkyl, Cyano, Cyanomethyl oder eine Gruppe der Formeln

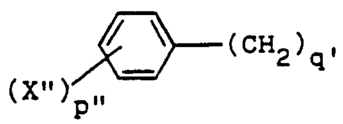 , 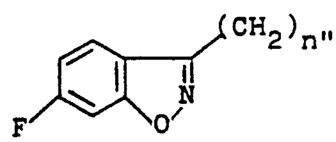 ,

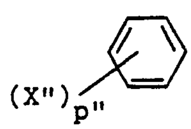 ;

2- oder 4-Piperidinyl oder 2-Pyrimidinyl ist, und X, m, n und p wie oben definiert sind, oder gegebenenfalls das Umsetzen einer Verbindung der Formel 1, worin $R^1$ Wasserstoff ist, und R wie oben definiert ist, mit der Maßgabe, daß R nicht Wasserstoff ist, und X, m, n und p wie oben definiert sind, mit einem Formyl-, $C_1$—$C_7$-Alkanoyl- oder $C_3$—$C_7$-Cycloalkyl-$C_1$—$C_7$-alkanoylchlorid, -bromid oder -iodid, oder einer Verbindung der Formeln

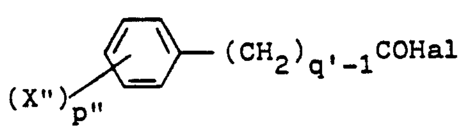 ,

$R^{2'}$OCO Hal oder $R^5$COHal, worin $R^{2'}$, $R^5$, X'', p'' und q' wie oben definiert sind, und Hal Chlor, Brom oder Iod ist, oden den entsprechenden Säureanhydriden, zur Gewinnung einer Verbindung der Formel 1, worin $R^1$ Formyl, $C_1$—$C_7$-Alkanoyl, $C_3$—$C_7$-Cycloalkyl-$C_1$—$C_7$-alkanoyl oder eine Gruppe der Formeln

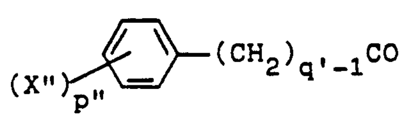 ,

$R^{2'}OCO$ oder $R^5CO$ ist, oder gegebenenfalls das Umsetzen einer Verbindung der Formel 1, worin $R^1$ Wasserstoff ist, und R wie oben definiert ist, mit der Maßgabe, daß R nicht Wasserstoff ist, mit Formaldehyd zur Gewinnung einer Verbindung der Formel 1, worin $R^1$ Hydroxymethyl ist, oder das Umsetzen einer Verbindung der Formel 1, worin $R^1$ Wasserstoff ist, und R Phenoxycarbonyl ist, mit Formaldehyd und anschließendes Entfernen der Carbamoylgruppe durch Hydrolyse zur Gewinnung einer Verbindung der Formel 1, worin $R^1$ Hydroxymethyl ist, und R Wasserstoff ist, oder gegebenenfalls das Umsetzen einer Verbindung der Formel 1, worin $R^1$ Wasserstoff ist, und R wie oben definiert ist, aber nicht Wasserstoff ist, mit einem Benzolsulfonylhalogenid der Formel

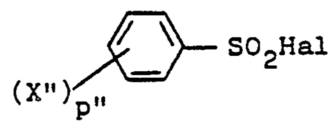 ,

worin X'' und p'' wie oben definiert sind, und Hal Chlor oder Brom ist, zur Gewinnung einer Verbindung der Formel 1, worin $R^1$ die Gruppe

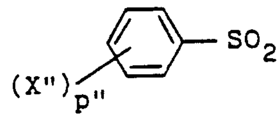

ist, oder gegebenenfalls das Umwandeln einer Verbindung der Formel 1, worin $R^1$ eine Gruppe der Formel

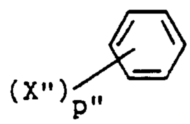

ist, worin X'' die Nitrogruppe ist, durch Reduktion der Nitrogruppe zu einer Aminofunktion, Diazotieren und anschließende Verdrängung oder Reduktion des Diazoniumrestes, zu einer Verbindung der Formel 1, worin $R^1$ eine Gruppe der Formel

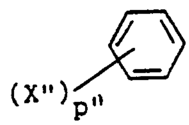

ist, wobei X'' Wasserstoff, Halogen, $C_1$—$C_7$-Alkyl, $C_1$—$C_7$-Alkoxy, Hydroxy oder Amino ist, und p'' 1 oder 2 ist, gegebenenfalls das Umsetzen einer Verbindung der Formel 1, worin R und $R^1$ beide Wasserstoff sind, mit einem $C_1$—$C_7$-Alkanoylchlorid, -bromid oder -iodid oder mit einem Halogenid der Formeln

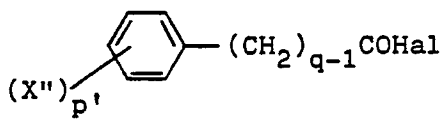 oder $R^{2'}OCOHal$ ,

wobei $R^{2'}$, X', p' und q wie oben definiert sind, und Hal Chlor, Brom oder Iod ist, zur Gewinnung einer Verbindung der Formel 1, worin R $C_1$—$C_7$-Alkanoyl oder eine Gruppe der Formeln

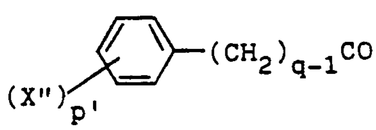 oder $R^2OCO$

ist, oder gegebenenfalls das Umsetzen einer Verbindung der Formel 1, worin R $C_1$—$C_7$-Alkyl ist, und $R^1$ Formyl, $C_1$—$C_7$-Alkanoyl oder eine Gruppe der Formel

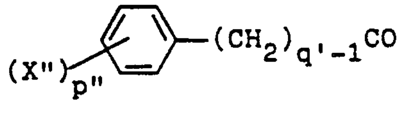

ist, wobei X'', p'' und q' wie oben definiert sind, mit Bromcyan oder Chlorcyan zur Gewinnung einer Verbindung der Formel 1, worin R Cyano ist, und $R^1$ wie oben beschrieben ist, oder gegebenenfalls das Entfernen der Cyanogruppe aus einer Verbindung der Formel 1, worin $R^1$ wie oben definiert ist, und R Cyano ist, durch Hydrolyse zur Gewinnung einer Verbindung der Formel 1, worin R Wasserstoff ist, oder gegebenenfalls das Hydrolysieren einer Verbindung der Formel 1, worin R eine Gruppe der Formel

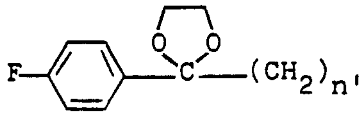

ist, zur Gewinnung einer Verbindung der Formel 1, worin R eine Gruppe der Formel

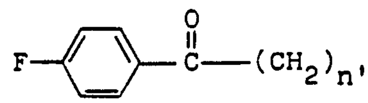

ist, wobei n′ wie oben definiert ist, und gegebenenfalls das Herstellen der pharmazeutisch annehmbaren Salze davon in einer herkömmlichen Weise.

13. Das Verfahren des Anspruchs 12, wobei die Cyclisierung mit Hydrazinhydrat bewirkt wird.

14. Das Verfahren des Anspruchs 12, wobei die Cyclisierung bei einer Temperatur im Bereich von 100° bis 200°C und bei einem Druck von 14 bis 21 bar in einem Lösungsmittel ausgeführt wird.

15. Eine pharmazeutische Zusammensetzung, enthaltend eine Verbindung der Formel 1, wie in Anspruch 1 beansprucht, oder ein pharmazeutisch annehmbares Säureadditionssalz davon, als Wirkbestandteil, gemeinsam mit einem pharmazeutisch annehmbaren Träger.

**Patentansprüche für den Vertragsstaat: AT**

1. Ein Verfahren zur Herstellung einer Verbindung der Formel 1

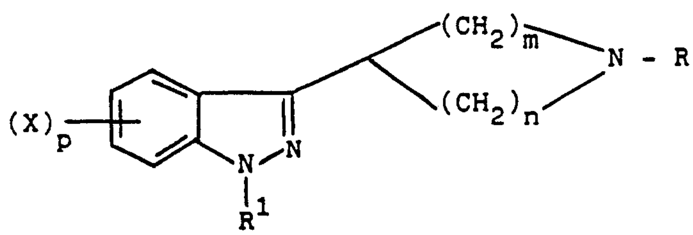

(1) ,

worin R Wasserstoff, $C_1$—$C_7$-Alkyl, $C_3$—$C_7$-Alkenyl, $C_3$—$C_7$-Cycloalkyl-$C_1$—$C_7$-alkyl,

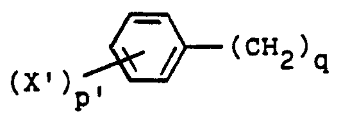 ,

Cyano, Cyanomethyl, Formyl, $C_1$—$C_7$-Alkanoyl, oder eine Gruppe der Formeln

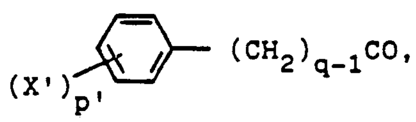 $R^2OCO$,

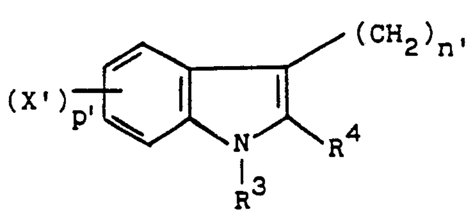 ,

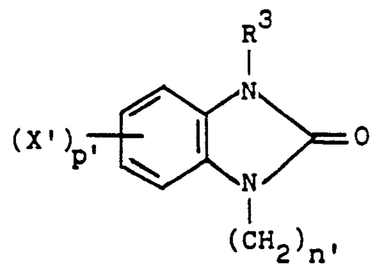 ,

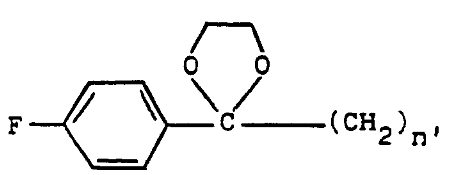 ,

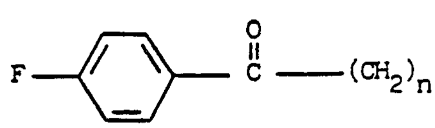 ,

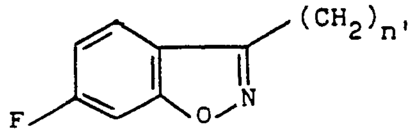

oder

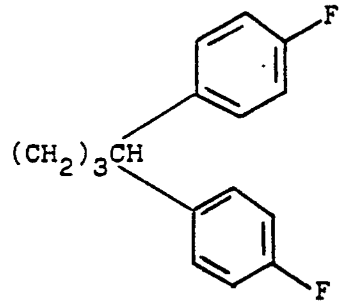

ist, $R^1$ Wasserstoff, $C_1$—$C_7$-Alkyl, $C_3$—$C_7$-Alkenyl, $C_3$—$C_7$-Cycloalkyl-$C_1$—$C_7$-alkyl,

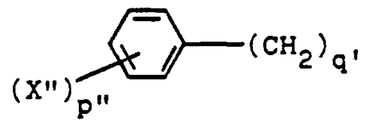

,

Di-$C_1$—$C_7$-alkylamino-$C_1$—$C_7$-alkyl, Cyano, Cyanomethyl, Formyl, $C_1$—$C_7$-Alkanoyl, Hydroxymethyl, $C_3$—$C_7$-Cycloalkyl-$C_1$—$C_7$-alkanoyl, oder eine Gruppe der Formeln

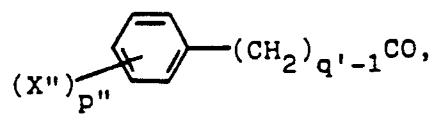

$R^{2'}CO$ ,

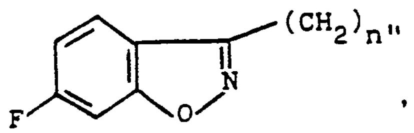

,

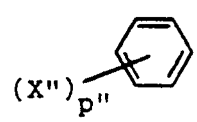

,

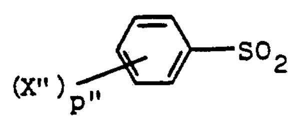

oder

$R^5CO$, 2- oder 4-Pyridinyl oder 2-Pyrimidinyl ist; $R^2$ und $R^{2'}$ unabhängig $C_1$—$C_7$-Alkyl, 2,2,2-Trichlorethyl oder Phenyl sind; $R^3$ und $R^4$ unabhängig Wasserstoff oder $C_1$—$C_7$-Alkyl sind; $R^5$ Furyl, Thienyl, Pyridinyl, Thiazolyl, Isothiazolyl, Oxazolyl oder Isoxazolyl bedeutet; X, X' und X'' unabhängig Wasserstoff, Halogen, $C_1$—$C_7$-Alkyl, $C_1$—$C_7$-Alkoxy, Hydroxy, Nitro, Amino oder Trifluormethyl sind; m 2 oder 3 ist; n 1 oder 2 ist; und die Summe von m und n 3 oder 4 ist; n' und n'' unabhängig 2 oder 3 sind; p, p' und p'' unabhängig 1 oder 2 sind; und q und q' unabhängig 1, 2, 3 oder 4 sind; des optischen Antipoden davon, oder des pharmazeutisch annehmbaren Säureadditionssalzes davon, welches umfaßt: das Cyclisieren einer Verbindung der Formel

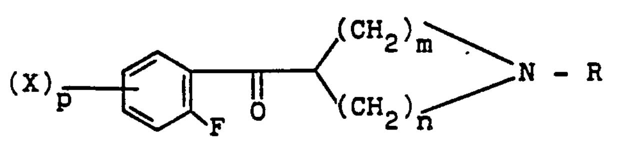

,

worin R, X, m, n und p wie oben definiert sind, mit Hydrazin oder Hydrazinhydrat, zur Gewinnung einer Verbindung der Formel 1, worin $R^1$ Wasserstoff ist, und R, X, m, n und p wie oben definiert sind, gegebenenfalls das Behandeln einer Verbindung der Formel 1, worin $R^1$ Wasserstoff ist, und R wie oben definiert ist, mit der Maßgabe, daß R nicht Wasserstoff ist, mit einem $C_1$—$C_7$-Alkylhalogenid, $C_3$—$C_7$-Alkenylhalogenid, $C_3$—$C_7$-Cycloalkyl-$C_1$—$C_7$-alkylhalogenid, Di-$C_1$—$C_7$-alkylamino-$C_1$—$C_7$-alkylhalogenid, Halogencyan, Cyanomethylhalogenid, 2- oder 4-Pyridylhalogenid, 2-Pyrimidinylhalogenid, oder einer Verbindung der Formeln

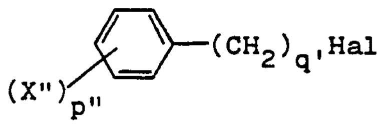 , 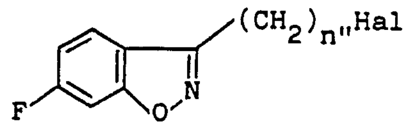 ,

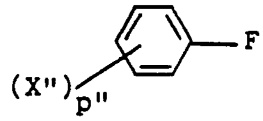 ,

worin X'', n'', q' und p'' wie oben definiert sind, und Hal Iod, Brom oder Chlor ist, in Gegenwart eines Alkalimetallhydrids, zur Gewinnung einer Verbindung der Formel 1, worin $R^1$ $C_1$—$C_7$-Alkyl, $C_3$—$C_7$-Alkenyl, $C_3$—$C_7$-Cycloalkyl-$C_1$—$C_7$-alkyl, Di-$C_1$—$C_7$-alkylamino-$C_1$—$C_7$-alkyl, Cyano, Cyanomethyl oder eine Gruppe der Formeln

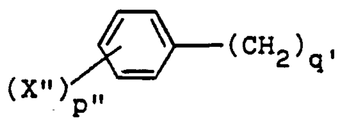 , 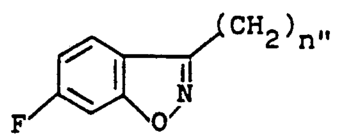 ,

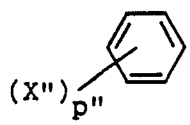 ;

2- oder 4-Piperidinyl oder 2-Pyrimidinyl ist, und X, m, n und p wie oben definiert sind, oder gegebenenfalls das Umsetzen einer Verbindung der Formel 1, worin $R^1$ Wasserstoff ist, und R wie oben definiert ist, mit der Maßgabe, daß R nicht Wasserstoff ist, und X, m, n und p wie oben definiert sind, mit einem Formyl-, $C_1$—$C_7$-Alkanoyl- oder $C_3$—$C_7$-Cycloalkyl-$C_1$—$C_7$-alkanoylchlorid, -bromid oder -iodid, oder einer Verbindung der Formeln

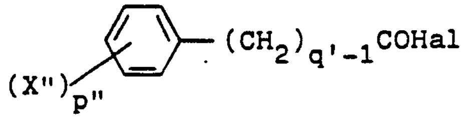 ,

$R^{2'}$OCO Hal oder $R^5$COHal, worin $R^{2'}$, $R^5$, X'', p'' und q' wie oben definiert sind, und Hal Chlor, Brom oder Iod ist, oden den entsprechenden Säureanhydriden, zur Gewinnung einer Verbindung der Formel 1, worin $R^1$ Formyl, $C_1$—$C_7$-Alkanoyl, $C_3$—$C_7$-Cycloalkyl-$C_1$—$C_7$-alkanoyl oder eine Gruppe der Formeln

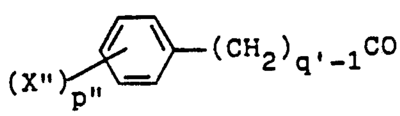 ,

$R^{2'}$OCO oder $R^5$CO ist, oder gegebenenfalls das Umsetzen einer Verbindung der Formel 1, worin $R^1$ Wasserstoff ist, und R wie oben definiert ist, mit der Maßgabe, daß R nicht Wasserstoff ist, mit Formaldehyd zur Gewinnung einer Verbindung der Formel 1, worin $R^1$ Hydroxymethyl ist, oder das Umsetzen einer Verbindung der Formel 1, worin $R^1$ Wasserstoff ist, und R Phenoxycarbonyl ist, mit Formaldehyd und anschließendes Entfernen der Carbamoylgruppe durch Hydrolyse zur Gewinnung einer Verbindung der Formel 1, worin $R^1$ Hydroxymethyl ist, und R Wasserstoff ist, oder gegebenenfalls das Umsetzen einer Verbindung der Formel 1, worin $R^1$ Wasserstoff ist, und R wie oben definiert ist, aber nicht Wasserstoff ist, mit einem Benzolsulfonylhalogenid der Formel

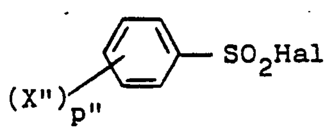 ,

worin X'' und p'' wie oben definiert sind, und Hal Chlor oder Brom ist, zur Gewinnung einer Verbindung der Formel 1, worin $R^1$ die Gruppe

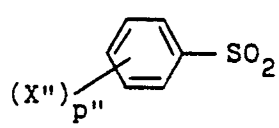

ist, oder gegebenenfalls das Umwandeln einer Verbindung der Formel 1, worin $R^1$ eine Gruppe der Formel

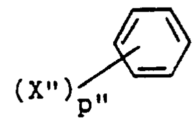

ist, worin X'' die Nitrogruppe ist, durch Reduktion der Nitrogruppe zu einer Aminofunktion, Diazotieren und anschließende Verdrängung oder Reduktion des Diazoniumrestes, zu einer Verbindung der Formel 1, worin $R^1$ eine Gruppe der Formel

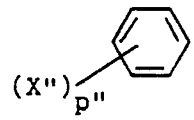

ist, wobei X'' Wasserstoff, Halogen, $C_1$—$C_7$-Alkyl, $C_1$—$C_7$-Alkoxy, Hydroxy oder Amino ist, und p'' 1 oder 2 ist, gegebenenfalls das Umsetzen einer Verbindung der Formel 1, worin R und $R^1$ beide Wasserstoff sind, mit einem $C_1$—$C_7$-Alkanoylchlorid, -bromid oder -iodid oder mit einem Halogenid der Formeln

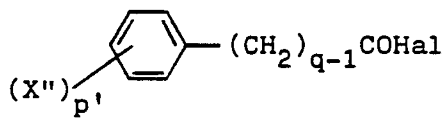 oder $R^{2'}OCOHal$ ,

wobei $R^{2'}$, X', p' und q wie oben definiert sind, und Hal Chlor, Brom oder Iod ist, zur Gewinnung einer Verbindung der Formel 1, worin R $C_1$—$C_7$-Alkanoyl oder eine Gruppe der Formeln

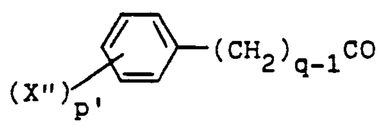 oder $R^2OCO$

ist, oder gegebenenfalls das Umsetzen einer Verbindung der Formel 1, worin R $C_1$—$C_7$-Alkyl ist, und $R^1$ Formyl, $C_1$—$C_7$-Alkanoyl oder eine Gruppe der Formel

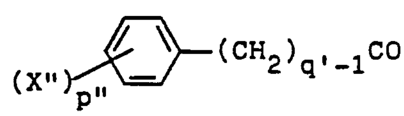

ist, wobei X'', p'' und q' wie oben definiert sind, mit Bromcyan oder Chlorcyan zur Gewinnung einer Verbindung der Formel 1, worin R Cyano ist, und $R^1$ wie oben beschrieben ist, oder gegebenenfalls das Entfernen der Cyanogruppe aus einer Verbindung der Formel 1, worin $R^1$ wie oben definiert ist, und R Cyano ist, durch Hydrolyse zur Gewinnung einer Verbindung der Formel 1, worin R Wasserstoff ist, oder gegebenenfalls das Hydrolysieren einer Verbindung der Formel 1, worin R eine Gruppe der Formel

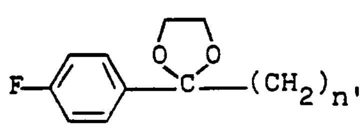

ist, zur Gewinnung einer Verbindung der Formel 1, worin R eine Gruppe der Formel

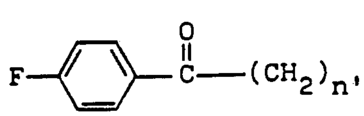

ist, wobei n' wie oben definiert ist, und gegebenenfalls das Herstellen der pharmazeutisch annehmbaren Salze davon in einer herkömmlichen Weise.

2. Das Verfahren des Anspruchs 1, wobei die Cyclisierung mit Hydrazinhydrat bewirkt wird.

3. Das Verfahren des Anspruchs 2, wobei die Cyclisierung bei einer Temperatur im Bereich von 100° bis 200°C und bei einem Druck von 14 bis 21 bar in einem Lösungsmittel ausgeführt wird.

**Revendications pour les Etats contractants: BE CH DE FR GB IT LI LU NL SE**

1. Composé de formule

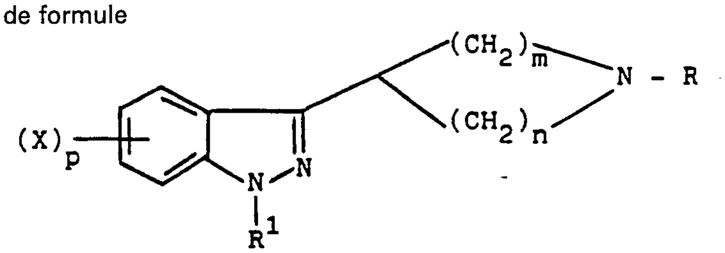

(1) ,

dans laquelle R est un atome d'hydrogène ou un groupe alkyle en $C_1$—$C_7$, alcényle en $C_3$—$C_7$, cycloalkyl($C_3$—$C_7$)-alkyle($C_1$—$C_7$), un groupe

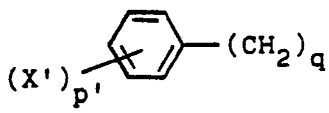

cyano, cyanométhyle, formyle, alcanoyle en $C_1$—$C_7$ ou un groupe de formules

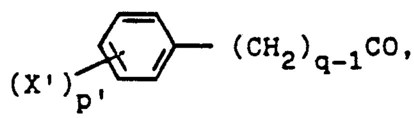 $R^2OCO$,

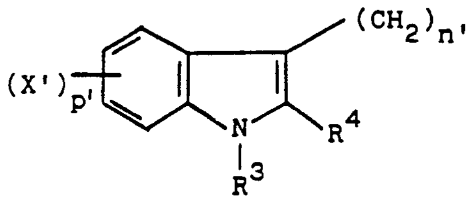 , 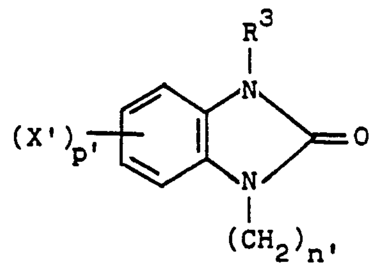 ,

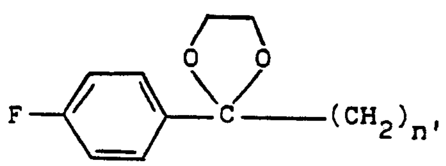 , 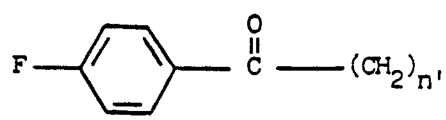 ,

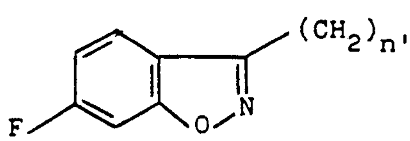

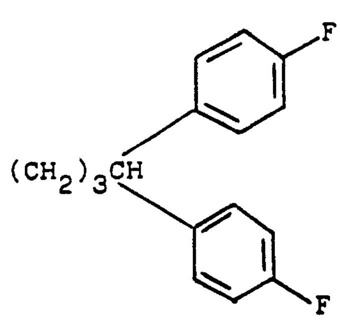 ;

$R^1$ est un atome d'hydrogène ou un groupe alkyle en $C_1$—$C_7$, alcényle en $C_3$—$C_7$, cycloalkyl($C_3$—$C_7$)-alkyle($C_1$—$C_7$), un groupe

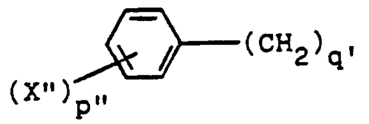

un groupe dialkyl($C_1$—$C_7$)-aminoalkyle($C_1$—$C_7$), cyano, cyanométhyle, formyle, alcanoyle en $C_1$—$C_7$, hydroxyméthyle, cycloalkyl($C_3$—$C_7$)-alcanoyle($C_1$—$C_7$) ou un groupe de formules

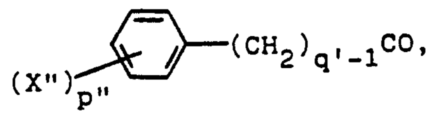 $R^{2'}OCO$,

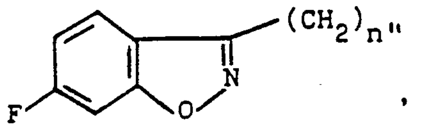 , 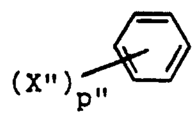 ,

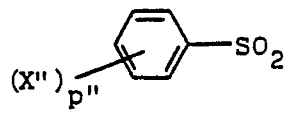 .

$R^5CO$, 2- ou 4-pyridinyle ou 2-pyrimidinyle; $R^2$ et $R^{2'}$ sont indépendamment un groupe alkyle en $C_1$—$C_7$, 2,2,2-trichloroéthyle ou phényle; $R^3$ et $R^4$ sont indépendamment un atome d'hydrogène ou un groupe alkyle en $C_1$—$C_7$; $R^5$ est un groupe choisi parmi les groupes furyle, thiényle, pyridinyle, thiazolyle, isothiazolyle, oxazolyle et isoxazolyle; X, X' et X'' sont indépendamment un atome d'hydrogène ou d'halogène ou un groupe alkyle en $C_1$—$C_7$, alcoxy en $C_1$—$C_7$, hydroxy, nitro, amino ou trifluorométhyle; m est 2 ou 3; n est 1 ou 2; et la somme de m et n est 3 ou 4; n' et n'' sont indépendamment 2 ou 3; p, p' et p'' sont indépendamment 1 ou 2; q et q' sont indépendamment 1, 2, 3 ou 4; inverse optique de celui-ci ou sel pharmaceutiquement acceptable de celui-ci.

2. Composé selon la revendication 1, dans lequel R est un atome d'hydrogène ou un groupe alkyle en $C_1$—$C_7$ ou un groupe

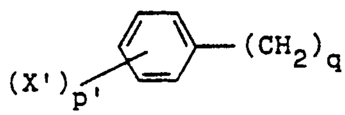 ;

et $R^1$ est un atome d'hydrogène ou un groupe alkyle en $C_1$—$C_7$, cycloalkyl($C_3$—$C_7$)alkyle($C_1$—$C_7$), alcanoyle en $C_1$—$C_7$, hydroxyméthyle, un groupe

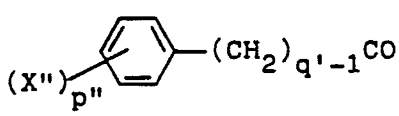

dans lequel X' et X'' sont indépendamment un atome d'hydrogène ou d'halogène ou un groupe alkyle en $C_1$—$C_7$, alcoxy en $C_1$—$C_7$, hydroxy, nitro, amino ou trifluorométhyle, p' et p'' sont 1 ou 2, et l et q' sont indépendamment 1, 2, 3 ou 4; ou $R^1$ est un groupe $R^{2'}OCO$ dans lequel $R^{2'}$ est un radical alkyle en $C_1$—$C_7$, 2,2,2-trichloroéthyle ou phényle; ou $R^1$ est un groupe

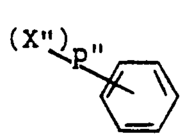

dans lequel X'' est un atome d'hydrogène ou d'halogène ou un groupe alkyle en $C_1$—$C_7$, alcoxy en $C_1$—$C_7$, hydroxy, nitro, amino ou trifluorométhyle et p'' est 1 ou 2.

3. Composé selon la revendication 2, dans lequel R est un atome d'hydrogène ou un groupe alkyle en $C_1$—$C_7$, et $R^1$ est un atome d'hydrogène ou un groupe alkyle en $C_1$—$C_7$ ou un groupe de formules

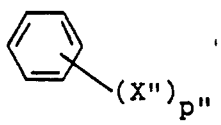 ou 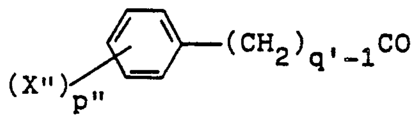

dans lesquelles X'' est un atome d'hydrogène ou un groupe alkyle en $C_1$—$C_7$, alcoxy en $C_1$—$C_7$, hydroxy, nitro, amino ou trifluorométhyle; p'' est 1 ou 2; et q' est 1.

4. Composé selon la revendication 1, lequel est le 3-(1-méthyl-4-pipéridinyl)-1H-indazole;

5. Composé selon la revendication 1, lequel est le 1-éthyl-3-(1-méthyl-4-pipéridinyl)-1H-indazole.

6. Composé selon la revendication 1, lequel est le 1-acétyl-3-(1-méthyl-4-pipéridinyl)-1H-indazole.

7. Composé selon la revendication 1, lequel est le 1-benzoyl-3-(1-méthyl-4-pipéridinyl)-1H-indazole.

8. Composé selon la revendication 1, lequel est le 1-phénylméthyl-3-(1-méthyl-4-pipéridinyl)-1H-indazole.

9. Composé selon la revendication 1, lequel est le 1-cyclopropylméthyl-3-(1-méthyl-4-pipéridinyl)-1H-indazole.

10. Composé selon la revendication 1, à utiliser en tant que médicament.

11. Composition pharmaceutique comprenant, en tant que composant actif, un composé de formule 1 ou un sel pharmaceutiquement acceptable de celui-ci, tel que défini dans la revendication 1, en association avec un véhicule pharmaceutiquement acceptable.

12. Procédé pour la préparation d'un composé selon la revendication 1, lequel comprend la cyclisation d'un composé de formule

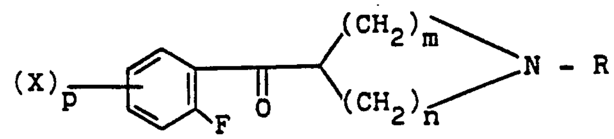

dans laquelle R, X, m, n et p sont tels que définis dans la revendication 1, avec de l'hydrazine ou un hydrate d'hydrazine, pour l'obtention d'un composé de formule 1 dans lequel $R^1$ est un atome d'hydrogène, et R, X, m, n et p sont tels que définis; si on le désire, le traitement d'un composé de formule 1 dans lequel $R^1$ est un atome d'hydrogène et R est tel que défini, avec la restriction que R n'est pas un atome d'hydrogène, par un halogénure d'alkyle en $C_1$—$C_7$, un halogénure d'alcényle en $C_3$—$C_7$, un halogénure de cycloalkyl($C_3$—$C_7$)alkyle($C_1$—$C_7$), un halogénure de dialkyl($C_1$—$C_7$)-aminoalkyle-($C_1$—$C_7$), un halogénure de cyanogène, un halogénure de cyanométhyle, un halogénure de 2- ou 4-pyridyle, un halogénure de 2-pyrimidinyle, un composé de formules

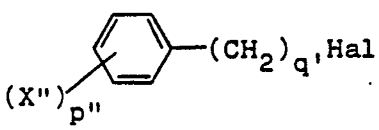 , 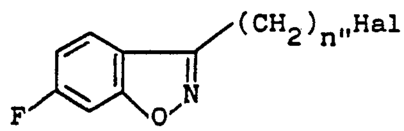 ,

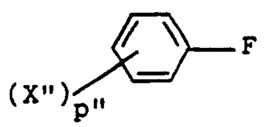 ,

dans lesquelles X'', n'', q' et p'' sont tels que définis et Hal est un atome d'iode, brome ou chlore, en présence d'un hydrure de métal alcalin, pour l'obtention d'un composé de formule 1 dans lequel $R^1$ est un groupe alkyle en $C_1$—$C_7$, alcényle en $C_3$—$C_7$, cycloalkyl($C_3$—$C_7$)-alkyle($C_1$—$C_7$), dialkyl($C_1$—$C_7$)-amino-alkyle($C_1$—$C_7$), cyano, cyanométhyle ou un groupe de formules

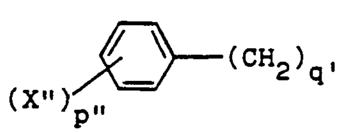 , 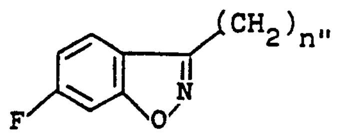 ,

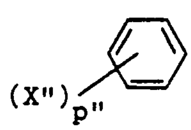 ;

un groupe 2- ou 4-pipéridinyle ou 2-pyrimidinyle, et X, m, n et p sont tels que définis plus haut; ou si on le désire, la réaction d'un composé de formule 1 dans lequel $R^1$ est un atome d'hydrogène et R est tel que défini plus haut, avec la restriction que R n'est pas un atome d'hydrogène, et X, m, n et p sont tels que définis plus haut, avec un chlorure, bromure ou iodure de formyle, d'alcanoyle en $C_1$—$C_7$ ou de cyclo-alkyl($C_3$—$C_7$)-alcanoyle($C_1$—$C_7$) ou avec un composé de formule

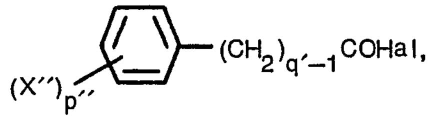 $R^{2'}OCOHal$ ou $R^5COHal$,

dans lesquelles $R^{2'}$, $R^5$, X'', p'' et q' sont tels que définis plus haut et hal est un atome de chlore, brome ou iode, ou avec les anhydrides d'acide correspondants, pour l'obtention d'un composé de formule 1 dans lequel $R^1$ est un groupe formyle, alcanoyle en $C_1$—$C_7$, cycloalkyl($C_3$—$C_7$)-alcanoyle($C_1$—$C_7$) ou un groupe de formules

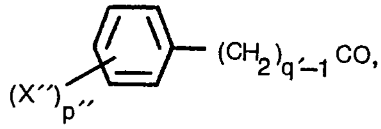 $R^{2'}OCO$ ou $R^5CO$; ou

si on le désire, la réaction d'un composé de formule 1 dans lequel $R^1$ est un atome d'hydrogène et R est tel que défini plus haut, avec la restriction que R n'est pas un atome d'hydrogène, avec le formaldéhyde, pour l'obtention d'un composé de formule 1 dans lequel $R^1$ est le groupe hydroxyméthyle; ou la réaction d'un composé de formule 1 dans lequel $R^1$ est un atome d'hydrogène et R est le groupe phénoxycarbonyle, avec le formaldéhyde, et ensuite élimination du groupe carbamoyle par hydrolyse, pour l'obtention d'un composé de formule 1 dans lequel $R^1$ est le groupe hydroxyméthyle et R est un atome d'hydrogène; ou si on le désire, la réaction d'un composé de formule 1 dans lequel $R^1$ est un atome d'hydrogène et R est tel que défini plus haut, mais n'est pas un atome d'hydrogène, avec un halogénure de benzènesulfonyle, de formule

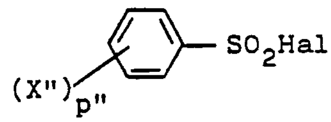

dans laquelle X'' et p'' sont tels que définis plus haut et Hal est le chlore ou le brome, pour l'obtention d'un composé de formule 1 dans lequel $R^1$ est un groupe de formule

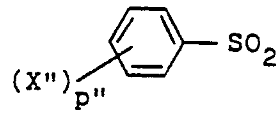 ; ou

si on le désire, la conversion d'un composé de formule 1 dans lequel $R^1$ est un groupe de formule

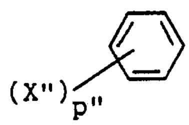

dans laquelle X'' est le groupe nitro, par réduction du groupe nitro en une fonction amino, diazotation et déplacement subséquent ou réduction subséquente du fragment diazonium, conduisant à un composé de formule 1 dans lequel $R^1$ est un groupe de formule

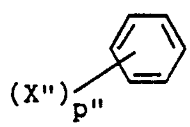

dans laquelle X'' est un atome d'hydrogène ou d'halogène ou un groupe alkyle en $C_1$—$C_7$, alcoxy en $C_1$—$C_7$, hydroxy ou amino, et p'' est 1 ou 2; si on le désire, la réaction d'un composé de formule 1 dans lequel R et $R^1$ sont chacun un atome d'hydrogène, avec un chlorure, bromure ou iodure d'alcanoyle en $C_1$—$C_7$ ou avec un halogénure de formules

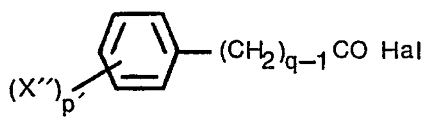 ou $R^{2'}OCO$ Hal ,

dans lesquelles $R^{2'}$, X', p' et q sont tels que définis plus haut, et Hal est le chlore, le brome ou l'iode, pour l'obtention d'un composé de formule 1 dans lequel R est un groupe alcanoyle en $C_1$—$C_7$ ou un groupe de formules

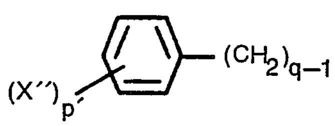 ou $R^2OCO$ ; ou

si on le désire, la réaction d'un composé de formule 1 dans lequel R est un groupe alkyle en $C_1$—$C_7$ et $R^1$ est un groupe formyle, alcanoyle en $C_1$—$C_7$, ou un groupe de formule

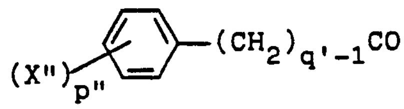

dans laquelle X'', p'' et q' sont tels que définis plus haut, avec le bromure ou le chlorure de cyanogène, pour l'obtention d'un composé de formule 1 dans lequel R est le groupe cyano et $R^1$ est tel que décrit précédemement; ou si on le désire, l'élimination du groupe cyano hors d'un composé de formule 1 dans lequel $R^1$ est tel que défini plus haut et R est le groupe cyano, par hydrolyse, pour l'obtention d'un composé de formule 1 dans lequel R est un atome d'hydrogène; ou si on le désire, l'hydrolyse d'un composé de formule 1 dans lequel R est un groupe de formule

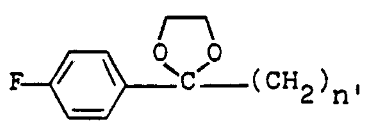

pour l'obtention d'un composé de formule 1 dans lequel R est un groupe de formule

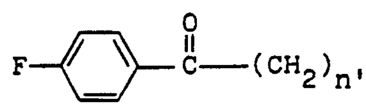

n' étant tel que défini plus haut; et éventuellement la préparation, d'une façon classique, des sels pharmaceutiquement acceptables de celui-ci.

13. Procédé selon la revendication 12, dans lequel la cyclisation est effectuée avec un hydrate d'hydrazine.

14. Procédé selon la revendication 12, dans lequel la cyclisation est effectuée à une température dans la plage de 100 à 200°C et sous une pression de 14 à 21 bars, et dans un solvant.

15. Composition pharmaceutique comprenant, en tant que composant actif, un composé de formule 1 selon la revendication 1, ou un sel d'addition avec un acide pharmaceutiquement acceptable de celui-ci, en association avec un véhicule pharmaceutiquement acceptable.

**Revendications pour l'Etat contractant: AT**

1. Procédé pour la préparation d'un composé de formule 1

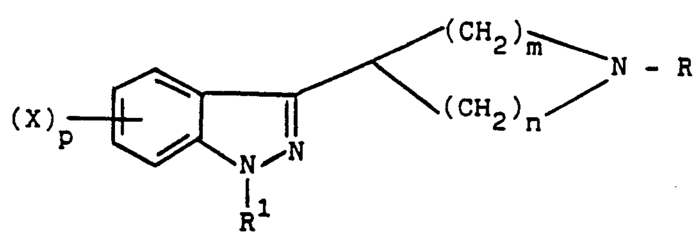

(1) ,

dans laquelle R est un atome d'hydrogène ou un groupe alkyle en $C_1$—$C_7$, alcényle en $C_3$—$C_7$, cycloalkyl($C_3$—$C_7$)-alkyle($C_1$—$C_7$), un groupe

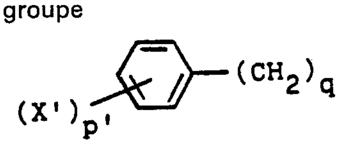

cyano, cyanométhyle, formyle, alcanoyle en $C_1$—$C_7$ ou un groupe de formules

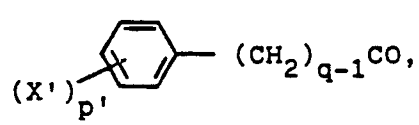 $R^2OCO$,

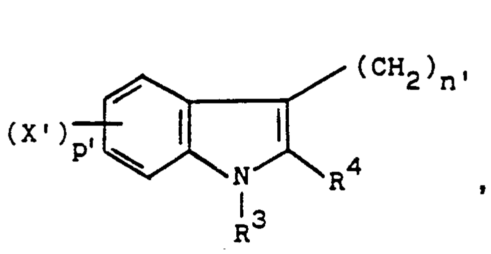

,

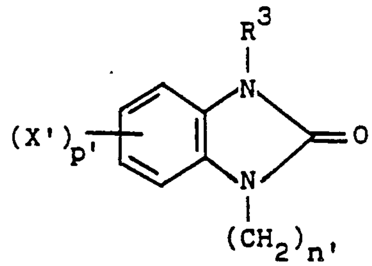

,

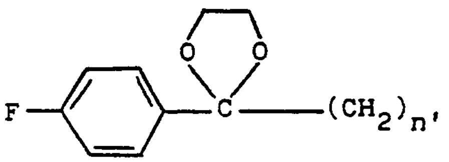

,

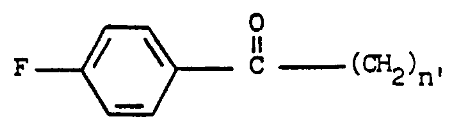

,

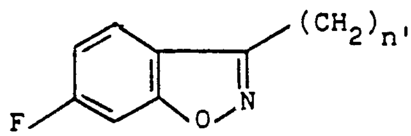

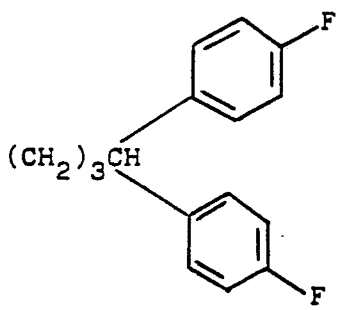

$R^1$ est un atome d'hydrogène ou un groupe alkyle en $C_1$—$C_7$, alcényle en $C_3$—$C_7$, cycloalkyl($C_3$—$C_7$)-alkyle($C_1$—$C_7$), un groupe

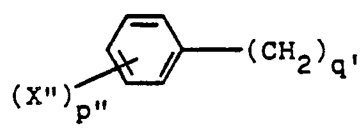

un groupe dialkyl($C_1$—$C_7$)-aminoalkyle($C_1$—$C_7$), cyano, cyanométhyle, formyle, alcanoyle en $C_1$—$C_7$, hydroxyméthyle, cycloalkyl($C_3$—$C_7$)-alcanoyle($C_1$—$C_7$) ou un groupe de formules

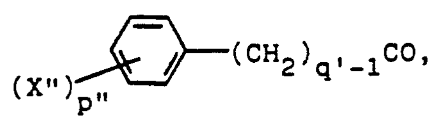

$R^{2'}OCO$,

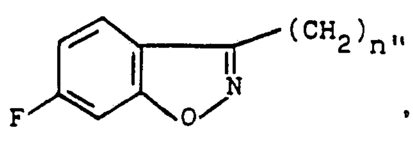

,

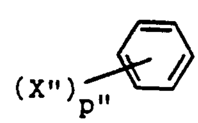

,

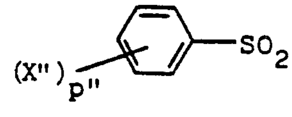

; ou

$R^5CO$, 2- ou 4-pyridinyle ou 2-pyrimidinyle; $R^2$ et $R^{2'}$ sont indépendamment un groupe alkyle en $C_1$—$C_7$, 2,2,2-trichloroéthyle ou phényle; $R^3$ et $R^4$ sont indépendamment un atome d'hydrogène ou un groupe alkyle en $C_1$—$C_7$; $R^5$ représente le groupe furyle, thiényle, pyridinyle, thiazolyle, isothiazolyle, oxazolyle ou isoxazolyle; X, X' et X'' sont indépendamment un atome d'hydrogène ou d'halogène ou un groupe alkyle

en $C_1$—$C_7$, alcoxy en $C_1$—$C_7$, hydroxy, nitro, amino ou trifluorométhyle; m est 2 ou 3; n est 1 ou 2; et la somme de m et n est 3 ou 4; n′ et n″ sont indépendamment 2 ou 3; p, p′ et p″ sont indépendamment 1 ou 2; q et q′ sont indépendamment 1, 2, 3 ou 4; de l'inverse optique de celui-ci ou d'un sel d'addition avec un acide pharmaceutiquement acceptable de celui-ci, lequel comprend la cyclisation d'un composé de formule

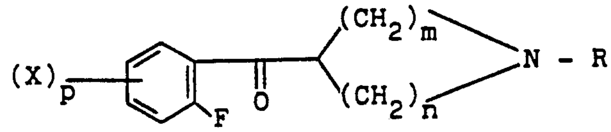

dans laquelle R, X, m, n et p sont tels que définis ci-dessus, avec de l'hydrazine ou un hydrate d'hydrazine, pour l'obtention d'un composé de formule 1 dans lequel $R^1$ est un atome d'hydrogène, et R, X, m, n et p sont tels que définis plus haut; si on le désire, le traitement d'un composé de formule 1 dans lequel $R^1$ est un atome d'hydrogène et R est tel que défini, avec la restriction que R n'est pas un atome d'hydrogène, par un halogénure d'alkyle en $C_1$—$C_7$, un halogénure d'alcényle en $C_3$—$C_7$, un halogénure de cycloalkyl($C_3$—$C_7$)alkyle($C_1$—$C_7$), un halogénure de dialkyl($C_1$—$C_7$)-aminoalkyle-($C_1$—$C_7$), un halogénure de cyanogène, un halogénure de cyanométhyle, un halogénure de 2- ou 4-pyridyle, un halogénure de 2-pyrimidinyle, un composé de formules

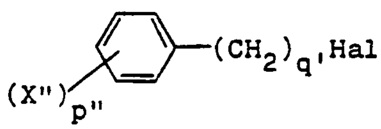 , 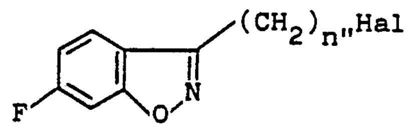 ,

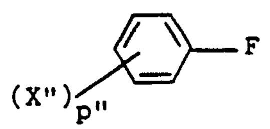 ,

dans lesquelles X″, n″, q′ et p″ sont tels que définis plus haut et Hal est un atome d'iode, brome ou chlore, en présence d'un hydrure de métal alcalin, pour l'obtention d'un composé de formule 1 dans lequel $R^1$ est un groupe alkyle en $C_1$—$C_7$, alcényle en $C_3$—$C_7$, cycloalkyl($C_3$—$C_7$)-alkyle($C_1$—$C_7$), dialkyl($C_1$—$C_7$)-amino-alkyle($C_1$—$C_7$), cyano, cyanométhyle ou un groupe de formules

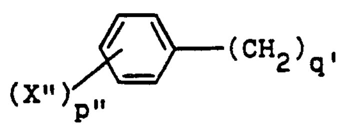 , 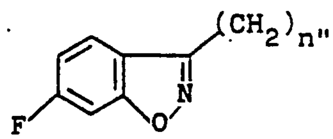 ,

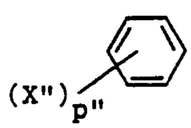 ;

un groupe 2- ou 4-pipéridinyle ou 2-pyrimidinyle, et X, m, n et p sont tels que définis plus haut; ou si on le désire, la réaction d'un composé de formule 1 dans lequel $R^1$ est un atome d'hydrogène et R est tel que défini plus haut, avec la restriction que R n'est pas un atome d'hydrogène, et X, m, n et p sont tels que définis plus haut, avec un chlorure, bromure ou iodure de formyle, d'alcanoyle en $C_1$—$C_7$ ou de cyclo-alkyl($C_3$—$C_7$)-alcanoyle($C_1$—$C_7$) ou avec un composé de formule

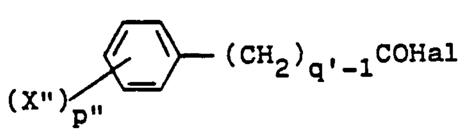 , $R^{2\prime}$OCOHal ou $R^5$COHal,

dans lesquelles $R^{2\prime}$, $R^5$, X″, p″ et q′ sont tels que définis plus haut et hal est un atome de chlore, brome ou iode, ou avec les anhydrides d'acide correspondants, pour l'obtention d'un composé de formule 1 dans lequel $R^1$ est un groupe formyle, alcanoyle en $C_1$—$C_7$, cycloalkyl($C_3$—$C_7$)-alcanoyle($C_1$—$C_7$) ou un groupe de formules

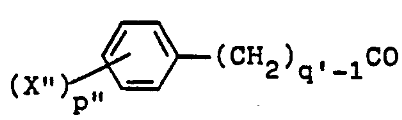 , $R^{2\prime}$OCO ou $R^5$CO; ou

si on le désire, la réaction d'un composé de formule 1 dans lequel $R^1$ est un atome d'hydrogène et R est tel que défini plus haut, avec la restriction que R n'est pas un atome d'hydrogène, avec le formaldéhyde, pour

l'obtention d'un composé de formule 1 dans lequel $R^1$ est le groupe hydroxyméthyle; ou la réaction d'un composé de formule 1 dans lequel $R^1$ est un atome d'hydrogène et R est le groupe phénoxycarbonyle, avec le formaldéhyde, et ensuite élimination du groupe carbamoyle par hydrolyse, pour l'obtention d'un composé de formule 1 dans lequel $R^1$ est le groupe hydroxyméthyle et R est un atome d'hydrogène; ou si on le désire, la réaction d'un composé de formule 1 dans lequel $R^1$ est un atome d'hydrogène et R est tel que défini plus haut, mais n'est pas un atome d'hydrogène, avec un halogénure de benzènesulfonyle, de formule

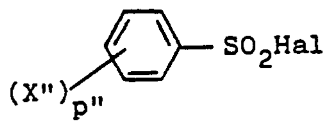

dans laquelle X'' et p'' sont tels que définis plus haut et Hal est le chlore ou le brome, pour l'obtention d'un composé de formule 1 dans lequel $R^1$ est un groupe de formule

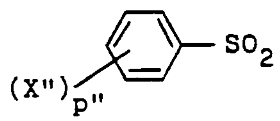 ; ou

si on le désire, la conversion d'un composé de formule 1 dans lequel $R^1$ est un groupe de formule

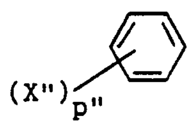

dans laquelle X'' est le groupe nitro, par réduction du groupe nitro en une fonction amino, diazotation et déplacement subséquent ou réduction subséquente du fragment diazonium, conduisant à un composé de formule 1 dans lequel $R^1$ est un groupe de formule

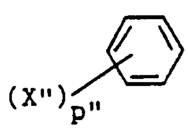

dans laquelle X'' est un atome d'hydrogène ou d'halogène ou un groupe alkyle en $C_1$—$C_7$, alcoxy en $C_1$—$C_7$, hydroxy ou amino, et p'' est 1 ou 2; si on le désire, la réaction d'un composé de formule 1 dans lequel R et $R^1$ sont chacun un atome d'hydrogène, avec un chlorure, bromure ou iodure d'alcanoyle en $C_1$—$C_7$ ou avec un halogénure de formules

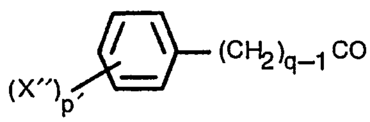 ou $R^{2'}$OCO Hal ,

dans lesquelles $R^{2'}$, X', p' et q sont tels que définis plus haut, et Hal est le chlore, le brome ou l'iode, pour l'obtention d'un composé de formule 1 dans lequel R est un groupe alcanoyle en $C_1$—$C_7$ ou un groupe de formules

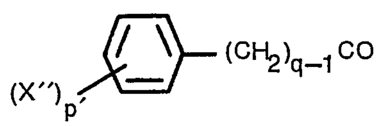 ou $R^2$OCO, ; ou

si on le désire, la réaction d'un composé de formule 1 dans lequel R est un groupe alkyle en $C_1$—$C_7$ et $R^1$ est un groupe formyle, alcanoyle en $C_1$—$C_7$, ou un groupe de formule

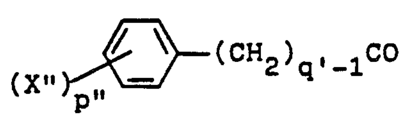

dans laquelle X'', p'' et q' sont tels que définis plus haut, avec le bromure ou le chlorure de cyanogène, pour l'obtention d'un composé de formule 1 dans lequel R est le groupe cyano et $R^1$ est tel que décrit précédemment; ou si on le désire, l'élimination du groupe cyano hors d'un composé de formule 1 dans lequel $R^1$ est tel que défini plus haut et R est le groupe cyano, par hydrolyse, pour l'obtention d'un composé

de formule 1 dans lequel R est un atome d'hydrogène; ou si on le désire, l'hydrolyse d'un composé de formule 1 dans lequel R est un groupe de formule

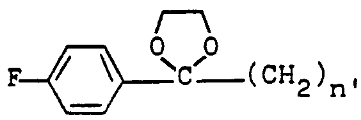

pour l'obtention d'un composé de formule 1 dans lequel R est un groupe de formule

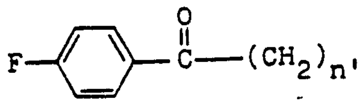

n' étant tel que défini plus haut; et éventuellement la préparation, d'une façon classique, des sels pharmaceutiquement acceptables de celui-ci.

2. Procédé selon la revendication 1, dans lequel la cyclisation est effectuée avec un hydrate d'hydrazine.

3. Procédé selon la revendication 2, dans lequel la cyclisation est effectuée à une température dans la plage de 100 à 200°C et sous une pression de 14 à 21 bars, et dans un solvant.